(19) Europäisches Patentamt
European Patent Office
Office européen des brevets

(11) EP 3 389 719 B1

(12) # EUROPEAN PATENT SPECIFICATION

(45) Date of publication and mention of the grant of the patent:
**17.11.2021 Bulletin 2021/46**

(21) Application number: **16876554.3**

(22) Date of filing: **14.12.2016**

(51) Int Cl.:
*A61K 47/50* (2017.01)
*A61P 31/16* (2006.01)
*A61K 47/64* (2017.01)
*A61P 31/00* (2006.01)
*A61P 31/06* (2006.01)
*A61P 43/00* (2006.01)
*A61P 21/00* (2006.01)
*A61K 47/58* (2017.01)
*A61P 25/00* (2006.01)
*A61P 31/04* (2006.01)
*A61P 31/12* (2006.01)

(86) International application number:
**PCT/US2016/066595**

(87) International publication number:
**WO 2017/106304 (22.06.2017 Gazette 2017/25)**

(54) **PEPTIDE OLIGONUCLEOTIDE CONJUGATES**

PEPTID-OLIGONUKLEOTID-KONJUGATE

CONJUGUÉS PEPTIDE-OLIGONUCLÉOTIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **15.12.2015 US 201562267723 P**

(43) Date of publication of application:
**24.10.2018 Bulletin 2018/43**

(73) Proprietor: **Sarepta Therapeutics, Inc.**
**Cambridge, MA 02142 (US)**

(72) Inventors:
- **HANSON, Gunnar, J.**
  **Cambridge, MA 02142 (US)**
- **ZHOU, Ming**
  **Cambridge, MD 02142 (US)**

(74) Representative: **D Young & Co LLP**
**120 Holborn**
**London EC1N 2DY (GB)**

(56) References cited:
**EP-A1- 2 623 507**
**WO-A2-2014/144978**
**WO-A2-2016/138534**
**US-A1- 2005 288 246**
**US-A1- 2015 238 627**

- **Peter Sazani ET AL: "Repeat-Dose Toxicology Evaluation in Cynomolgus Monkeys of AVI-4658, a Phosphorodiamidate Morpholino Oligomer (PMO) Drug for the Treatment of Duchenne Muscular Dystrophy", International Journal of Toxicology, vol. 30, n.3, 1 May 2011 (2011-05-01), pages 313-321, XP055555010, DOI: 10.1177/1091581811403505 Retrieved from the Internet: URL:http://journals.sagepub.com/doi/full-xml/10.1177/1091581811403505 [retrieved on 2019-02-12]**
- **SAZANI et al.: "Repeat-Dose Toxicology Evaluation in Cynomolgus Monkeys of AVI-4658, a Phosphorodiamidate Morpholino Oligomer (PMO) Drug for the Treatment of Duchenne Muscular Dystrophy", International Journal of Toxicology., vol. 30, 2011, pages 313-321, XP055555010,**
- **HEALD et al.: "Safety and Pharmacokinetic Profiles of Phosphorodiamidate Morpholino Oligomers with Activity against Ebola Virus and Marburg Virus: Results of Two Single-Ascending-Dose Studies", Antimicrobial Agents and Chemotherapy., vol. 58, no. 11, 2014, pages 6639-6647, XP055391922,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 389 719 B1

## Description

### SEQUENCE LISTING

The present application is being filed along with a Sequence Listing in computer readable format. The Sequence Listing is provided as a file entitled 586558SPT-002PC_SL.txt, created December 13, 2016, which is 4,076 bytes in size.

### RELATED APPLICATIONS

This application claims the benefit of priority to U.S. Provisional Patent Application No. 62/267,723, filed December 15, 2015.

### BACKGROUND

Antisense technology provides a means for modulating the expression of one or more specific gene products, including alternative splice products, and is uniquely useful in a number of therapeutic, diagnostic, and research applications. The principle behind antisense technology is that an antisense compound, e.g., an oligonucleotide, which hybridizes to a target nucleic acid, modulates gene expression activities such as transcription, splicing or translation through any one of a number of antisense mechanisms. The sequence specificity of antisense compounds makes them attractive as tools for target validation and gene functionalization, as well as therapeutics to selectively modulate the expression of genes involved in disease.

Although significant progress has been made in the field of antisense technology, there remains a need in the art for oligonucleotides, and peptide-oligonucleotide-conjugates with improved antisense or antigene performance. Such improved antisense or antigene performance includes, at least, for example: lower toxicity, stronger affinity for DNA and RNA without compromising sequence selectivity, improved pharmacokinetics and tissue distribution, improved cellular delivery, and both reliable and controllable in vivo distribution.

WO 2014/144978 and EP 2623507A describe peptide-oligonucleotide conjugates. Sazani et al. Int. J. Toxicol. 2011, 30(2), 313-321 describes the antisense oligonucleotide AVI-4658 (eteplirsen). WO 2016/138534 (which was published after the priority date of the present application) also describes peptide-oligonucleotide conjugates. All of these differ from those of the present application, at least, in the linker group defined herein as $R^{12}$.

### SUMMARY

Provided herein are peptide-oligomer-conjugates. Also provided herein are peptide-oligomer-conjugates for use in treating a disease in a subject in need thereof.

Accordingly, in one aspect, provided herein is a peptide-oligonucleotide-conjugate of Formula I:

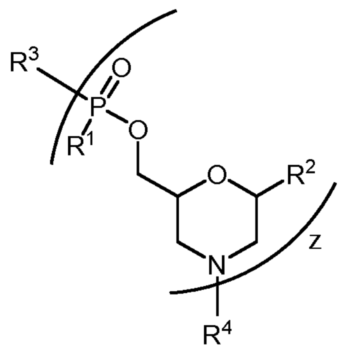

(I)

or a pharmaceutically acceptable salt thereof, wherein $R^1$, $R^2$, $R^3$, $R^4$, and z are as defined herein.

In one embodiment, the peptide-oligomer-conjugate of Formula I is a peptide-oligomer-conjugate of Formula Ia:

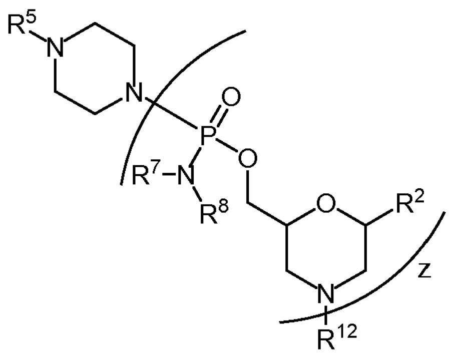

(Ia)

or a pharmaceutically acceptable salt thereof, wherein $R^2$, $R^5$, $R^7$, $R^8$, $R^{12}$, and z are as defined herein.

In another embodiment, the peptide-oligomer-conjugate of Formula I is a peptide-oligomer-conjugate of Formula Ib:

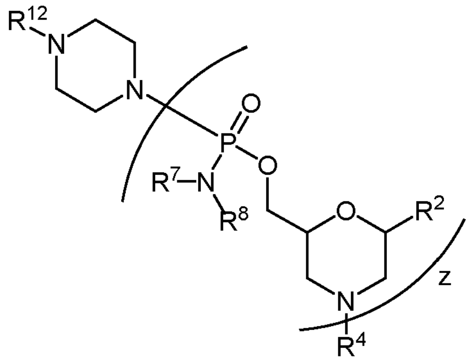

(Ib)

or a pharmaceutically acceptable salt thereof, wherein $R^2$, $R^4$, $R^7$, $R^8$, $R^{12}$, and z are as defined herein.

In yet another embodiment, the peptide-oligomer-conjugate of Formula I is a peptide-oligomer-conjugate of Formula Ic:

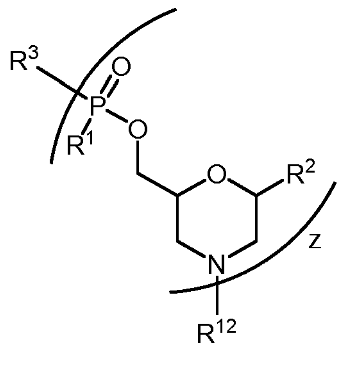

(Ic)

or a pharmaceutically acceptable salt thereof, wherein $R^1$, $R^2$, $R^3$, $R^{12}$, and z are as defined herein.

In another aspect, provided herein are peptide-oligomer-conjugates as defined above for use in treating a central nervous system disorder, a muscle disease, a viral infection, or a bacterial infection in a subject in need thereof.

**BRIEF DESCRIPTION OF THE FIGURES**

**Figure 1** shows a general synthetic scheme used to prepare PPMO-5 and PPMO-1.
**Figure 2** shows a general synthetic scheme used to prepare PPMO-4.
**Figure 3** shows the % exon 23 skipping of a PEG-3 linker has improved efficacy when compared with other linkers described herein (QC = quadriceps, HT = heart, DP = diaphragm).
**Figure 4** shows the % exon 23 skipping of an Apa linker or all-D amino acid have improved efficacy when compared with PPMO-8.
**Figure 5** compares PEG-3 linker compound efficacy compared to PPMO-2 and PPMO-8.
**Figure 6** shows BUN (blood urea nitrogen) levels at various dosing levels of peptide-oligomer-conjugates of the disclosure. BUN levels are increased when compared to PPMO-8.
**Figure 7** shows serum chemistry levels of ALT (alanine aminotransferase), alkaline phosphatase, triglycerides, creatinine, and AST (aspartate aminotransferase) at various dosing levels of peptide-oligomer-conjugates of the disclosure (dashed lines/shaded regions represent Average and SD, respectively, from internal untreated database).
**Figure 8** shows serum chemistry levels of chloride, phosphorous, potassium, and sodium at various dosing levels of peptide-oligomer-conjugates of the disclosure (dashed lines/shaded regions represent Average and SD, respectively, from internal untreated database).
**Figure 9** shows KIM-1 levels at various dosing levels of peptide-oligomer-conjugates of the disclosure (dashed lines/shaded regions represent Average and SD, respectively, from internal untreated database).
**Figure 10** compares the % exon 23 skipping and KIM-1 levels of PPMO-4, PPMO-2, and PPMO-8.

**DETAILED DESCRIPTION**

Provided herein are peptide-oligomer-conjugates. Also provided herein are peptide-oligomer-conjugates for use in treating a disease in a subject in need thereof. The oligomers, and thereby the peptide-oligomer-conjugates, described herein display stronger affinity for DNA and RNA without compromising sequence selectivity, relative to native or unmodified oligonucleotides. In some embodiments, the oligomers of the disclosure minimize or prevent cleavage by RNase H. In some embodiments, the antisense oligomers of the disclosure do not activate RNase H.
The peptides described herein impart to their corresponding peptide-oligomer-conjugates lower toxicity, enhance the activity of the oligomer, improve pharmacokinetics and tissue distribution, improve cellular delivery, and impart both reliable and controllable in vivo distribution.

*Definitions*

Listed below are definitions of various terms used to describe this disclosure. These definitions apply to the terms as they are used throughout this specification and claims, unless otherwise limited in specific instances, either individually or as part of a larger group.
The term "about" will be understood by persons of ordinary skill in the art and will vary to some extent on the context in which it is used. As used herein when referring to a measurable value such as an amount, a temporal duration, and the like, the term "about" is meant to encompass variations of ±20% or ±10%, including ±5%, ±1%, and ±0.1% from the specified value, as such variations are appropriate to perform the disclosed uses.
The term "alkyl" refers to saturated, straight- or branched-chain hydrocarbon moieties containing, in certain embodiments, between one and six, or one and eight carbon atoms, respectively. Examples of $C_{1-6}$-alkyl moieties include, but are not limited to, methyl, ethyl, propyl, isopropyl, *n*-butyl, *tert*-butyl, neopentyl, n-hexyl moieties; and examples of $C_{1-8}$-alkyl moieties include, but are not limited to, methyl, ethyl, propyl, isopropyl, *n*-butyl, *tert*-butyl, neopentyl, n-hexyl, heptyl, and octyl moieties.
The number of carbon atoms in an alkyl substituent can be indicated by the prefix "$C_{x-y}$," where x is the minimum and y is the maximum number of carbon atoms in the substituent. Likewise, a $C_x$ chain means an alkyl chain containing x carbon atoms.
The term "heteroalkyl" by itself or in combination with another term means, unless otherwise stated, a stable straight or branched chain alkyl group consisting of the stated number of carbon atoms and one or two heteroatoms selected from the group consisting of O, N, and S, and wherein the nitrogen and sulfur atoms may be optionally oxidized and the nitrogen heteroatom may be optionally quaternized. The heteroatom(s) may be placed at any position of the heteroalkyl group, including between the rest of the heteroalkyl group and the fragment to which it is attached, as well as attached to the most distal carbon atom in the heteroalkyl group. Examples include: $-O-CH_2-CH_2-CH_3$, $-CH_2-CH_2-CH_2-OH$, $-CH_2-CH_2-NH-CH_3$, $-CH_2-S-CH_2-CH_3$, and $-CH_2-CH_2-S(=O)-CH_3$. Up to two heteroatoms may be

consecutive, such as, for example, $-CH_2-NH-OCH_3$, or $-CH_2-CH_2-S-S-CH_3$.

The term "aryl," employed alone or in combination with other terms, means, unless otherwise stated, a carbocyclic aromatic system containing one or more rings (typically one, two, or three rings), wherein such rings may be attached together in a pendent manner, such as a biphenyl, or may be fused, such as naphthalene. Examples of aryl groups include phenyl, anthracyl, and naphthyl. In various embodiments, examples of an aryl group may include phenyl (e.g., $C_6$-aryl) and biphenyl (e.g., $C_{12}$-aryl). In some embodiments, aryl groups have from six to sixteen carbon atoms. In some embodiments, aryl groups have from six to twelve carbon atoms (e.g., $C_{6-12}$-aryl). In some embodiments, aryl groups have six carbon atoms (e.g., $C_6$-aryl).

As used herein, the term "heteroaryl" or "heteroaromatic" refers to a heterocycle having aromatic character. Heteroaryl substituents may be defined by the number of carbon atoms, e.g., $C_{1-9}$-heteroaryl indicates the number of carbon atoms contained in the heteroaryl group without including the number of heteroatoms. For example, a $C_{1-9}$-heteroaryl will include an additional one to four heteroatoms. A polycyclic heteroaryl may include one or more rings that are partially saturated. Non-limiting examples of heteroaryls include pyridyl, pyrazinyl, pyrimidinyl (including, e.g., 2- and 4-pyrimidinyl), pyridazinyl, thienyl, furyl, pyrrolyl (including, e.g., 2-pyrrolyl), imidazolyl, thiazolyl, oxazolyl, pyrazolyl (including, e.g., 3- and 5-pyrazolyl), isothiazolyl, 1,2,3-triazolyl, 1,2,4-triazolyl, 1,3,4-triazolyl, tetrazolyl, 1,2,3-thiadiazolyl, 1,2,3-oxadiazolyl, 1,3,4-thiadiazolyl and 1,3,4-oxadiazolyl.

Non-limiting examples of polycyclic heterocycles and heteroaryls include indolyl (including, e.g., 3-, 4-, 5-, 6- and 7-indolyl), indolinyl, quinolyl, tetrahydroquinolyl, isoquinolyl (including, e.g., 1- and 5-isoquinolyl), 1,2,3,4-tetrahydroisoquinolyl, cinnolinyl, quinoxalinyl (including, e.g., 2- and 5-quinoxalinyl), quinazolinyl, phthalazinyl, 1,8-naphthyridinyl, 1,4-benzodioxanyl, coumarin, dihydrocoumarin, 1,5-naphthyridinyl, benzofuryl (including, e.g., 3-, 4-, 5-, 6- and 7-benzofuryl), 2,3-dihydrobenzofuryl, 1,2-benzisoxazolyl, benzothienyl (including, e.g., 3-, 4-, 5-, 6-, and 7-benzothienyl), benzoxazolyl, benzothiazolyl (including, e.g., 2-benzothiazolyl and 5-benzothiazolyl), purinyl, benzimidazolyl (including, e.g., 2-benzimidazolyl), benzotriazolyl, thioxanthinyl, carbazolyl, carbolinyl, acridinyl, pyrrolizidinyl, and quinolizidinyl.

The term "protecting group" or "chemical protecting group" refers to chemical moieties that block some or all reactive moieties of a compound and prevent such reactive moieties from participating in chemical reactions until the protective group is removed, for example, those moieties listed and described in T.W. Greene, P.G.M. Wuts, Protective Groups in Organic Synthesis, 3rd ed. John Wiley & Sons (1999). It may be advantageous, where different protecting groups are employed, that each (different) protective group be removable by a different means. Protective groups that are cleaved under totally disparate reaction conditions allow differential removal of such protecting groups. For example, protective groups can be removed by acid, base, and hydrogenolysis. Groups such as trityl, monomethoxytrityl, dimethoxytrityl, acetal and tert-butyldimethylsilyl are acid labile and may be used to protect carboxy and hydroxy reactive moieties in the presence of amino groups protected with Cbz groups, which are removable by hydrogenolysis, and Fmoc groups, which are base labile. Carboxylic acid moieties may be blocked with base labile groups such as, without limitation, methyl, or ethyl, and hydroxy reactive moieties may be blocked with base labile groups such as acetyl in the presence of amines blocked with acid labile groups such as tert-butyl carbamate or with carbamates that are both acid and base stable but hydrolytically removable.

Carboxylic acid and hydroxyl reactive moieties may also be blocked with hydrolytically removable protective groups such as the benzyl group, while amine groups may be blocked with base labile groups such as Fmoc. A particulary useful amine protecting group for the synthesis of compounds of Formula (I) is the trifluoroacetamide. Carboxylic acid reactive moieties may be blocked with oxidatively-removable protective groups such as 2,4-dimethoxybenzyl, while coexisting amino groups may be blocked with fluoride labile silyl carbamates.

Allyl blocking groups are useful in the presence of acid- and base-protecting groups since the former are stable and can be subsequently removed by metal or pi-acid catalysts. For example, an allyl-blocked carboxylic acid can be deprotected with a palladium(0)-catalyzed reaction in the presence of acid labile t-butyl carbamate or base-labile acetate amine protecting groups. Yet another form of protecting group is a resin to which a compound or intermediate may be attached. As long as the residue is attached to the resin, that functional group is blocked and cannot react. Once released from the resin, the functional group is available to react.

The term "nucleobase," "base-pairing moiety," "nucleobase-pairing moiety," or "base" refers to the heterocyclic ring portion of a nucleoside, nucleotide, and/or morpholino subunit. Nucleobases may be naturally occurring, or may be modified or analogs of these naturally occurring nucleobases, e.g., one or more nitrogen atoms of the nucleobase may be independently at each occurrence replaced by carbon. Exemplary analogs include hypoxanthine (the base component of the nucleoside inosine); 2, 6-diaminopurine; 5-methyl cytosine; C5-propynyl-modified pyrimidines; 10-(9-(aminoethoxy)phenoxazinyl) (G-clamp) and the like.

Further examples of base-pairing moieties include, but are not limited to, uracil, thymine, adenine, cytosine, guanine and hypoxanthine (inosine) having their respective amino groups protected by acyl protecting groups, 2-fluorouracil, 2-fluorocytosine, 5-bromouracil, 5-iodouracil, 2,6-diaminopurine, azacytosine, pyrimidine analogs such as pseudoisocytosine and pseudouracil and other modified nucleobases such as 8-substituted purines, xanthine, or hypoxanthine (the latter two being the natural degradation products). The modified nucleobases disclosed in Chiu and Rana, RNA,

2003, 9, 1034-1048, Limbach et al. Nucleic Acids Research, 1994, 22, 2183-2196 and Revankar and Rao, Comprehensive Natural Products Chemistry, vol. 7, 313, are also contemplated.

Further examples of base-pairing moieties include, but are not limited to, expanded-size nucleobases in which one or more benzene rings has been added. Nucleic base replacements described in the Glen Research catalog (www.glenresearch.com); Krueger AT et al., Acc. Chem. Res., 2007, 40, 141-150; Kool, ET, Acc. Chem. Res., 2002, 35, 936-943; Benner S.A., et al., Nat. Rev. Genet., 2005, 6, 553-543; Romesberg, F.E., et al., Curr. Opin. Chem. Biol., 2003, 7, 723-733; Hirao, I., Curr. Opin. Chem. Biol., 2006, 10, 622-627, , are contemplated as useful for the synthesis of the oligomers described herein. Examples of expanded-size nucleobases are shown below:

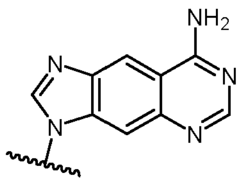
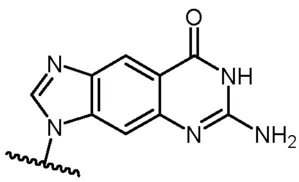
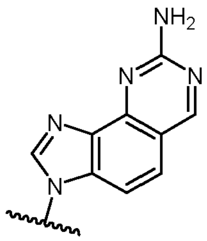
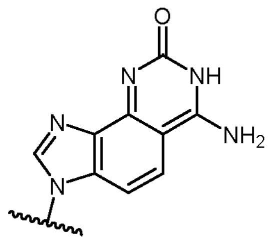

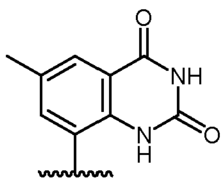
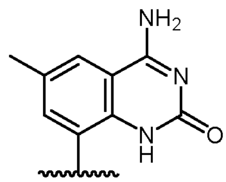
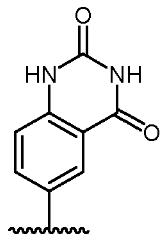
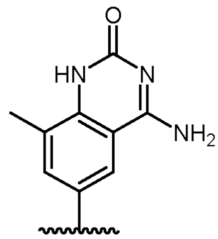

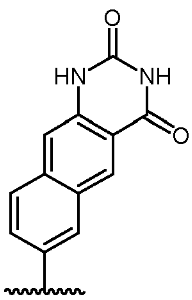
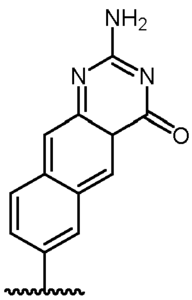

The terms "oligonucleotide" or "oligomer" refer to a compound comprising a plurality of linked nucleosides, nucleotides, or a combination of both nucleosides and nucleotides. In specific embodiments provided herein, an oligonucleotide is a morpholino oligonucleotide.

The phrase "morpholino oligonucleotide" or "PMO" refers to a modified oligonucleotide having morpholino subunits linked together by phosphoramidate or phosphorodiamidate linkages, joining the morpholino nitrogen of one subunit to the 5'-exocyclic carbon of an adjacent subunit. Each morpholino subunit comprises a nucleobase-pairing moiety effective to bind, by nucleobase-specific hydrogen bonding, to a nucleobase in a target.

The terms "antisense oligomer," "antisense compound" and "antisense oligonucleotide" are used interchangeably and refer to a sequence of subunits, each bearing a base-pairing moiety, linked by intersubunit linkages that allow the base-pairing moieties to hybridize to a target sequence in a nucleic acid (typically an RNA) by Watson-Crick base pairing, to form a nucleic acid: oligomer heteroduplex within the target sequence. The oligomer may have exact (perfect) or near (sufficient) sequence complementarity to the target sequence; variations in sequence near the termini of an oligomer are generally preferable to variations in the interior.

Such an antisense oligomer can be designed to block or inhibit translation of mRNA or to inhibit/alter natural or abnormal pre-mRNA splice processing, and may be said to be "directed to" or "targeted against" a target sequence with which it hybridizes. The target sequence is typically a region including an AUG start codon of an mRNA, a Translation Suppressing Oligomer, or splice site of a pre-processed mRNA, a Splice Suppressing Oligomer (SSO). The target sequence for a splice site may include an mRNA sequence having its 5' end 1 to about 25 base pairs downstream of a normal splice acceptor junction in a preprocessed mRNA. In various embodiments, a target sequence may be any region of a preprocessed mRNA that includes a splice site or is contained entirely within an exon coding sequence or spans a

splice acceptor or donor site. An oligomer is more generally said to be "targeted against" a biologically relevant target, such as a protein, virus, or bacteria, when it is targeted against the nucleic acid of the target in the manner described above.

The antisense oligonucleotide and the target RNA are complementary to each other when a sufficient number of corresponding positions in each molecule are occupied by nucleotides which can hydrogen bond with each other, such that stable and specific binding occurs between the oligonucleotide and the target. Thus, "specifically hybridizable" and "complementary" are terms which are used to indicate a sufficient degree of complementarity or precise pairing such that stable and specific binding occurs between the oligonucleotide and the target. It is understood in the art that the sequence of an oligonucleotide need not be 100% complementary to that of its target sequence to be specifically hybridizable. An oligonucleotide is specifically hybridizable when binding of the oligonucleotide to the target molecule interferes with the normal function of the target RNA, and there is a sufficient degree of complementarity to avoid non-specific binding of the antisense oligonucleotide to non-target sequences under conditions in which specific binding is desired, i.e., under physiological conditions in the case of in vivo assays or therapeutic treatment, and in the case of in vitro assays, under conditions in which the assays are performed.

Oligonucleotides may also include nucleobase (often referred to in the art simply as "base") modifications or substitutions. Oligonucleotides containing a modified or substituted base include oligonucleotides in which one or more purine or pyrimidine bases most commonly found in nucleic acids are replaced with less common or non-natural bases. In some embodiments, the nucleobase is covalently linked at the N9 atom of the purine base, or at the N1 atom of the pyrimidine base, to the morpholine ring of a nucleotide or nucleoside.

Purine bases comprise a pyrimidine ring fused to an imidazole ring, as described by the general formula:

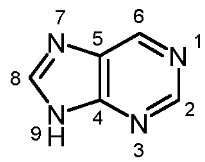

Purine

Adenine and guanine are the two purine nucleobases most commonly found in nucleic acids. These may be substituted with other naturally-occurring purines, including but not limited to N6-methyladenine, N2-methylguanine, hypoxanthine, and 7-methylguanine.

Pyrimidine bases comprise a six-membered pyrimidine ring as described by the general formula:

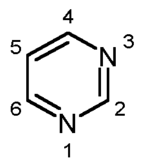

Pyrimidine

Cytosine, uracil, and thymine are the pyrimidine bases most commonly found in nucleic acids. These may be substituted with other naturally-occurring pyrimidines, including but not limited to 5-methylcytosine, 5-hydroxymethylcytosine, pseudouracil, and 4-thiouracil. In one embodiment, the oligonucleotides described herein contain thymine bases in place of uracil.

Other modified or substituted bases include, but are not limited to, 2,6-diaminopurine, orotic acid, agmatidine, lysidine, 2-thiopyrimidine (e.g. 2-thiouracil, 2-thiothymine), G-clamp and its derivatives, 5-substituted pyrimidine (e.g. 5-halouracil, 5-propynyluracil, 5-propynylcytosine, 5-aminomethyluracil, 5-hydroxymethyluracil, 5-aminomethylcytosine, 5-hydroxymethylcytosine, Super T), 7-deazaguanine, 7-deazaadenine, 7-aza-2,6-diaminopurine, 8-aza-7-deazaguanine, 8-aza-7-deazaadenine, 8-aza-7-deaza-2,6-diaminopurine, Super G, Super A, and N4-ethylcytosine, or derivatives thereof; N2-cyclopentylguanine (cPent-G), N2-cyclopentyl-2-aminopurine (cPent-AP), and N2-propyl-2-aminopurine (Pr-AP), pseudouracil or derivatives thereof; and degenerate or universal bases, like 2,6-difluorotoluene or absent bases like abasic sites (e.g. 1-deoxyribose, 1,2-dideoxyribose, 1-deoxy-2-O-methylribose; or pyrrolidine derivatives in which the ring oxygen has been replaced with nitrogen (azaribose)). Pseudouracil is a naturally occuring isomerized version of uracil, with a C-glycoside rather than the regular N-glycoside as in uridine.

Certain modified or substituted nucleobases are particularly useful for increasing the binding affinity of the antisense oligonucleotides of the disclosure. These include 5-substituted pyrimidines, 6-azapyrimidines and N-2, N-6 and 0-6 substituted purines, including 2-aminopropyladenine, 5-propynyluracil and 5-propynylcytosine. In various embodiments, nucleobases may include 5-methylcytosine substitutions, which have been shown to increase nucleic acid duplex stability by 0.6-1.2°C.

In some embodiments, modified or substituted nucleobases are useful for facilitating purification of antisense oligonucleotides. For example, in certain embodiments, antisense oligonucleotides may contain three or more (e.g. , 3, 4, 5, 6 or more) consecutive guanine bases. In certain antisense oligonucleotides, a string of three or more consecutive guanine bases can result in aggregation of the oligonucleotides, complicating purification. In such antisense oligonucleotides, one or more of the consecutive guanines can be substituted with inosine. The substitution of inosine for one or more guanines in a string of three or more consecutive guanine bases can reduce aggregation of the antisense oligonucleotide, thereby facilitating purification.

The oligonucleotides provided herein are synthesized and do not include antisense compositions of biological origin. The molecules of the disclosure may also be mixed, encapsulated, conjugated or otherwise associated with other molecules, molecule structures or mixtures of compounds, as for example, liposomes, receptor targeted molecules, oral, rectal, topical or other formulations, for assisting in uptake, distribution, or absorption, or a combination thereof.

The terms "complementary" and "complementarity" refer to oligonucleotides (i.e., a sequence of nucleotides) related by Watson-Crick base-pairing rules. For example, the sequence "T-G-A (5'-3')," is complementary to the sequence "T-C-A (5'-3')." Complementarity may be "partial," in which only some of the nucleic acids' bases are matched according to base pairing rules. Or, there may be "complete," "total," or "perfect" (100%) complementarity between the nucleic acids. The degree of complementarity between nucleic acid strands has significant effects on the efficiency and strength of hybridization between nucleic acid strands. While perfect complementarity is often desired, some embodiments can include one or more but preferably 6, 5, 4, 3, 2, or 1 mismatches with respect to the target RNA. Such hybridization may occur with "near" or "substantial" complementarity of the antisense oligomer to the target sequence, as well as with exact complementarity. In some embodiments, an oligomer may hybridize to a target sequence at about 50%, 55%, 60%, 65%, 70%, 75%, 80%, 85%, 90%, 95%, 99% or 100% complementarity. Variations at any location within the oligomer are included. In certain embodiments, variations in sequence near the termini of an oligomer are generally preferable to variations in the interior, and if present are typically within about 6, 5, 4, 3, 2, or 1 nucleotides of the 5'-terminus, 3'-terminus, or both termini.

The terms "TEG," "EG3," or "triethylene glycol tail" refer to triethylene glycol moieties conjugated to the oligomer, e.g., at its 3'- or 5'-end. For example, in some embodiments, "TEG" includes, for example, wherein $R^3$ of the peptide-oligomer-conjugate of Formula (I) or (Ic) is of the formula:

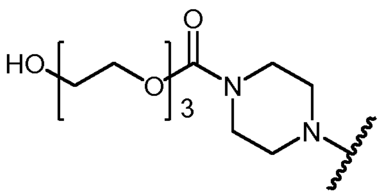

.

The term "peptide" refers to a compound comprising a plurality of linked amino acids. The peptides provided herein can be considered to be cell-penetrating peptides.

The terms "cell-penetrating peptide" and "CPP" are used interchangeably and refer to cationic cell-penetrating peptides, also called transport peptides, carrier peptides, or peptide transduction domains. The peptides, provided herein, have the capability of inducing cell penetration into 100% of cells of a given cell culture population and allow macromolecular translocation within multiple tissues in vivo upon systemic administration. In various embodiments, a CPP embodiment of the disclosure may include an arginine-rich peptide as described further below.

The term "treatment" refers to the application of one or more specific procedures used for the amelioration of a disease. In certain embodiments, the specific procedure is the administration of one or more pharmaceutical agents. "Treatment" of an individual (e.g. a mammal, such as a human) or a cell is any type of intervention used in an attempt to alter the natural course of the individual or cell. Treatment includes, but is not limited to, administration of a pharmaceutical composition, and may be performed either prophylactically or subsequent to the initiation of a pathologic event or contact with an etiologic agent. In certain embodiments, treatment includes, but is not limited to, administration of a pharmaceutical composition, and may be performed subsequent to the initiation of a pathologic event or contact with an etiologic agent. Treatment includes any desirable effect on the symptoms or pathology of a disease or condition, and may include, for example, minimal changes or improvements in one or more measurable markers of the disease or condition being treated. Also included are "prophylactic" treatments, which can be directed to reducing the rate of progression of the disease or condition being treated, delaying the onset of that disease or condition, or reducing the severity of its onset. An "effective amount" or "therapeutically effective amount" refers to an amount of therapeutic compound, such as an antisense oligomer, administered to a mammalian subject, either as a single dose or as part of

a series of doses, which is effective to produce a desired therapeutic effect.

The term "amelioration" means a lessening of severity of at least one indicator of a condition or disease. In certain embodiments, amelioration includes a delay or slowing in the progression of one or more indicators of a condition or disease. The severity of indicators may be determined by subjective or objective measures which are known to those skilled in the art.

As used herein, "pharmaceutically acceptable salts" refers to derivatives of the disclosed oligonucleotides wherein the parent oligonucleotide is modified by converting an existing acid or base moiety to its salt form. Lists of suitable salts are found in Remington's Pharmaceutical Sciences, 17th ed., Mack Publishing Company, Easton, Pa., 1985, p. 1418 and Journal of Pharmaceutical Science, 66, 2 (1977).

*Peptide-oligomer-conjugates*

Provided herein are oligomers (e.g., antisense compound) chemically linked to one or more moieties, such as a cell-penetrating peptide, that enhance the activity, cellular distribution, or cellular uptake of the oligomer. The oligomers can additionally be chemically linked to one or more heteroalkyl moieties (e.g., polyethylene glycol) that further enhance the activity, cellular distribution, or cellular uptake of the oligomer. In one exemplary embodiment, an arginine-rich polypeptide is covalently coupled at its N-terminal or C-terminal residue to either end of the antisense compound, or internally to the antisense compound.

Thus, in one aspect, provided herein is a peptide-oligomer-conjugate of Formula I:

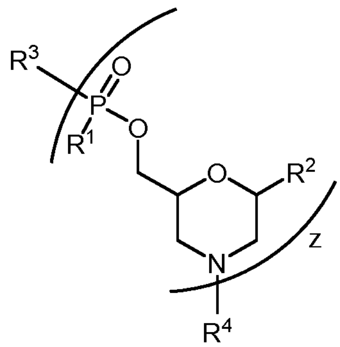

(I)

or a pharmaceutically acceptable salt thereof,
wherein:

$R^3$ is selected from OH, $-N(H)CH_2C(O)NH_2$, $-N(C_{1-6}\text{-alkyl})CH_2C(O)NH_2$,

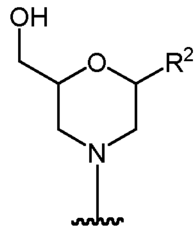

, and

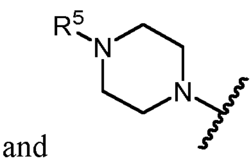

;

$R^5$ is $-C(O)(O\text{-alkyl})_xOH$, wherein x is 3-10 and each alkyl group is, independently at each occurrence, $C_{2-6}$-alkyl, or $R^5$ is selected from the group consisting of $-C(O)C_{1-6}$ alkyl, trityl, monomethoxytrityl, $-(C_{1-6}\text{-alkyl})R^6$, $-(C_{1-6}$ heteroalkyl)-$R^6$, aryl-$R^6$, heteroaryl-$R^6$, $-C(O)O-(C_{1-6}$ alkyl)-$R^6$, $-C(O)O$-aryl-$R^6$, $-C(O)O$-heteroaryl-$R^6$, and $R^{12}$;

$R^6$ is selected from OH, SH, and $NH_2$, or $R^6$ is O, S, or NH, covalently linked to a solid support;

$R^1$ is, independently at each occurrence, OH, $-NR^7R^{12}$, or $-NR^7R^8$;

each $R^7$ and $R^8$ are, independently at each occurrence, H or $-C_{1-6}$ alkyl;

$R^2$ is, independently at each occurrence, selected from the group consisting of H, a nucleobase and a nucleobase functionalized with a chemical protecting-group, wherein the nucleobase, independently at each occurrence, comprises a $C_{3-6}$ heterocyclic ring selected from pyridine, pyrimidine, triazinane, purine, and deaza-purine;

z is 8-40;

$R^4$ is selected from H, $-C_{1-6}$ alkyl, $-C(O)C_{1-6}$ alkyl, benzoyl, stearoyl, trityl, monomethoxytrityl, dimethoxytrityl, trimethoxytrityl,

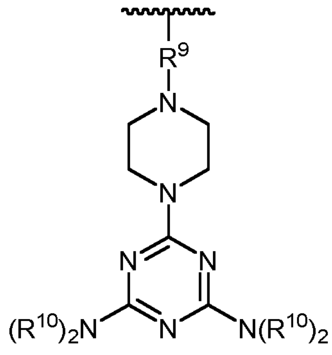, and $R^{12}$;

$R^9$ is $-C(O)(CH_2)_6C(O)-$ or $-C(O)(CH_2)_2S_2(CH_2)_2C(O)-$;
$R^{10}$ is $-(CH_2)_2OC(O)N((CH_2)_6N(H)C(=NH)NH_2)_2$;
$R^{11}$ is selected from OH and $-NR^7R^8$;
$R^{12}$ is selected from the group consisting of:

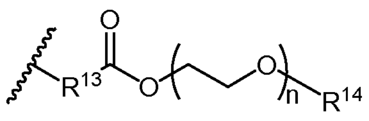, 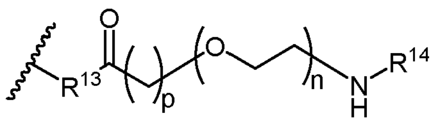,

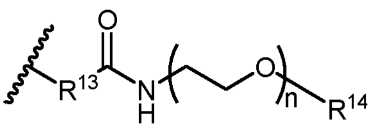, 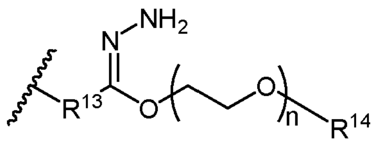,

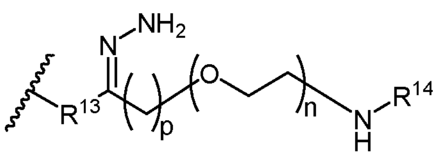, 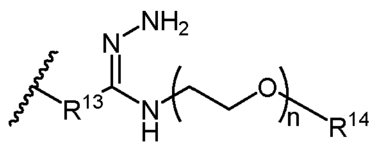,

, and

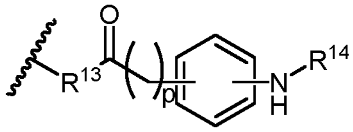;

n is 1, 2, 3, 4, 5, 6, 7, 8, 9, or 10;
p is 2, 3, 4, or 5;
$R^{13}$ is a bond, or $R^{13}$ is selected from the group consisting of:

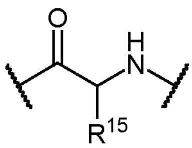, 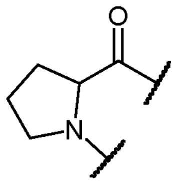, 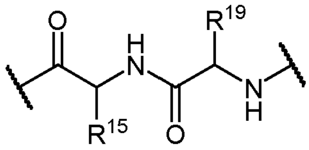,

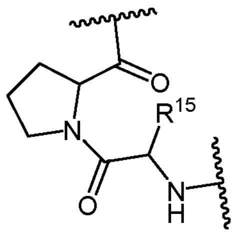, 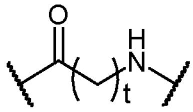, and 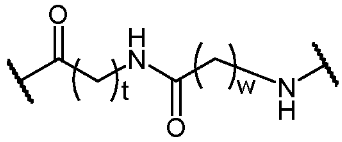;

$R^{15}$ and $R^{19}$ are, independently at each occurrence, selected from the group consisting of H, $-C_{1-4}$ alkyl, $-CH(-C_{1-4}$ alkyl$)_2$, and $-(CH_2)_3NH-C(=NH)-NH_2$;
t and w are, independently at each occurrence, 2, 3, 4, or 5;
$R^{14}$ is selected from the group consisting of:

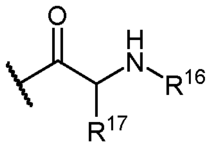, 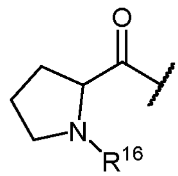, 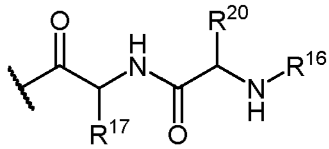,

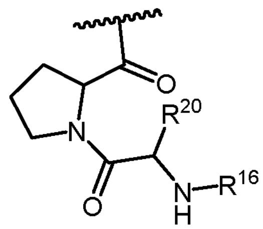, 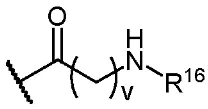, 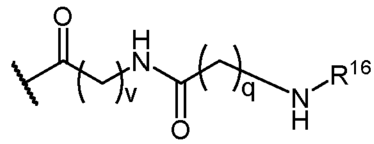,

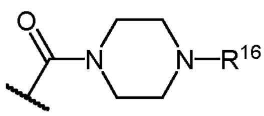, 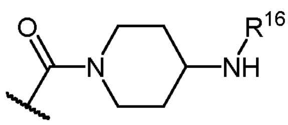, and 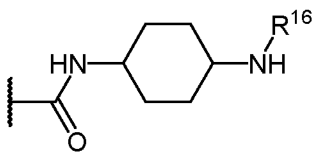;

$R^{17}$ is H or $-C_{1-4}$ alkyl;
$R^{20}$ is selected from the group consisting of H, $-C_{1-4}$ alkyl, $-CH(-C_{1-4}$ alkyl$)_2$, and $-(CH_2)_3NH-C(=NH)-NH_2$;
v and q are, independently at each occurrence, 2, 3, 4, or 5;
$R^{16}$ is selected from the group consisting of:

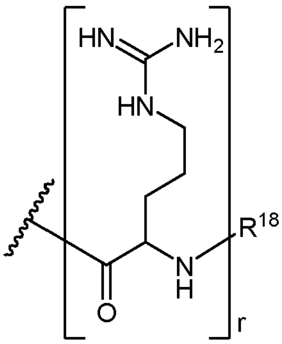,
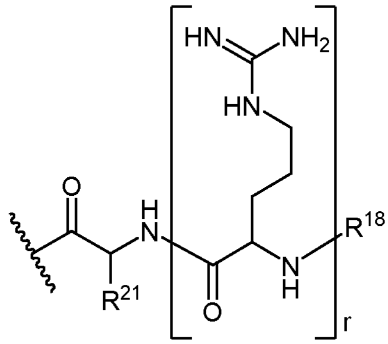,
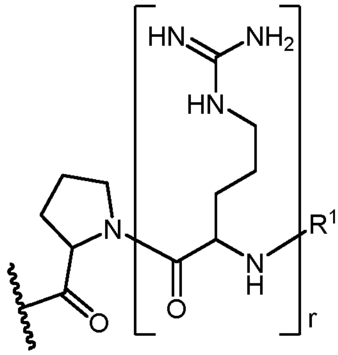,

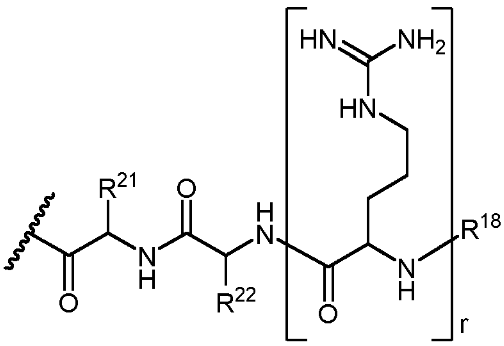,
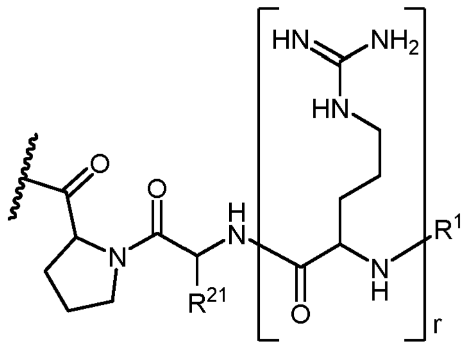,

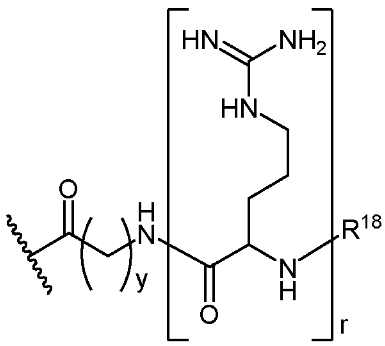, and
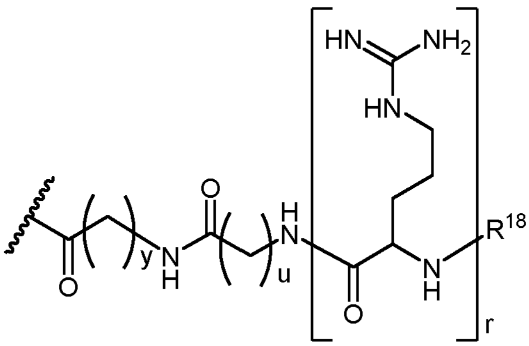;

$R^{21}$ and $R^{22}$ are, independently at each occurrence, H or $-C_{1-4}$ alkyl;
$R^{18}$ is selected from the group consisting of H, $-C(O)C_{1-6}$ alkyl, benzoyl, and stearoyl;
r is 1, 2, 3, 4, 5, 6, 7, 8, or 9; and
y and u are, independently at each occurrence, 2, 3, 4, or 5;
provided that any one of the following conditions is present: 1) $R^1$ is $NR^7R^{12}$; or 2) $R^4$ is $R^{12}$; or 3) $R^3$ is

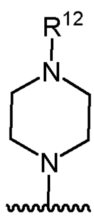.

In an embodiment of the peptide-oligomer-conjugate of Formula I, $R^4$ is selected from H, $-C_{1-6}$ alkyl, $-C(O)C_{1-6}$ alkyl, benzoyl, stearoyl, trityl, monomethoxytrityl, dimethoxytrityl, trimethoxytrityl, and $R^{12}$.

In another embodiment, $R^3$ is

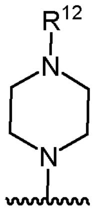, or

$R^4$ is $R^{12}$.

In yet another embodiment, $R^3$ is selected from -OH, $-N(C_{1-6}\text{-alkyl})CH_2C(O)NH_2$,

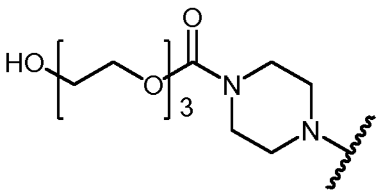, 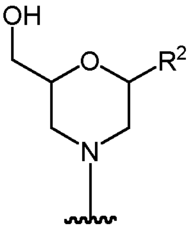, and 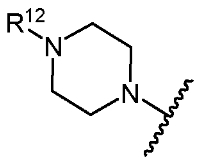.

In still another embodiment, $R^4$ is selected from H, $-C(O)CH_3$, trityl, 4-methoxytrityl, benzoyl, stearoyl, and $R^{12}$.

In another embodiment, $R^3$ is selected from -OH, $-N(C_{1-6}\text{-alkyl})CH_2C(O)NH_2$, and

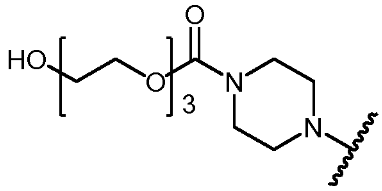; and

$R^4$ is $R^{12}$.

In another embodiment, $R^3$ is

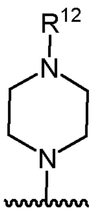.

In another embodiment, $R^4$ is selected from H, $-C(O)CH_3$, trityl, 4-methoxytrityl, benzoyl, and stearoyl.

In another embodiment, $R^4$ is selected from H and $-C(O)CH_3$.

In another embodiment, the peptide-oligomer-conjugate of Formula I is a peptide-oligomer-conjugate of Formula Ia:

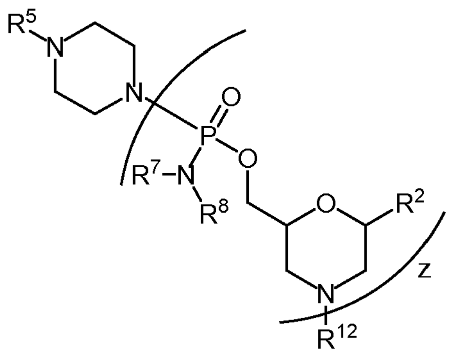

(Ia),

wherein $R^5$ is -C(O)(O-alkyl)$_x$OH, wherein x is 3-10 and each alkyl group is, independently at each occurrence, $C_{2-6}$-alkyl, or $R^5$ is selected from the group consisting of -C(O)$C_{1-6}$ alkyl, trityl, and monomethoxytrityl.

In an embodiment of the peptide-oligomer-conjugates of Formula I or Formula Ia, $R^5$ is -C(O)(O-alkyl)$_x$OH, wherein each alkyl group is, independently at each occurrence, $C_{2-6}$-alkyl.

In another embodiment of the peptide-oligomer-conjugates of Formula I or Formula Ia, $R^5$ is -C(O)(O-$CH_2CH_2)_3$OH.

In yet another embodiment, the peptide-oligomer-conjugate of Formula I is a peptide-oligomer-conjugate of Formula Ib:

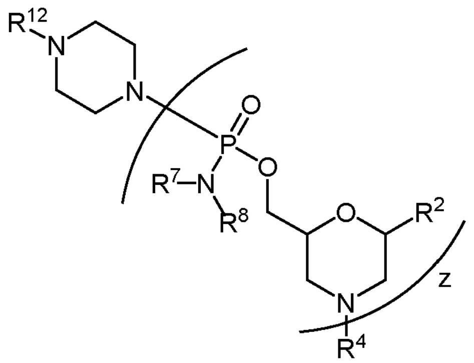

(Ib),

wherein $R^4$ is selected from H, -$C_{1-6}$ alkyl, -C(O)$C_{1-6}$ alkyl, benzoyl, stearoyl, trityl, monomethoxytrityl, dimethoxytrityl, and trimethoxytrityl.

In an embodiment of the peptide-oligomer-conjugate of Formula I or Formula Ib, $R^4$ is selected from H, $C_{1-6}$ alkyl, -C(O)$CH_3$, benzoyl, and stearoyl.

In an embodiment of the peptide-oligomer-conjugate of Formula Ib, $R^4$ is selected from H and -C(O)$CH_3$.

In an embodiment of the peptide-oligomer-conjugates of the disclosure, $R^{16}$ is selected from the group consisting of:

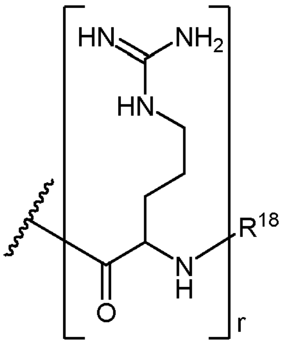
,
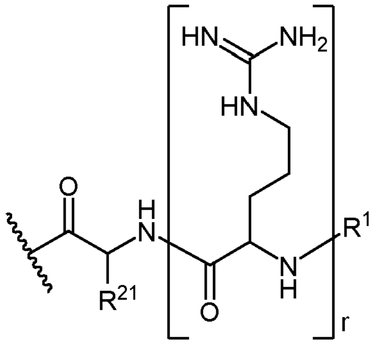
,

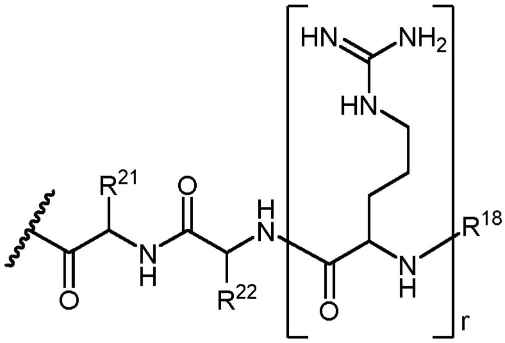
, and
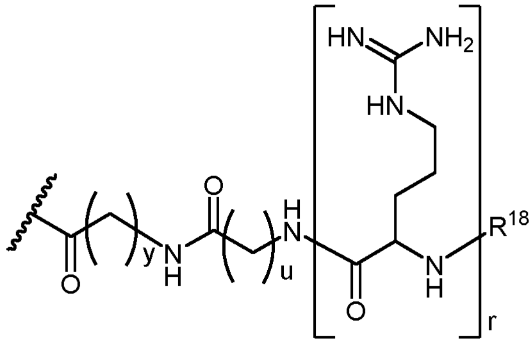
.

In another embodiment of the peptide-oligomer-conjugates of the disclosure, $R^{16}$ is

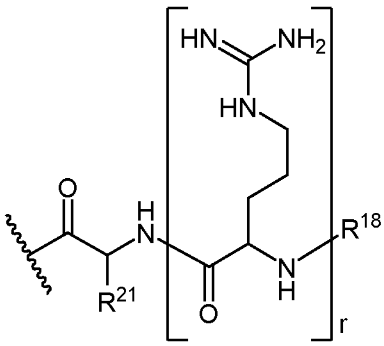
.

In another embodiment of the peptide-oligomer-conjugates of the disclosure, $R^{14}$ is selected from the group consisting of:

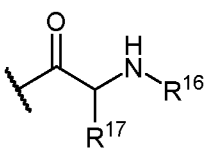
,
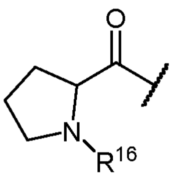
, and
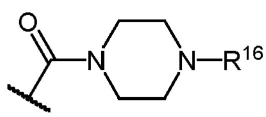
.

In another embodiment of the peptide-oligomer-conjugates of the disclosure, $R^{12}$ is

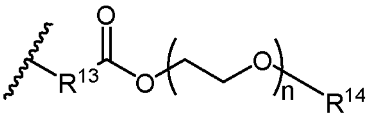 or 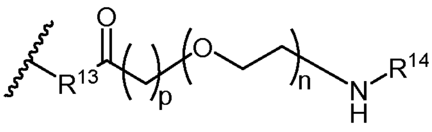 .

In another embodiment of the peptide-oligomer-conjugates of the disclosure, r is 3, 4, 5, 6, 7, or 8.

In another embodiment of the peptide-oligomer-conjugates of the disclosure, r is 5, 6, or 7.

In another embodiment of the peptide-oligomer-conjugates of the disclosure, r is 6.

In another embodiment of the peptide-oligomer-conjugates of the disclosure, $R^{13}$ is a bond.

In another embodiment of the peptide-oligomer-conjugates of the disclosure, z is 8-25.

In another embodiment of the peptide-oligomer-conjugates of the disclosure, z is 15-25.

In another embodiment of the peptide-oligomer-conjugates of the disclosure, z is 10-20.

In another embodiment of the peptide-oligomer-conjugates of the disclosure, each $R^1$ is independently $NR^7R^8$, wherein each $R^7$ and $R^8$ are, independently at each occurrence, $C_{1-3}$-alkyl.

In another embodiment of the peptide-oligomer-conjugates of the disclosure, each $R^1$ is $N(CH_3)_2$.

In another embodiment of the peptide-oligomer-conjugates of the disclosure, each $R^2$ is a nucleobase, wherein the nucleobase, independently at each occurrence, comprises a $C_{4-6}$-heterocyclic ring selected from pyridine, pyrimidine, triazinane, purine, and deaza-purine.

In another embodiment of the peptide-oligomer-conjugates of the disclosure, each $R^2$ is a nucleobase, wherein the nucleobase, independently at each occurrence, comprises a $C_{4-6}$-heterocyclic ring selected from pyrimidine, purine, and deaza-purine.

In another embodiment of the peptide-oligomer-conjugates of the disclosure, each $R^2$ is a nucleobase, wherein the nucleobase, independently at each occurrence, is selected from the group consisting of adenine, 2,6-diaminopurine, 7-deaza-adenine, guanine, 7-deaza-guanine, hypoxanthine, cytosine, 5-methyl-cytosine, thymine, and uracil.

In another embodiment of the peptide-oligomer-conjugates of the disclosure, each $R^2$ is a nucleobase, wherein the nucleobase, independently at each occurrence, is selected from the group consisting of adenine, guanine, cytosine, 5-methyl-cytosine, thymine, uracil, and hypoxanthine.

In another embodiment of the peptide-oligomer-conjugates of the disclosure, $R^{15}$ is selected from the group consisting of H, $CH_3$, $-CH(CH_3)_2$, and $-(CH_2)_3NH-C(=NH)-NH_2$.

In another embodiment of the peptide-oligomer-conjugates of the disclosure, $R^{19}$ is selected from the group consisting of H, $CH_3$, $-CH(CH_3)_2$, and $-(CH_2)_3NH-C(=NH)-NH_2$.

In another embodiment of the peptide-oligomer-conjugates of the disclosure, $R^{20}$ is selected from the group consisting of H, $CH_3$, $-CH(CH_3)_2$, and $-(CH_2)_3NH-C(=NH)-NH_2$.

In another embodiment of the peptide-oligomer-conjugates of the disclosure, $R^{17}$ is H or $CH_3$.

In another embodiment of the peptide-oligomer-conjugates of the disclosure, $R^{17}$ is H.

In another embodiment of the peptide-oligomer-conjugates of the disclosure, $R^{21}$ is H or $CH_3$.

In another embodiment of the peptide-oligomer-conjugates of the disclosure, $R^{21}$ is H.

In another embodiment of the peptide-oligomer-conjugates of the disclosure, $R^{22}$ is H or $CH_3$.

In another embodiment of the peptide-oligomer-conjugates of the disclosure, $R^{22}$ is H.

In another embodiment of the peptide-oligomer-conjugates of the disclosure, $R^6$ is selected from OH, SH, and $NH_2$.

In another embodiment of the peptide-oligomer-conjugates of the disclosure, each $R^7$ and $R^8$ are, independently at each occurrence, H or $CH_3$.

In another embodiment of the peptide-oligomer-conjugates of the disclosure, each $R^7$ and $R^8$ are $CH_3$.

In another embodiment of the peptide-oligomer-conjugates of the disclosure, n is 2, 3, 4, 5, 6, or 7.

In another embodiment of the peptide-oligomer-conjugates of the disclosure, p is 3 or 4.

In another embodiment of the peptide-oligomer-conjugates of the disclosure, t is 3 or 4.

In another embodiment of the peptide-oligomer-conjugates of the disclosure, w is 3 or 4.

In another embodiment of the peptide-oligomer-conjugates of the disclosure, v is 3 or 4.

In another embodiment of the peptide-oligomer-conjugates of the disclosure, x is 3 or 4.

In another embodiment of the peptide-oligomer-conjugates of the disclosure, y is 3 or 4.

In another embodiment of the peptide-oligomer-conjugates of the disclosure, u is 3 or 4.

In another embodiment of the peptide-oligomer-conjugates of the disclosure, q is 3 or 4.

In another embodiment of the peptide-oligomer-conjugates of the disclosure, $R^{18}$ is selected from H, $-C(O)C_1-C_3$ alkyl, benzoyl, and stearoyl.

In another embodiment of the peptide-oligomer-conjugates of the disclosure, $R^{18}$ is H or $-C(O)C_1-C_3$ alkyl.

In another embodiment of the peptide-oligomer-conjugates of the disclosure, $R^{18}$ is H or $-C(O)CH_3$.

In another embodiment, the peptide-oligomer-conjugate of Formula I is a peptide-oligomer-conjugate of Formula Ic:

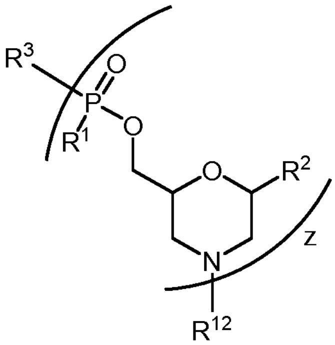

(Ic)

or a pharmaceutically acceptable salt thereof,
wherein:

$R^3$ is OH,

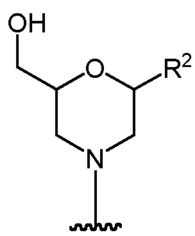

, or

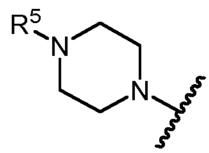

;

$R^5$ is -C(O)(O-alkyl)$_x$OH, wherein x is 3-10 and each alkyl group is, independently at each occurrence, $C_{2-6}$-alkyl, or $R^5$ is -C(O)$C_{1-6}$ alkyl;
$R^1$ is, independently at each occurrence, OH or -$NR^7R^8$;
each $R^7$ and $R^8$ are independently at each occurrence -$C_{1-6}$ alkyl;
$R^2$ is, independently at each occurrence, selected from the group consisting of H, adenine, 2,6-diaminopurine, 7-deaza-adenine, guanine, 7-deaza-guanine, hypoxanthine, cytosine, 5-methyl-cytosine, thymine, and uracil;
z is 8-40;
$R^{12}$ is selected from the group consisting of:

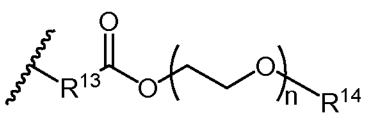

,

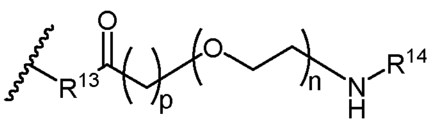

,

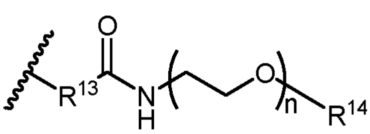

, and

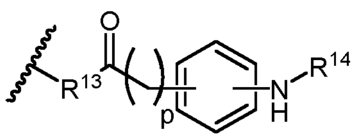

;

n is 1, 2, 3, 4, 5, 6, 7, 8, 9, or 10;
p is 2, 3, 4, or 5;
$R^{13}$ is a bond;
$R^{14}$ is selected from the group consisting of:

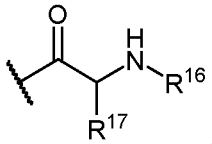, 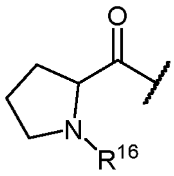, and 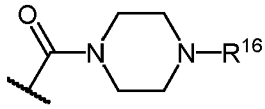;

$R^{17}$ is H or $-C_{1-4}$ alkyl;

$R^{16}$ is selected from the group consisting of:

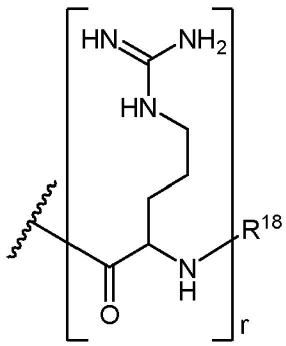, 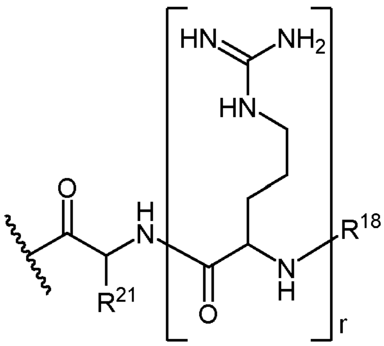, and 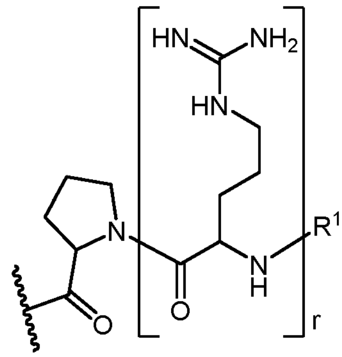;

$R^{21}$ is H or $-C_{1-4}$ alkyl;

$R^{18}$ is of H or $-C(O)C_{1-6}$ alkyl; and

r is 1, 2, 3, 4, 5, 6, 7, 8, or 9.

In an embodiment of the peptide-oligomer-conjugate of Formula Ic, $R^3$ is

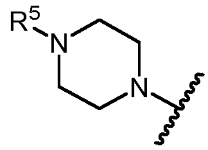;

and $R^5$ is $-C(O)(O-C_{2-6}\text{-alkyl})_3OH$ or $-C(O)C_{1-6}$ alkyl.

In another embodiment of the peptide-oligomer-conjugate of Formula Ic, $R^1$ is, independently at each occurrence, OH or $—N(C_{1-6}\text{ alkyl})_2$.

In another embodiment of the peptide-oligomer-conjugate of Formula Ic, $R^2$, independently at each occurrence, is selected from the group consisting of adenine, guanine, cytosine, 5-methyl-cytosine, thymine, uracil, and hypoxanthine.

In another embodiment of the peptide-oligomer-conjugates of the disclosure, $R^{14}$ is

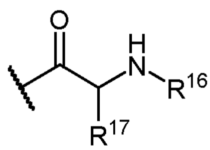 or 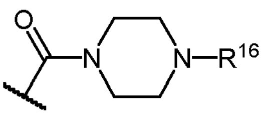;

and $R^{17}$ is H.

In another embodiment of the peptide-oligomer-conjugates of the disclosure, $R^{16}$ is

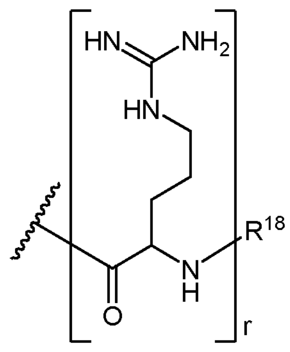

or

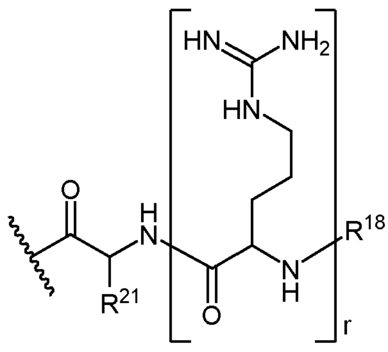

.

In another embodiment of the peptide-oligomer-conjugates of the disclosure, r is 5, 6, or 7.

In another embodiment of the peptide-oligomer-conjugate of Formula Ic, or a pharmaceutically acceptable salt thereof, the peptide-oligomer-conjugate is selected from the group consisting of:

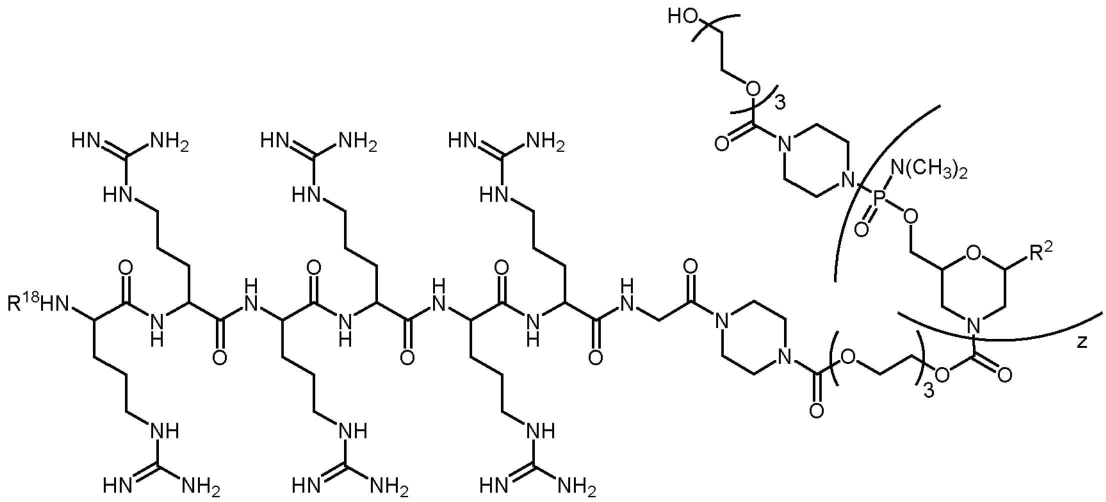

,

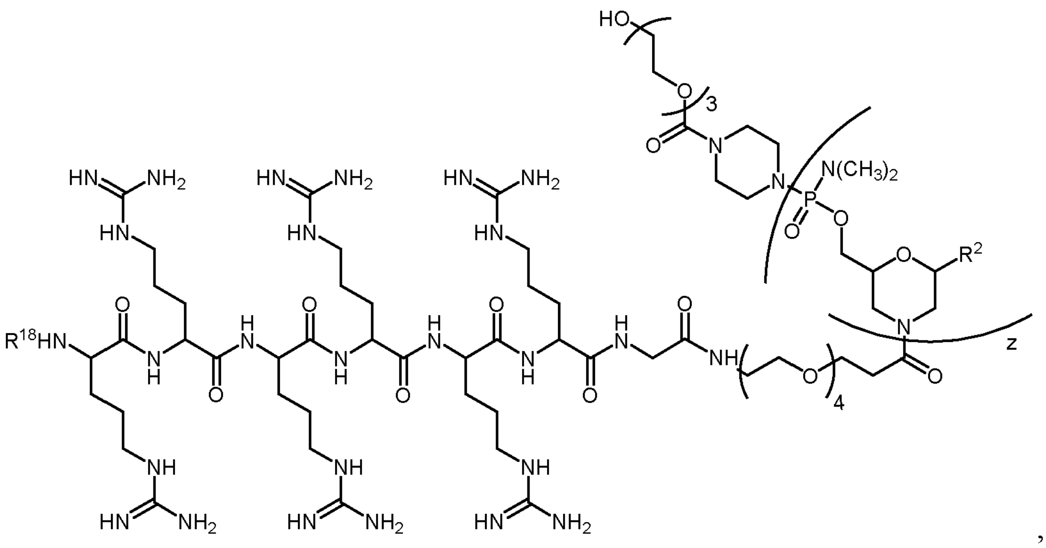

,

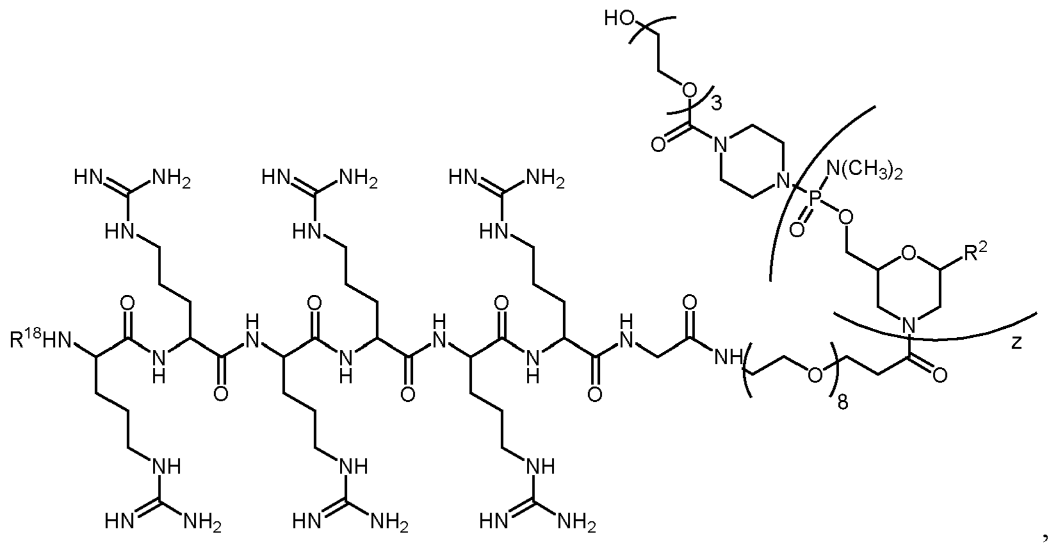

,

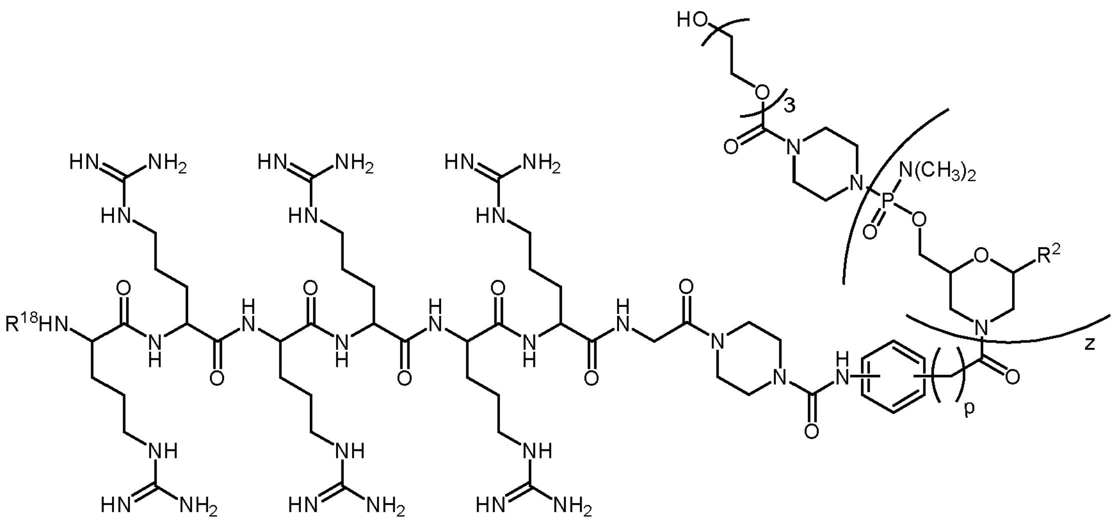

,

and

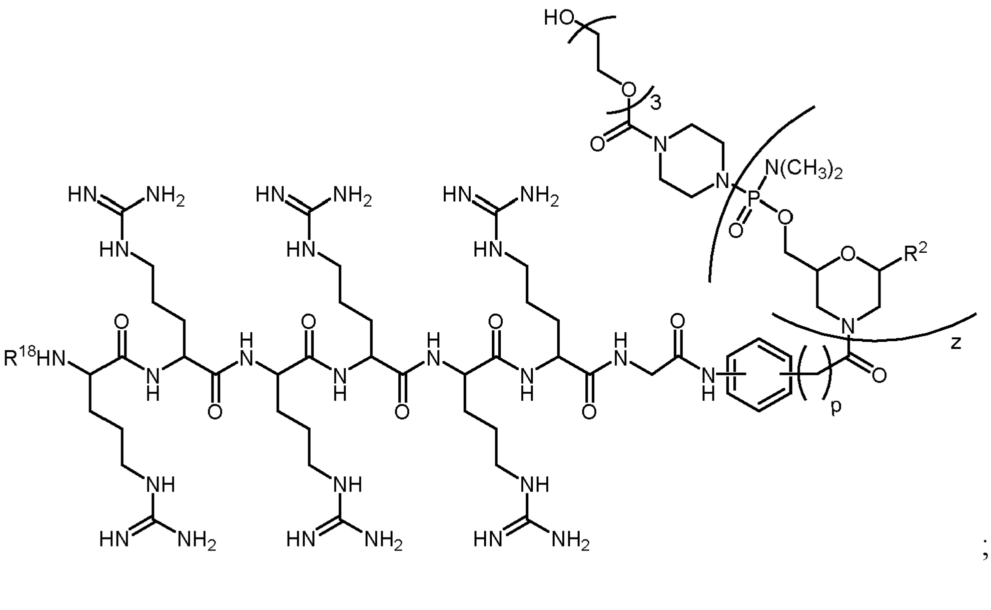

;

wherein $R^{18}$ is selected from H and $-C(O)CH_3$.

In another embodiment of the peptide-oligomer-conjugate of Formula Ic, $R^{18}$ is H.

In another embodiment of the peptide-oligomer-conjugate of Formula Ic, $R^{18}$ is - $C(O)CH_3$.

In the peptide-oligomer-conjugate of Formula I, any one of the following conditions is present: 1) $R^1$ is $NR^7R^{12}$; or 2) $R^4$ is $R^{12}$; or 3) $R^3$ is

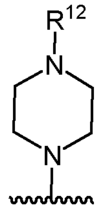

.

In another embodiment of the peptide-oligomer-conjugates of the disclosure, the oligonucleotide comprises a targeting sequence having sequence complementarity to an RNA target.

In another embodiment of the peptide-oligomer-conjugates of the disclosure, the RNA target is a cellular RNA target.

In another embodiment of the peptide-oligomer-conjugates of the disclosure, the targeting sequence has sufficient sequence complementarity to bind to the RNA target.

In another embodiment of the peptide-oligomer-conjugates of the disclosure, the targeting sequence has perfect sequence complementarity to the RNA target.

In some embodiments, the peptide-oligomer-conjugates of the disclosure are unsolvated. In other embodiments, one or more of the peptide-oligomer-conjugates are in solvated form. As known in the art, the solvate can be any of pharmaceutically acceptable solvent, such as water, ethanol, and the like.

Although the peptide-oligomer-conjugates of Formula I, Formula Ia, Formula Ib, and Formula Ic are depicted in their neutral forms, in some embodiments, these peptide-oligonucleotide-conjugates are used in a pharmaceutically acceptable salt form.

*Oligomers*

Important properties of morpholino-based subunits include: 1) the ability to be linked in a oligomeric form by stable, uncharged or positively charged backbone linkages; 2) the ability to support a nucleotide base (e.g. adenine, cytosine, guanine, thymidine, uracil, 5-methyl-cytosine and hypoxanthine) such that the polymer formed can hybridize with a complementary-base target nucleic acid, including target RNA, with $T_M$ values above about 45°C in relatively short oligomers (e.g. , 10-15 bases); 3) the ability of the oligomer to be actively or passively transported into mammalian cells; and 4) the ability of the oligomer and oligomer:RNA heteroduplex to resist RNAse and RNase H degradation, respectively.

The stability of the duplex formed between an oligomer and a target sequence is a function of the binding $T_M$ and the susceptibility of the duplex to cellular enzymatic cleavage. The $T_M$ of an oligomer with respect to complementary-sequence RNA may be measured by conventional methods, such as those described by Hames et al., Nucleic Acid Hybridization, IRL Press, 1985, pp. 107-108 or as described in Miyada C. G. and Wallace R. B., 1987, Oligomer Hybridization Techniques, Methods Enzymol. Vol. 154 pp. 94-107. In certain embodiments, antisense oligomers may have a binding $T_M$, with respect to a complementary-sequence RNA, of greater than body temperature and, in some embodiments greater than about 45°C or 50°C. $T_M$'s in the range 60-80°C or greater are also included. According to well-known principles, the $T_M$ of an oligomer, with respect to a complementary-based RNA hybrid, can be increased by increasing the ratio of C:G paired bases in the duplex, or by increasing the length (in base pairs) of the heteroduplex, or both. At the same time, for purposes of optimizing cellular uptake, it may be advantageous to limit the size of the oligomer. For this reason, compounds of the disclosure include compounds that show a high $T_M$ (45-50°C or greater) at a length of 25 bases or less.

The length of an oligomer may vary so long as it is capable of binding selectively to the intended location within the pre-mRNA molecule. The length of such sequences can be determined in accordance with selection procedures described herein. Generally, the oligomer will be from about 8 nucleotides in length up to about 50 nucleotides in length. For example, the length of the oligomer (z) can be 8-40, 8-25, 15-25, 10-20, or about 18. It will be appreciated however that any length of nucleotides within this range may be used in the methods described herein.

In some embodiments, the antisense oligomers contain base modifications or substitutions. For example, certain nucleobases may be selected to increase the binding affinity of the antisense oligonucleotides described herein. These include 5-substituted pyrimidines, 6-azapyrimidines and N-2, N-6 and O-6 substituted purines, including 2-aminopropyladenine, 5-propynyluracil, 5-propynylcytosine and 2,6-diaminopurine. 5-methylcytosine substitutions have been shown to increase nucleic acid duplex stability by 0.6-1.2°C, and may be incorporated into the antisense oligomers described herein. In one embodiment, at least one pyrimidine base of the oligomer comprises a 5-substituted pyrimidine base, wherein the pyrimidine base is selected from the group consisting of cytosine, thymine and uracil. In one embodiment, the 5-substituted pyrimidine base is 5-methylcytosine. In another embodiment, at least one purine base of the oligonucleotide comprises an N-2, N-6 substituted purine base. In one embodiment, the N-2, N-6 substituted purine base is 2, 6-diaminopurine.

Morpholino-based oligomers (including antisense oligomers) are detailed, for example, in U.S. Patent Nos. 5,698,685; 5,217,866; 5,142,047; 5,034,506; 5,166,315; 5,185,444; 5,521,063; 5,506,337, 8,299,206; and 8,076,476;; PCT Publication Nos. WO 2009/064471 and WO 2012/043730; and Summerton et al. 1997, Antisense and Nucleic Acid Drug Development, 7, 187-195.

Provided in Table 1 are various embodiments of nucleotide moieties as described herein.

**Table 1:** Various embodiments of nucleotide moieties.

| | |
|---|---|
| A = 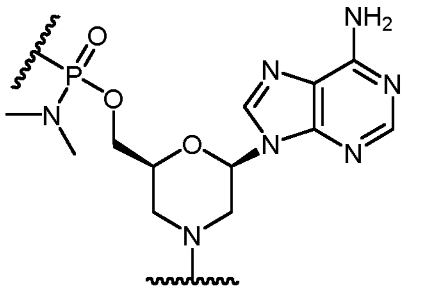 | G = 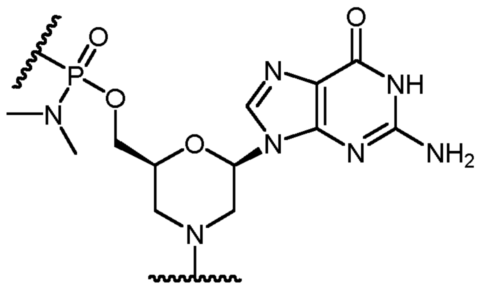 |
| C = 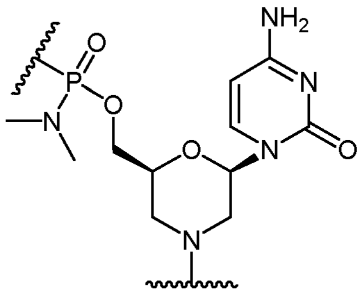 | T = 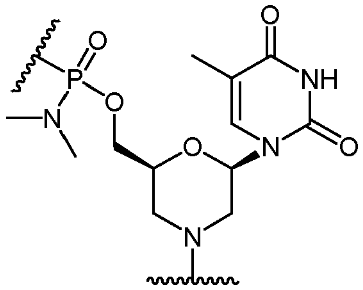 |
| 5-Me-C = 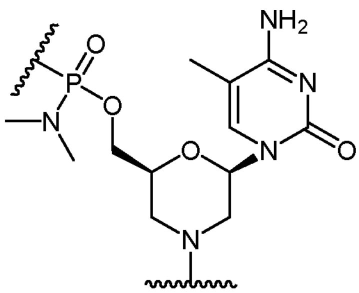 | I = 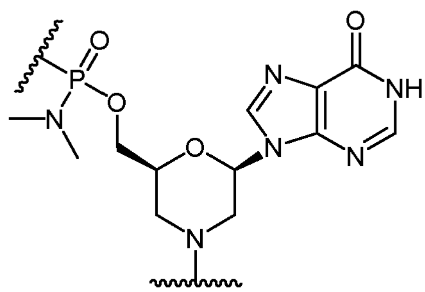 |
| U = 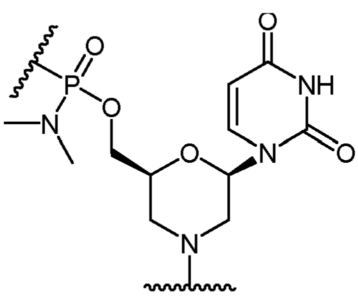 | |

In some embodiments, the oligomers described herein are unsolvated. In other embodiments, one or more of the oligomers are in solvated form. As known in the art, the solvate can be any of pharmaceutically acceptable solvent, such as water, ethanol, and the like.

*Peptides*

The oligomers provided herein include an oligomer moiety conjugated to a CPP. In some embodiments, the CPP can be an arginine-rich peptide transport moiety effective to enhance transport of the compound into cells. The transport moiety is, in some embodiments, attached to a terminus of the oligomer. The peptides have the capability of inducing cell penetration within 30%, 40%, 50%, 60%, 70%, 80%, 90% or 100% of cells of a given cell culture population, including all integers in between, and allow macromolecular translocation within multiple tissues in vivo upon systemic administration. In one embodiment, the cell-penetrating peptide may be an arginine-rich peptide transporter. In various embodiments, a peptide-oligomer-conjugate of the present disclosure may utilize glycine as the linker between the CPP and the antisense oligonucleotide.

The transport moieties as described above have been shown to greatly enhance cell entry of attached oligomers, relative to uptake of the oligomer in the absence of the attached transport moiety. Uptake may be enhanced at least ten fold, and, in some embodiments, twenty fold, relative to the unconjugated compound.

The use of arginine-rich peptide transporters (i.e., cell-penetrating peptides) is particularly useful in practicing the present disclosure. Certain peptide transporters have been shown to be highly effective at delivery of antisense compounds into primary cells including muscle cells.

*Uses*

Provided herein are peptide-oligomer-conjugate as described above for use in treating a central nervous system disorder, a muscle disease, a viral infection, or a bacterial infection in a subject in need thereof.

Accordingly, in one aspect, provided herein are peptide-oligomer-conjugate as described above for use in treating a muscle disease, a viral infection, or a bacterial infection in a subject in need thereof.

In one embodiment, the muscle disease is Duchenne Muscular Dystrophy.

In another embodiment, the viral infection is caused by a virus selected from marburg virus, ebola virus, influenza virus, and dengue virus.

In yet another embodiment, the bacterial infection is caused by *Mycobacterium tuberculosis.*

In still another embodiment, the central nervous system disorder is spinal muscular atrophy.

The subject considered herein is typically a human. However, the subject can be any mammal for which treatment is desired. Thus, the uses described herein can be applied to both human and veterinary applications.

*Administration/Dose*

The formulation of therapeutic compositions and their subsequent administration (dosing) is within the skill of those in the art. Dosing is dependent on severity and responsiveness of the disease state to be treated, with the course of treatment lasting from several days to several months, or until a sufficient diminution of the disease state is achieved. Optimal dosing schedules can be calculated from measurements of drug accumulation in the body of the patient.

Persons of ordinary skill can easily determine optimum dosages, dosing methodologies and repetition rates. Optimum dosages may vary depending on the relative potency of individual oligomers, and can generally be estimated based on $EC_{50}$s found to be effective in in vitro and in vivo animal models. In general, dosage is from 0.01 μg to 100 g/kg of body weight, and may be given once or more daily, weekly, monthly or yearly, or even once every 2 to 20 years. Persons of ordinary skill in the art can easily estimate repetition rates for dosing based on measured residence times and concentrations of the drug in bodily fluids or tissues. Following successful treatment, it may be desirable to have the patient undergo maintenance therapy to prevent the recurrence of the disease state, wherein the oligomer is administered in maintenance doses, ranging from 0.01 μg to 100 g/kg of body weight, once or more daily, to once every 20 years.

In some embodiments, the peptide-oligomer-conjugate (a peptide-oligomer-conjugate of Formula I, Formula Ia, Formula Ib, or Formula Ic) is administered alone.

In some embodiments, the peptide-oligomer-conjugate is administered in a therapeutically effective amount or dosage. A "therapeutically effective amount" is an amount of the peptide-oligomer-conjugate (a peptide-oligonucleotide-conjugate of Formula I, Formula Ia, Formula Ib, or Formula Ic) that, when administered to a patient by itself, effectively treats a muscle disease, a viral infection, or a bacterial infection. An amount that proves to be a "therapeutically effective amount" in a given instance, for a particular subject, may not be effective for 100% of subjects similarly treated for the disease or condition under consideration, even though such dosage is deemed a "therapeutically effective amount" by skilled practitioners. The amount of the peptide-oligomer-conjugate that corresponds to a therapeutically effective amount

is strongly dependent on the type of disease, stage of the disease, the age of the patient being treated, and other facts.

In different embodiments, depending on the peptide-oligomer-conjugate (a peptide-oligomer-conjugate of Formula I, Formula Ia, Formula Ib, or Formula Ic) and the effective amounts used, the peptide-oligomer-conjugate can modulate the expression of a gene involved in a muscle disease, a viral infection, or a bacterial infection.

While the amounts of the peptide-oligomer-conjugate (a peptide-oligomer-conjugate of Formula I, Formula Ia, Formula Ib, or Formula Ic) should result in the effective treatment of a central nervous system disorder, a muscle disease, a viral infection, or a bacterial infection, the amounts, are preferably not excessively toxic to the patient (i.e., the amounts are preferably within toxicity limits as established by medical guidelines). In some embodiments, either to prevent excessive toxicity or provide a more efficacious treatment, or both, of a central nervous system disorder, a muscle disease, a viral infection, or a bacterial infection, a limitation on the total administered dosage is provided. Typically, the amounts considered herein are per day; however, half-day and two-day or three-day cycles also are considered herein.

Different dosage regimens may be used to treat a central nervous system disorder, a muscle disease, a viral infection, or a bacterial infection. In some embodiments, a daily dosage, such as any of the exemplary dosages described above, is administered once, twice, three times, or four times a day for three, four, five, six, seven, eight, nine, or ten days. Depending on the stage and severity of the disease being treated, a shorter treatment time (e.g., up to five days) may be employed along with a high dosage, or a longer treatment time (e.g., ten or more days, or weeks, or a month, or longer) may be employed along with a low dosage. In some embodiments, a once- or twice-daily dosage is administered every other day.

Peptide-oligomer-conjugates (peptide-oligomer-conjugates of Formula I, Formula Ia, Formula Ib, or Formula Ic), or their pharmaceutically acceptable salts or solvate forms, in pure form or in an appropriate pharmaceutical composition, can be administered via any of the accepted modes of administration or agents known in the art. The peptide-oligomer-conjugates can be administered, for example, orally, nasally, parenterally (intravenous, intramuscular, or subcutaneous), topically, transdermally, intravaginally, intravesically, intracistemally, or rectally. The dosage form can be, for example, a solid, semi-solid, lyophilized powder, or liquid dosage forms, such as for example, tablets, pills, soft elastic or hard gelatin capsules, powders, solutions, suspensions, suppositories, aerosols, or the like, for example, in unit dosage forms suitable for simple administration of precise dosages. A particular route of administration is oral, particularly one in which a convenient daily dosage regimen can be adjusted according to the degree of severity of the disease to be treated.

Auxiliary and adjuvant agents may include, for example, preserving, wetting, suspending, sweetening, flavoring, perfuming, emulsifying, and dispensing agents. Prevention of the action of microorganisms is generally provided by various antibacterial and antifungal agents, such as, parabens, chlorobutanol, phenol, sorbic acid, and the like. Isotonic agents, such as sugars, sodium chloride, and the like, may also be included. Prolonged absorption of an injectable pharmaceutical form can be brought about by the use of agents delaying absorption, for example, aluminum monostearate and gelatin. The auxiliary agents also can include wetting agents, emulsifying agents, pH buffering agents, and antioxidants, such as, for example, citric acid, sorbitan monolaurate, triethanolamine oleate, butylated hydroxytoluene, and the like.

Solid dosage forms can be prepared with coatings and shells, such as enteric coatings and others well-known in the art. They can contain pacifying agents and can be of such composition that they release the active peptide-oligomer-conjugates in a certain part of the intestinal tract in a delayed manner. Examples of embedded compositions that can be used are polymeric substances and waxes. The active peptide-oligomer-conjugates also can be in microencapsulated form, if appropriate, with one or more of the above-mentioned excipients.

Liquid dosage forms for oral administration include pharmaceutically acceptable emulsions, solutions, suspensions, syrups, and elixirs. Such dosage forms are prepared, for example, by dissolving, dispersing, etc., peptide-oligomer-conjugates described herein, or a pharmaceutically acceptable salt thereof, and optional pharmaceutical adjuvants in a carrier, such as, for example, water, saline, aqueous dextrose, glycerol, ethanol and the like; solubilizing agents and emulsifiers, as for example, ethyl alcohol, isopropyl alcohol, ethyl carbonate, ethyl acetate, benzyl alcohol, benzyl benzoate, propyleneglycol, 1,3-butyleneglycol, dimethyl formamide; oils, in particular, cottonseed oil, groundnut oil, corn germ oil, olive oil, castor oil and sesame oil, glycerol, tetrahydrofurfuryl alcohol, polyethyleneglycols and fatty acid esters of sorbitan; or mixtures of these substances, and the like, to thereby form a solution or suspension.

Generally, depending on the intended mode of administration, the pharmaceutically acceptable compositions will contain about 1% to about 99% by weight of the peptide-oligomer-conjugates of the disclosure, or a pharmaceutically acceptable salt thereof, and 99% to 1% by weight of a pharmaceutically acceptable excipient. In one example, the composition will be between about 5% and about 75% by weight of a peptide-oligomer-conjugate of the disclosure, or a pharmaceutically acceptable salt thereof, with the rest being suitable pharmaceutical excipients.

Actual methods of preparing such dosage forms are known, or will be apparent, to those skilled in this art. Reference is made, for example, to Remington's Pharmaceutical Sciences, 18th Ed. (Mack Publishing Company, Easton, Pa., 1990).

*Kits*

In other embodiments, kits are provided. Kits according to the disclosure include package(s) comprising peptide-oligomer-conjugates, or compositions of the disclosure. In some embodiments, kits comprise a peptide-oligomer-conjugate according to Formula I, Formula Ia, Formula Ib, or Formula Ic, or a pharmaceutically acceptable salt thereof.

The phrase "package" means any vessel containing peptide-oligomer-conjugates or compositions presented herein. In some embodiments, the package can be a box or wrapping. Packaging materials for use in packaging pharmaceutical products are well-known to those of skill in the art. Examples of pharmaceutical packaging materials include, but are not limited to, bottles, tubes, inhalers, pumps, bags, vials, containers, syringes, bottles, and any packaging material suitable for a selected formulation and intended mode of administration and treatment.

The kit can also contain items that are not contained within the package, but are attached to the outside of the package, for example, pipettes.

Kits can further contain instructions for administering peptide-oligomer-conjugates or compositions of the disclosure to a patient. Kits also can comprise instructions for approved uses of peptide-oligomer-conjugates herein by regulatory agencies, such as the United States Food and Drug Administration. Kits can also contain labeling or product inserts for the peptide-oligomer-conjugates. The package(s) or any product insert(s), or both, may themselves be approved by regulatory agencies. The kits can include peptide-oligomer-conjugates in the solid phase or in a liquid phase (such as buffers provided) in a package. The kits can also include buffers for preparing solutions for conducting the uses, and pipettes for transferring liquids from one container to another.

## EXAMPLES

Examples have been set forth below for the purpose of illustration and to describe certain specific embodiments of the disclosure. However, the scope of the claims is not to be in any way limited by the examples set forth herein. Various changes and modifications to the disclosed embodiments will be apparent to those skilled in the art and such changes and modifications including, without limitation, those relating to the chemical structures, substituents, derivatives, formulations or uses of the disclosure may be made without departing from the scope of the appended claims. Definitions of the variables in the structures in the schemes herein are commensurate with those of corresponding positions in the formulae presented herein.

### Example 1: Synthesis of PPMO-5 and PPMO-1

As shown in Figure 1, to a mixture of the PMO (PPMO-3, 1 eq.) described in Table 1a below, Fmoc-amino-PEGm-propionic acid (5 eq.), HATU (5 eq) in DMSO was added DIPEA (10 eq.) at room temperature. After stirring for 4 hours, excess 4-methylpiperidine was added and stirring continued at room temperature overnight. The crude product was diluted with deionized water and then purified by SPE (Amberchrom CG300M). The product was obtained by lyophilization as a white powder and its structure confirmed by LC/MS.

To a mixture of the product above, Ac-$R_6$-Gly (4 eq.; SEQ ID NO:1), and HATU (4 eq.) in DMSO was added DIPEA (10 eq.) at room temperature and stirred for 4 hours. The reaction mixture was diluted with deionized water and purified by an ISCO chromatography system (SCX column, Source 30s, mobile phase (pH = 7): Solvent A: 20 mM $NaHPO_4$/25% ACN (pH = 7); Solvent B: 1.5 M guanidine hydrochloride, 20 mM $NaH_2PO_4$/25% ACN, then desalted by SPE (Amberchrom CG300M). The product was obtained by lyophilization as a white powder and its structure confirmed by LC/MS.

**Table 1a**

| Compound Name | Nucleobase Sequence | 5' Attachment | 3' Attachment |
|---|---|---|---|
| PPMO-3 | 5'-GCT ATT ACC TTA ACC CAG T-3' (SEQ ID NO:3) | TEG | H |

### Example 2: Synthesis of PPMO-4

As shown in Figure 2, to a mixture of the PMO (PPMO-3, 1 eq.) and "P3P" linker (2.5 eq.) in DMSO was added N-ethyl morpholine (2.5 eq.) at room temperature. After stirring for 2 hours, morpholine (3 eq.) was added and the

reaction mixture stirred at room temperature for 1 hour. Excess 50 mM CYTFA solution was added and stirred for 1h, then the solution was basified by adding 1 M $Na_2HPO_4$ solution. The crude product was diluted with deionized water and purified by SPE (Amberchrom CG300M). The product was obtained by lyophilization as a white powder and its structure confirmed by LC/MS.

To a mixture of the product above, Ac-$R_6$-Gly (4 eq.; SEQ ID NO:1), and HATU (4 eq.) in DMSO was added DIPEA (10 eq.) at room temperature and stirred for 4 hours. The crude product was diluted with deionized water and purified by an ISCO chromatography system (SCX column, Source 30s, mobile phase (pH = 7): Solvent A: 20 mM $NaHPO_4$/25% ACN (pH = 7); Solvent B: 1.5 M guanidine hydrochloride, 20 mM $NaH_2PO_4$/25% ACN, then desalted by SPE (Amberchrom CG300M). The product was obtained by lyophilization as a white powder and its structure confirmed by LC/MS.

**Example 3: Dose Response for Efficacy of Linker Modified Compounds in MDX Mice**

The aim of this study was to compare efficacy of peptide-oligomer-conjugates of the disclosure in a dose response study in mice. The spacing between the peptide and PMO modulates efficacy. Accordingly, PEG lengths of 3, 4, and 8 were used to systematically increase spacing between Ac-$R_6$-Gly (SEQ ID NO: 1) and the PMO. Additionally, an all-D amino acid version of Ac-$R_6$-Gly (SEQ ID NO: 1) and Ac-$R_6$-Apa (SEQ ID NO:4) (4-amino phenyl acetic acid; aromatic, hydrophobic linker) were used (Table 2).

The study was conducted in compliance with the following Animal Health regulation: USDA Animal Welfare Act 9 CFR Parts 1 — 3. Federal Register 39129, July 22, 1993. Animal care was in accordance with the study protocol and adhered to regulations outlined in the USDA Animal Welfare Act (9 CFR parts 1, 2, and 3) and the conditions specified in the Guide for the Care and Use of Laboratory Animals (ILAR Publication 1996, National Academy Press).

*Test Materials*

Selected peptide-oligomer-conjugates that were tested are listed in Table 2 (the peptide-oligomer-conjugates were formulated in saline and stored at 5°C).

**Table 2.**

| Peptide-oligomer-conjugate | Peptide-oligomer-conjugate Structure |
|---|---|
| PPMO-4 | 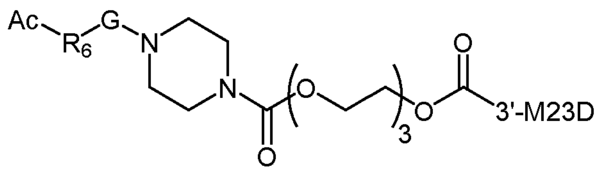 Ac-$R_6$-G-PIP-PEG3 (SEQ ID NO:5) |
| PPMO-1 | 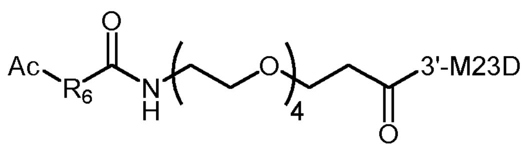 Ac-$R_6$-G-PEG4 (SEQ ID NO:6) |
| PPMO-5 | 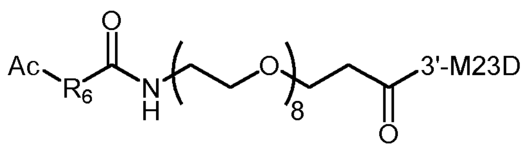 Ac-$R_6$-G-PEG8 (SEQ ID NO:7) |
| PPMO-6 | 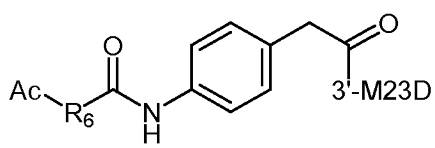 Ac-$R_6$-Apa (SEQ ID NO:4) |

(continued)

| Peptide-oligomer-conjugate | Peptide-oligomer-conjugate Structure |
|---|---|
| PPMO-7 | Ac-d$R_6$-G (all-D amino acid R) (SEQ ID NO:8) |
| *G = Gly, Ac = acetyl, R = Arg, M23D = 5'-GCT ATT ACC TTA ACC CAG-3' (SEQ ID NO:2) | |

*Test System*

Animals used for the study are described in Table 3.

**Table 3.**

| | |
|---|---|
| Species: | Mouse |
| Strain/sub-strain/Source: | mdx (C57Bl/10ScSn-Dmd$^{mdx}$/J; Jackson Laboratories (#001801)) |
| Age on Arrival: | 6-9 weeks old |
| Weight on Arrival: | 18-22 grams |
| Number and Sex: | 130 females including extras |
| Identification: | Ear tag and color-coded cage card |

Upon receipt, the animals were unpacked and placed in cages. A visual health inspection was performed on each animal to include evaluation of the coat, extremities and orifices. Each animal was examined for any abnormal signs in posture or movement. The mice were acclimated for a minimum of eight or nine days (Cohorts 1 and 2, respectively) prior to the commencement of the experimental procedures.

The animals were housed up to 5 per cage in clear polycarbonate microisolator cages with certified irradiated contact bedding. The cages conformed to standards set forth in the Animal Welfare Act (with all amendments) and the Guide for the Care and Use of Laboratory Animals, National Academy Press, Washington, D.C., 1996. Oval pellet Certified Picolab Rodent 20 Diet (PMI Feeds Inc., Richmond, Indiana, USA) was provided ad libitum. Deionized water was available to animals *ad libitum* throughout the study period. Enrich-o-cob bedding and sanitized igloos and/or tunnels were provided as enrichment. There were no known contaminants in the feed, water, enrichment materials or bedding that would be expected to interfere with this study. Environmental controls were set to maintain temperatures of 18°C to 26°C (64°F to 79°F) with a relative humidity of 30% to 70%. These parameters were recorded at least once daily. A 12:12 hour light:dark cycle was maintained.

*Experimental Procedures*

Animals were randomized into treatment groups based on cage weights as specified in Table 4.

**Table 4.** Treatment Groups

| Group n=5 | Peptide-Oligomer-Conjugate | Dose per injection(mg/kg) | Regimen | Route of Admin. |
|---|---|---|---|---|
| 1 | PPMO-4 | 5 | Single injection | Tail i.v. 200 μL |
| 2 | | 10 | | |
| 3 | | 20 | | |
| 4 | | 40 | | |
| 5 | | 80 | | |
| 6 | PPMO-1 | 5 | | |
| 7 | | 10 | | |
| 8 | | 20 | | |
| 9 | | 40 | | |
| 10 | | 80 | | |
| 11 | PPMO-5 | 5 | | |
| 12 | | 10 | | |
| 13 | | 20 | | |
| 14 | | 40 | | |
| 15 | | 80 | | |
| 16 | PPMO-6 | 5 | | |
| 17 | | 10 | | |
| 18 | | 20 | | |
| 19 | | 40 | | |
| 20 | | 80 | | |
| 21 | PPMO-7 | 5 | | |
| 22 | | 10 | | |
| 23 | | 20 | | |
| 24 | | 40 | | |
| 25 | | 80 | | |

The day of dosing on the study was designated as Study Day 1. Each peptide-oligomer-conjugate was vortexted for approximately 10 seconds prior to dosing, and administered via tail vein as a slow push bolus (~5 seconds; 200 μL). Dosing was performed over two days. All animals receiving the same peptide-oligomer-conjugate were dosed on the same day. An animal assigned to a treatment group that could not be dosed, had a failed injection or died immediately post-dose was replaced by a spare mouse. Any remaining spare animals were necropsied and tissues collected as specified below.

Animals were observed for moribundity and mortality once daily. Any animal showing signs of distress, particularly if death appeared imminent was humanely euthanized according to Numira Biosciences Standard Operating Procedures. Body weights were recorded on the day after arrival, the day of dosing, and the day of necropsy. Detailed clinical observations were conducted and recorded at 0 minutes, 15 minutes, and 2 hours post-dose to assess tolerability of injections.

Animals unlikely to survive until the next scheduled observation were weighed and euthanized. Animals found dead were weighed and the time of death was estimated as closely as possible. Blood and tissue samples were not collected.

On day 8 (7 days post-dose), all animals, including any untreated or spare animals, were humanely euthanized with carbon dioxide. Euthanasia was performed in accordance with accepted American Veterinary Medical Association (AVMA) guidelines on Euthanasia, June 2007.

The partial gross necropsy included examination and documentation of findings. All external surfaces and

orifices were evaluated. All abnormalities observed during the collection of the tissues listed below were described completely and recorded. No additional tissues were taken.

Tissues were collected within 15 minutes or less of euthanasia. All instruments and tools used were changed between treatment groups. All tissues were flash frozen and stored at a temperature lower than -70°C as soon as possible after collection. The following tissues were collected: liver, kidneys, heart, quads, and diaphragm.

*Results - Animal Health and Body Weight*

Animal #2407 was underweight and sick upon arrival. It was not placed on study and was humanely euthanized. Animals in TG (treatment group) 4 (PPMO-4 at 40mg/kg), TG 5 (PPMO-4 at 80mg/kg) and TG 20 (PPMO-6 at 80mg/kg) were all noted as slow to recover at the 15 minute observation but recovered by the 2 hour observation. Animal 2356, TG 14, (PPMO-5 40mg/kg) was found dead the day after dosing. At the time of necropsy, animal 2406, TG 1 (PPMO-4 at 5mg/kg) was noted as having its right eye closed and had a white substance exuding from it. Also at the time of time of necropsy, animal 2422, TG 18 (PPMO-6 at 20mg/kg) was noted as having a small amount of fluid present in the left kidney. Body weights of the animals throughout the study are presented in Table 5.

**Table 5.** Individual Body Weights (g)

| Treatment Group | Animal # | Arrival | Pre-dose | Pre-necropsy |
|---|---|---|---|---|
| 1 (PPMO-4; 5mg/kg) | 2406 | 18.58 | 20.72 | 22.61 |
| | 2408 | 16.92 | 17.75 | 19.15 |
| | 2409 | 16.61 | 18.49 | 19.6 |
| | 2410 | 18.96 | 20.61 | 21.53 |
| | 2336 | 19.6 | 20.26 | 21.45 |
| 2 (PPMO-4; 10mg/kg) | 2426 | 16.1 | 18.7 | 19.41 |
| | 2427 | 19.05 | 20.74 | 21.91 |
| | 2428 | 16.91 | 18.43 | 18.98 |
| | 2429 | 18.03 | 19.59 | 20.43 |
| | 2430 | 17.09 | 19.96 | 20.49 |
| 3 (PPMO-4; 20mg/kg) | 2396 | 16.51 | 19.41 | 20.09 |
| | 2397 | 20.65 | 20.72 | 21.25 |
| | 2398 | 18.96 | 21.98 | 22.62 |
| | 2399 | 17.29 | 19.79 | 20.71 |
| | 2400 | 15.39 | 16.73 | 17.08 |
| 4 (PPMO-4; 40mg/kg) | 2391 | 18.51 | 19.95 | 20.59 |
| | 2392 | 15.27 | 17.51 | 18.25 |
| | 2393 | 18.55 | 21.06 | 22.63 |
| | 2394 | 18.34 | 20.52 | 21.35 |
| | 2395 | 18.18 | 20.98 | 21.22 |
| 5 (PPMO-4; 80mg/kg) | 2411 | 20.37 | 21.88 | 23.86 |
| | 2412 | 15.27 | 16.88 | 17.9 |
| | 2413 | 18.03 | 20.42 | 21.36 |
| | 2414 | 16.73 | 19.86 | 21.1 |
| | 2415 | 18.56 | 20.38 | 20.69 |

(continued)

| Treatment Group | Animal # | Arrival | Pre-dose | Pre-necropsy |
|---|---|---|---|---|
| 6 (PPMO-1; 5mg/kg) | 2381 | 17.05 | 19.35 | 20.27 |
| | 2382 | 17.17 | 19.24 | 20.27 |
| | 2383 | 16.02 | 17.78 | 18.74 |
| | 2384 | 19.37 | 21.41 | 22 |
| | 2385 | 19.56 | 20.24 | 21.26 |
| 7 (PPMO-1; 10mg/kg) | 2346 | 19.43 | 21.36 | 22.54 |
| | 2347 | 20.23 | 21.43 | 21.7 |
| | 2348 | 17.1 | 19.67 | 21.11 |
| | 2349 | 17.28 | 18.95 | 20.33 |
| | 2350 | 16.29 | 17.88 | 19.54 |
| 8 (PPMO-1; 20mg/kg) | 2306 | 17.75 | 19.24 | 20.28 |
| | 2307 | 18.61 | 19.94 | 21.18 |
| | 2308 | 18.49 | 20.86 | 21.54 |
| | 2309 | 17.7 | 18.89 | 20.73 |
| | 2310 | 17.88 | 19.14 | 20.87 |
| 9 (PPMO-1; 40mg/kg) | 2386 | 18.09 | 18.06 | 15.42 |
| | 2387 | 16.99 | 17.87 | 18.87 |
| | 2388 | 18.51 | 19.57 | 19.6 |
| | 2389 | 18.63 | 21.16 | 22.33 |
| | 2390 | 19.21 | 20.33 | 22.3 |
| 10 (PPMO-1; 80mg/kg) | 2401 | 18.05 | 19.9 | 20.89 |
| | 2402 | 19.76 | 21.33 | 23.12 |
| | 2403 | 17.77 | 19.79 | 20.4 |
| | 2404 | 18.68 | 19.65 | 21.22 |
| | 2405 | 17.74 | 19.44 | 20.1 |
| 11 (PPMO-5; 5mg/kg) | 2311 | 17.82 | 20.79 | 22.42 |
| | 2312 | 18.75 | 20.83 | 22.42 |
| | 2313 | 18.56 | 19.65 | 20.08 |
| | 2314 | 18.69 | 19.36 | 21.04 |
| | 2315 | 19.59 | 20.18 | 21.81 |
| 12 (PPMO-5; 10mg/kg) | 2316 | 17.15 | 19.79 | 19.66 |
| | 2317 | 19.26 | 20.41 | 21.44 |
| | 2318 | 20.42 | 21.83 | 23.67 |
| | 2319 | 17.6 | 18.48 | 20.23 |
| | 2320 | 19.05 | 20.38 | 21.78 |

(continued)

| Treatment Group | Animal # | Arrival | Pre-dose | Pre-necropsy |
|---|---|---|---|---|
| 13 (PPMO-5; 20mg/kg) | 2371 | 17.27 | 18.74 | 20.65 |
| | 2372 | 19.51 | 20.99 | 22.61 |
| | 2373 | 17.95 | 19.47 | 20.88 |
| | 2374 | 21.1 | 24.15 | 24.22 |
| | 2375 | 17.85 | 19.7 | 21.63 |
| 14 (PPMO-5; 40mg/kg) | 2356 | 20.73 | 21.78 | 12.81 (FD) |
| | 2357 | 18.89 | 20.89 | 21.11 |
| | 2358 | 18.15 | 19.93 | 21 |
| | 2359 | 18.27 | 19.09 | 20.11 |
| | 2360 | 18.24 | 20.31 | 21.48 |
| 15 (PPMO-5; 80mg/kg) | 2361 | 19.1 | 19.82 | 20.45 |
| | 2362 | 19.59 | 20.53 | 21.35 |
| | 2363 | 18 | 18.87 | 18.64 |
| | 2364 | 18.97 | 21.17 | 22.56 |
| | 2365 | 19.03 | 20.91 | 20.99 |
| 16 (PPMO-6; 5mg/kg) | 2301 | 20.42 | 23.02 | 23.95 |
| | 2302 | 18.59 | 21.24 | 21.87 |
| | 2303 | 18.82 | 20.78 | 21.53 |
| | 2304 | 18.41 | 20.22 | 21.24 |
| | 2305 | 19.2 | 20.8 | 22.26 |
| 17 (PPMO-6; 10mg/kg) | 2376 | 20.29 | 21.43 | 21.67 |
| | 2377 | 21.16 | 21.31 | 22.17 |
| | 2378 | 16.57 | 18.14 | 19.57 |
| | 2379 | 19.4 | 20.53 | 21.55 |
| | 2380 | 18.18 | 20 | 20.42 |
| 18 (PPMO-6; 20mg/kg) | 2421 | 22.54 | 24.92 | 25.91 |
| | 2422 | 18.98 | 20.33 | 21.09 |
| | 2423 | 17.76 | 20.01 | 20.84 |
| | 2424 | 18.53 | 20.67 | 21.11 |
| | 2425 | 18.1 | 20.01 | 20.1 |
| 19 (PPMO-6; 40mg/kg) | 2351 | 18.61 | 20.74 | 21.71 |
| | 2352 | 18.1 | 20.65 | 21.52 |
| | 2353 | 19.57 | 22.29 | 22.99 |
| | 2354 | 21.16 | 23.68 | 24.62 |
| | 2355 | 18.53 | 20.62 | 21.32 |

(continued)

| Treatment Group | Animal # | Arrival | Pre-dose | Pre-necropsy |
|---|---|---|---|---|
| 20 (PPMO-6; 80mg/kg) | 2326 | 19.16 | 19.81 | 22.3 |
| | 2327 | 19.47 | 19.77 | 21.23 |
| | 2328 | 19.4 | 20.83 | 22.41 |
| | 2329 | 19.02 | 19.27 | 20.43 |
| | 2330 | 19.97 | 21.05 | 21.85 |
| 21 (PPMO-7; 5mg/kg) | 2416 | 18.63 | 21.43 | 22.3 |
| | 2417 | 18.57 | 21.73 | 22.46 |
| | 2418 | 20.34 | 22.33 | 23.21 |
| | 2419 | 17.61 | 19.86 | 21.43 |
| | 2420 | 21.96 | 22.24 | 23.41 |
| 22 (PPMO-7; 10mg/kg) | 2366 | 19.05 | 21.41 | 17.85 |
| | 2367 | 20.79 | 20.8 | 22.7 |
| | 2368 | 19.83 | 21.93 | 23.56 |
| | 2369 | 19.62 | 21.47 | 21.97 |
| | 2370 | 17.98 | 21.01 | 21.8 |
| 23 (PPMO-7; 20mg/kg) | 2341 | 18.39 | 19.79 | 20.85 |
| | 2342 | 19.93 | 19.9 | 20.17 |
| | 2343 | 18.06 | 19.44 | 21.1 |
| | 2344 | 18.76 | 21.15 | 21.38 |
| | 2345 | 22.35 | 23.99 | 24.55 |
| 24 (PPMO-7; 40mg/kg) | 2321 | 20.83 | 22.31 | 22.58 |
| | 2322 | 20.79 | 21.57 | 22.21 |
| | 2323 | 19.99 | 19.79 | 21.29 |
| | 2324 | 18.1 | 17.99 | 18.19 |
| | 2325 | 18.58 | 19.77 | 20.93 |
| 25 (PPMO-7; 80mg/kg) | 2331 | 19.91 | 20.46 | 21.98 |
| | 2332 | 19.55 | 21.11 | 22.63 |
| | 2333 | 18.8 | 18.51 | 19.65 |
| | 2334 | 20.64 | 19.79 | 19.9 |
| | 2335 | 20.61 | 23.11 | 22.86 |
| Spares | 2337 | 21.03 | 23.08 | 23.22 |
| | 2338 | 21.14 | 22.31 | 23.76 |
| | 2339 | 20.39 | 21.22 | 21.07 |
| | 2340 | 21.24 | 23.82 | 23.78 |

*Results - PCR Analysis*

RNA from mouse quadriceps, heart, and diaphragm tissue was purified using GE Illustra RNAspin 96 well extraction kits. Briefly, 400 μL of lysis buffer (RA1 + 1% 2-mercaptoethanol was added to about 20-30 mg of frozen

tissue in a plate with zirconia beads (Biospec) and homogenized using GenoGrinder (Spex Sample Prep) at 4x8 minutes at 1750RPM; cooling between each run. The homogenate was immediately processed for RNA purification according to the GE RNAspin Illustra 96 well protocol. Total RNA was quantitated with a Nanodrop 2000 spectrophotometer (ThermoScientific).

RNA was analyzed by a classic nested PCR reaction. RT-PCR reagents were from Invitrogen unless otherwise specified. PCR reactions were run in a CFX96 or S1000 thermocycler (BioRad). Final cDNA products were separated on a NuPage 10% TBE gel (Invitrogen) run at 200V, 1 hr at room temperature. Gels were scanned with a Typhoon Trio (GE Healthcare) using a 670 BP 30 Cy5 emission filter and analyzed with ImageQuant software.

Primers used for PCR analysis were as follows: dystrophin outer forward (5'-CAATGTTTCTGGATGCAGACTTTGTGG-3'; SEQ ID NO:9), dystrophin outer reverse (5'-GTTCAGCTTCACTCTTTATCTTCTGCC-3'; SEQ ID NO:10), dystrophin inner forward (5'-CACATCTTTGATGGTGTGAGG-3'; SEQ ID NO:11), and dystrophin inner reverse (5'-CAACTTCAGCCATCCATTTCTG-3'; SEQ ID NO:12). PCR reactions were performed according to the protocols described in Table 6, and results are summarized in Table 7 and Figures 3-5.

**Table 6.** PCR Method

| | | | |
|---|---|---|---|
| **Reaction setup for RT-PCR and primary amplification (25 μL reaction)** | | | |
| 2x Reaction Mix | 12.5 μL | | |
| Dys Outer Forward Primer (30 μM) | 0.25 μL | | |
| Dys Outer Reverse Primer (30 μM) | 0.25 μL | | |
| Superscript III Platinum *Taq* mix | 1 μL | | |
| Template RNA (10 ng/μL) | 5 μL | | |
| Water to 25 μL total volume | 6 μL | | |
| Add 20 μL MM + 5 μL sample to reaction plate. Shake plate then spin down briefly to ensure all liquid at bottom of well. Run amplification program. | | | |
| **RT-PCR and primary amplification program** | | | |
| | **Temperature** | **Time** | |
| Reverse Transcription | 55°C | 30 minutes | |
| RT Inactivation | 94°C | 2 minutes | |
| Denaturing | 94°C | 1 minute | 8 cycles |
| Annealing | 59°C | 1 minute | |
| Extension | 68°C | 1 minute | |
| | 4°C | (hold) | |
| **Reaction setup for nested secondary amplification (30 μL reaction)** | | | |
| 10× PCR Buffer | 3 μL | | |
| dNTP solution (10 mM) | 0.3 μL | | |
| 50 mM MgCl | 0.9 μL | | |
| Dys Inner Forward Primer (30 μM) | 0.2 μL | | |
| Dys Inner Reverse Primer (30 μM) | 0.2 μL | | |
| Platinum *Taq* DNA Polymerase | 0.15 μL | | |
| 0.1 mM Cy5-dCTP | 0.6 μL | | |
| RT-PCR product | 2 μL | | |
| Water to 30 μL total volume | 22.65 μL | | |
| Add 28 μL MM + 2 μL from reaction plate to second reaction plate; shake, spin, run secondary amplification program. | | | |

(continued)

| Nested secondary amplification program | | | |
|---|---|---|---|
| | **Temperature** | **Time** | |
| Primary Denature | 94°C | 3 minutes | |
| Denaturing | 94°C | 45 seconds | 22 cycles |
| Annealing | 59°C | 30 seconds | |
| Extension | 68°C | 1 minute | |
| | 4°C | (hold) | |

**Table 7.** Summary of % Exon 23 Skipping

| | | | Quadricep (L) | | Diaphragm | | Heart | |
|---|---|---|---|---|---|---|---|---|
| **Target Group** | **Peptide-Oligomer-Conjugate** | **Target Dose (mg/kg)** | **% Exon 23 Skip (Average)** | **Error (SD)** | **% Exon 23 Skip (Average)** | **Error (SD)** | **% Exon 23 Skip (Average)** | **Error (SD)** |
| 1 | PPMO-4 | 5 | 7 | 5 | 0 | 0 | 0 | 0 |
| 2 | | 10 | 24 | 20 | 13 | 4 | 5 | 2 |
| 3 | | 20 | 76 | 9 | 67 | 13 | 25 | 6 |
| 4 | | 40 | 89 | 7 | 90 | 5 | 89 | 2 |
| 5 | | 80 | 93 | 4 | 93 | 2 | 95 | 1 |
| 6 | PPMO-1 | 5 | 0 | 0 | 0 | 0 | 0 | 0 |
| 7 | | 10 | 8 | 9 | 6 | 6 | 0 | 0 |
| 8 | | 20 | 24 | 20 | 16 | 12 | 5 | 4 |
| 9 | | 40 | 81 | 7 | 73 | 12 | 62 | 17 |
| 10 | | 80 | 91 | 3 | 92 | 3 | 92 | 2 |
| 11 | PPMO-5 | 5 | 1 | 2 | 0 | 0 | 0 | 0 |
| 12 | | 10 | 0 | 0 | 1 | 2 | 0 | 0 |
| 13 | | 20 | 29 | 20 | 9 | 2 | 2 | 1 |
| 14 | | 40 | 76 | 11 | 75 | 10 | 34 | 13 |
| 15 | | 80 | 93 | 3 | 91 | 3 | 91 | 3 |
| 16 | PPMO-6 | 5 | 7 | 5 | 3 | 3 | 0 | 0 |
| 17 | | 10 | 16 | 11 | 15 | 11 | 3 | 3 |
| 18 | | 20 | 53 | 22 | 44 | 13 | 30 | 16 |
| 19 | | 40 | 85 | 14 | 90 | 4 | 81 | 14 |
| 20 | | 80 | 91 | 3 | 95 | 2 | 96 | 1 |
| 21 | PPMO-7 | 5 | 4 | 3 | 1 | 1 | 0 | 0 |
| 22 | | 10 | 11 | 5 | 6 | 2 | 2 | 2 |
| 23 | | 20 | 53 | 17 | 30 | 12 | 13 | 7 |
| 24 | | 40 | 89 | 2 | 67 | 21 | 42 | 10 |
| 25 | | 80 | 92 | 2 | 93 | 3 | 93 | 3 |

### Example 4: Maximum Tolerated Dose Study of Linker-Modified Compounds in MDX Mice

The maximum tolerated dose (MTD) of selected peptide-oligomer-conjugates of the disclosure was determined in mice according to the regimens shown in Table 8. The results are summarized in Table 9, which indicates that all compounds, except PPMO-5, have a MTD of between 150 and 200 mg/kg. PPMO-5 has a MTD >200 mg/kg. In-life observations during the MTD study are summarized in Table 10.

**Table 8.** MTD Study

| Group n=3 | Compound | Dose per injection (mg/kg) | Regimen | Route of Admin. |
|---|---|---|---|---|
| 1 | PPMO-4 | 50 | Single injection | TV, i.v. 200 μL |
| 2 | | 100 | | |
| 3 | | 150 | | |
| 4 | | 200 | | |
| 5 | PPMO-1 | 50 | | |
| 6 | | 100 | | |
| 7 | | 150 | | |
| 8 | | 200 | | |
| 9 | PPMO-5 | 50 | | |
| 10 | | 100 | | |
| 11 | | 150 | | |
| 12 | | 200 | | |
| 13 | PPMO-6 | 50 | | |
| 14 | | 100 | | |
| 15 | | 150 | | |
| 16 | | 200 | | |
| 17 | PPMO-7 | 50 | | |
| 18 | | 100 | | |
| 19 | | 150 | | |
| 20 | | 200 | | |

**Table 9.** MTD % Survival

| | Dose (mg/kg) | | | | | |
|---|---|---|---|---|---|---|
| **Compound** | **50** | **100** | **150** | **200** | **400** | |
| PPMO-4 | 100 | 100 | 100 | 0 | ND | % Survival |
| PPMO-1 | 100 | 100 | 100 | 33 | ND | |
| PPMO-5 | 100 | 100 | 100 | 100 | ND | |
| PPMO-6 | 100 | 100 | 100 | 0 | ND | |
| PPMO-7 | 100 | 100 | 100 | 33 | ND | |
| PPMO-8 | 100 | 100 | 100 | 100 | 100 | |

**Table 10.** MTD In-Life Observations

| | |
|---|---|
| **PPMO-4** | |
| 100 mg/kg | 3/3 slow to recover at 15 min., ok by 2 hours |
| 150 mg/kg | 3/3 lethargic at 15 min., ok by 2 hours |
| 200 mg/kg | 3/3 lethargic at 15 min and 2 hours, 3/3 dead at 24 hours |
| **PPMO-1** | |
| 100 mg/kg | 3/3 slow to recover at 15 min., ok by 2 hours |
| 200 mg/kg | 3/3 lethargic at 15 min., 1/3 dead at 2 hours, 2/3 lethargic at 2 hours |
| | another died before 24 hours (total 2/3 dead) |
| **PPMO-5** | |
| 200 mg/kg | 3/3 lethargic at 15 min., ok by 2 hours |
| **PPMO-6** | |
| 150 mg/kg | 3/3 slow to recover at 15 min, slow moving at 2 hours |
| 200 mg/kg | 3/3 slow to recover at 15 min, lethargic at 2 hours, 3/3 dead at 24 hrs |
| **PPMO-7** | |
| 100 mg/kg | 3/3 slow to recover at 15 min., ok by 2 hours |
| 150 mg/kg | 3/3 slow to recover at 15 min., ok by 2 hours |
| 200 mg/kg | 2/3 dead at 15 minutes, 1/3 lethargic at 15 min and 2 hours |

In summary, modifying the linker length of a peptide-oligomer-conjugate leads to improved potency, although tolerability may be reduced. PPMO-4 displayed improved potency over PPMO-8 ($ED_{40}$ of PPMO-4 is approximately three-fold greater than PPMO-8), and displayed greater efficacy than PPMO-2 in all tissue types assayed. Toxicity also appears to be affected by the linker length as a PEG-3 linker was shown to increase efficacy but also toxicity (Figure 10).

All compounds showed greater than ten-fold elevation of KIM-1 starting at 50mg/kg, which suggests the compounds were not well tolerated (Figure 9). PPMO-1 and PPMO-7 200 mg/kg doses showed lower than expected KIM-1, which was seen previously with PPMO-2 and could be due to severe necrosis in the kidney. PPMO-7 (all D-Amino Acid) improved efficacy but was not well-tolerated and increased liver and kidney serum chemistry markers, in particular at high doses (Figure 7). PPMO-6 also improved efficacy but was not well-tolerated and increased KIM-1 levels (Figures 6-9).

**Example 5: Therapeutic Index**

The therapeutic index (TI) can be determined according to the following equation:

$$TI = \frac{MTD}{ED_{40}}$$

where ED refers to the effective dose.

Importantly, the toxicity of a peptide-oligomer-conjugate can be described in two phases: $t_1$ and $t_2$. $t_1$ refers to rapid death, or death within 48 hours, most likely due to cardiopulmonary collapse. $t_2$ refers to chronic kidney toxicity, observed with a peptide-oligomer-conjugate after multiple weekly doses. The MTD measurements described herein refer to $t_1$ toxicity (48 hour endpoint).

Thus, as measured from quadriceps samples, the TI of PPMO-8 is 16.6 (400 mg/kg MTD; 24 mg/kg $ED_{40}$). Although PPMO-4 has a lower MTD compared to PPMO-8, the effective dose is half that of PPMO-8, which results in a lower TI of 14.6. Likewise, PPMO-2 has a MTD *of ca.* 60 mg/kg, and an $ED_{40}$ of 10 mg/kg, which results in a TI of 6.

Unless otherwise defined, all technical and scientific terms used herein are accorded the meaning commonly known to one with ordinary skill in the art.

## Claims

**1.** A peptide-oligomer-conjugate of Formula I:

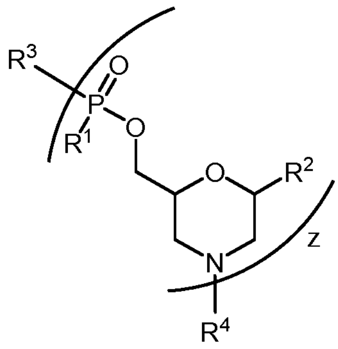

(I)

or a pharmaceutically acceptable salt thereof,
wherein:

$R^3$ is selected from OH, $-N(H)CH_2C(O)NH_2$, $-N(C_{1-6}\text{-alkyl})CH_2C(O)NH_2$,

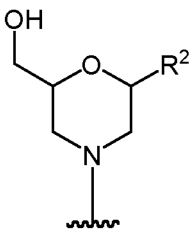
, and
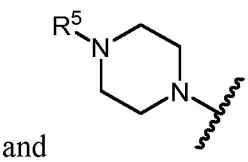
;

$R^5$ is $-C(O)(O\text{-alkyl})_xOH$, wherein x is 3-10 and each alkyl group is, independently at each occurrence, $C_{2-6}$-alkyl, or $R^5$ is selected from the group consisting of $-C(O)C_{1-6}$ alkyl, trityl, monomethoxytrityl, $-(C_{1-6}\text{-alkyl})R^6$, $-(C_{1-6}$ heteroalkyl)-$R^6$, aryl-$R^6$, heteroaryl-$R^6$, $-C(O)O-(C_{1-6}$ alkyl)-$R^6$, $-C(O)O$-aryl-$R^6$, $-C(O)O$-heteroaryl-$R^6$, and $R^{12}$;
$R^6$ is selected from OH, SH, and $NH_2$, or $R^6$ is O, S, or NH, covalently linked to a solid support;
$R^1$ is, independently at each occurrence, OH, $-NR^7R^{12}$, or $-NR^7R^8$;
each $R^7$ and $R^8$ are, independently at each occurrence, H or $-C_{1-6}$ alkyl;
$R^2$ is, independently at each occurrence, selected from the group consisting of H, a nucleobase and a nucleobase functionalized with a chemical protecting-group, wherein the nucleobase, independently at each occurrence, comprises a $C_{3-6}$ heterocyclic ring selected from pyridine, pyrimidine, triazinane, purine, and deaza-purine;
wherein the chemical protecting-group is selected from the group consisting of trityl, monomethoxytrityl, dimethoxytrityl, acetal, acetyl, tert-butyldimethylsilyl and benzyl for hydroxy groups,
wherein the chemical protecting-group is selected from the group consisting of trityl, monomethoxytrityl, dimethoxytrityl, acetal, tert-butyldimethylsilyl, methyl, ethyl, 2,4-dimethoxybenzyl and benzyl for carboxyl groups, and wherein the chemical protecting-group is selected from the group consisting of trifluoroacetyl, carboxybenzyl, tert-butyloxycarbonyl, silyloxycarbonyl and fluorenylmethyloxycarbonyl for amine groups;
z is 8-40;
$R^4$ is selected from H, $-C_{1-6}$ alkyl, $-C(O)C_{1-6}$ alkyl, benzoyl, stearoyl, trityl, monomethoxytrityl, dimethoxytrityl, trimethoxytrityl,

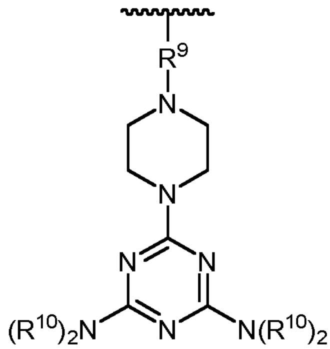, and $R^{12}$;

$R^9$ is $-C(O)(CH_2)_6C(O)-$ or $-C(O)(CH_2)_2S_2(CH_2)_2C(O)-$;
$R^{10}$ is $-(CH_2)_2OC(O)N((CH_2)_6N(H)C(=NH)NH_2)_2$;
$R^{11}$ is selected from OH and $-NR^7R^8$;
$R^{12}$ is selected from the group consisting of:

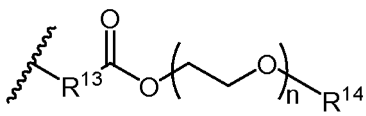, 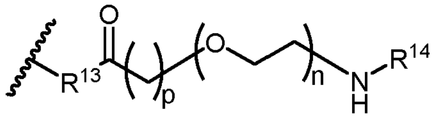,

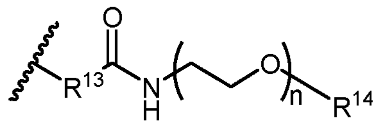, 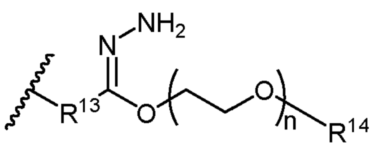,

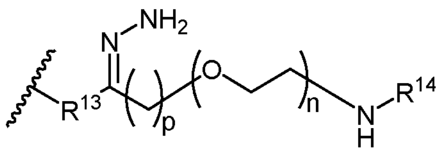, 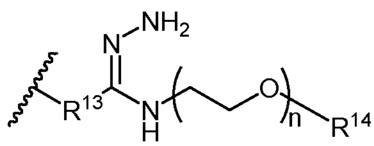, and

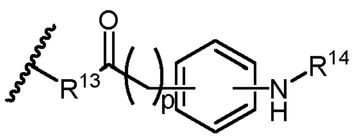;

n is 1, 2, 3, 4, 5, 6, 7, 8, 9, or 10;
p is 2, 3, 4, or 5;
$R^{13}$ is a bond, or $R^{13}$ is selected from the group consisting of:

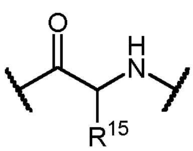, 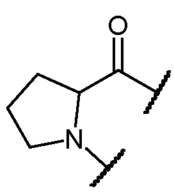, 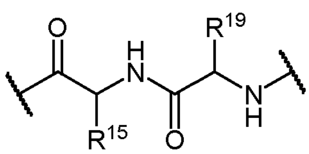,

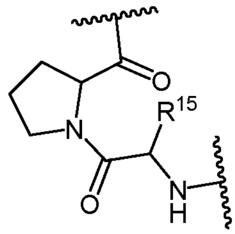, 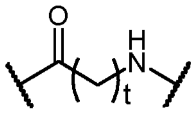, and 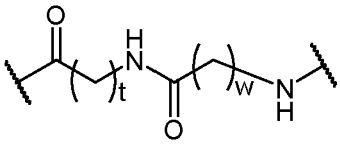;

$R^{15}$ and $R^{19}$ are, independently at each occurrence, selected from the group consisting of H, $-C_{1-4}$ alkyl, $-CH(-C_{1-4}$ alkyl$)_2$, and $-(CH_2)_3NH-C(=NH)-NH_2$;
t and w are, independently at each occurrence, 2, 3, 4, or 5;
$R^{14}$ is selected from the group consisting of:

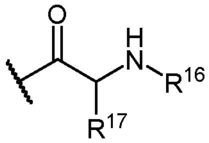, 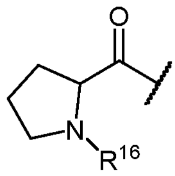, 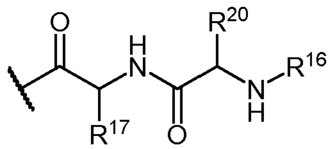,

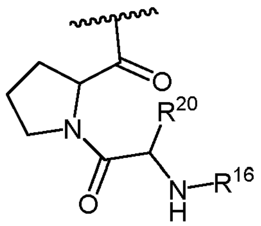, 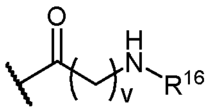, 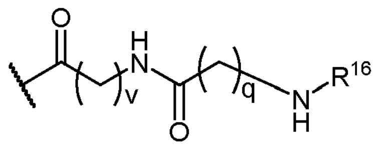,

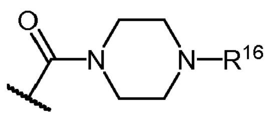, 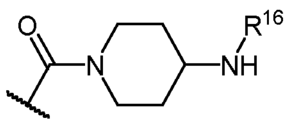, and 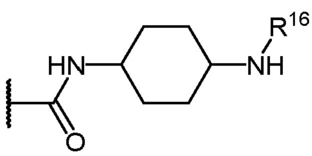;

$R^{17}$ is H or $-C_{1-4}$ alkyl;
$R^{20}$ is selected from the group consisting of H, $-C_{1-4}$ alkyl, $-CH(-C_{1-4}$ alkyl$)_2$, and $-(CH_2)_3NH-C(=NH)-NH_2$;
v and q are, independently at each occurrence, 2, 3, 4, or 5;
$R^{16}$ is selected from the group consisting of:

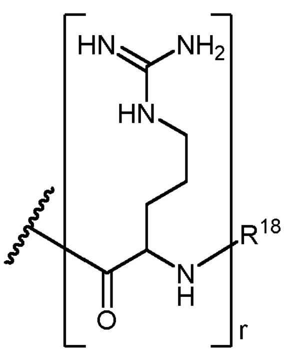, 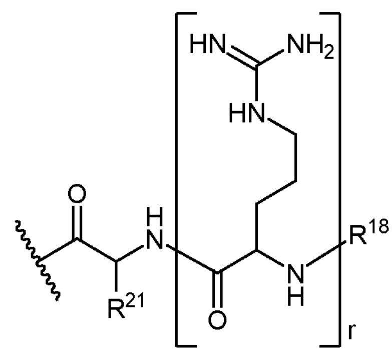, 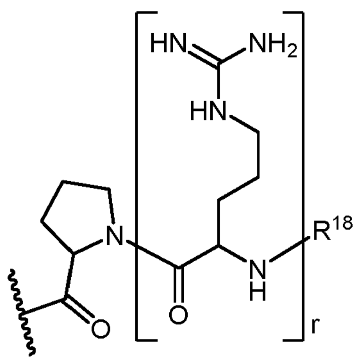,

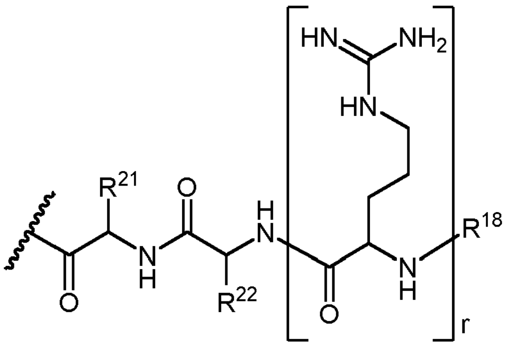, 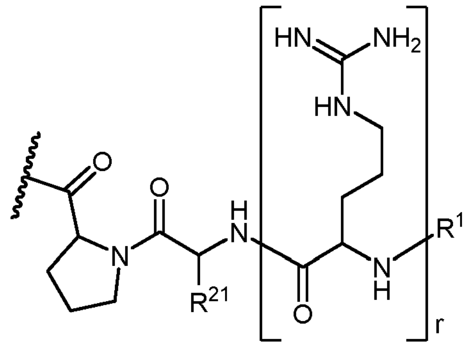,

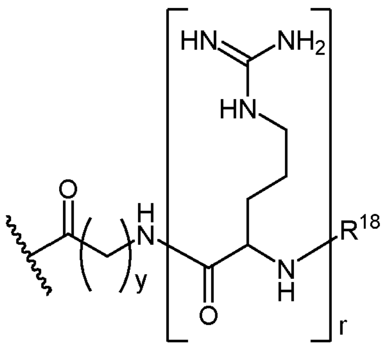, and 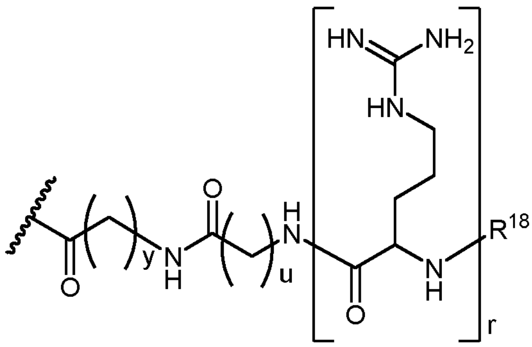;

$R^{21}$ and $R^{22}$ are, independently at each occurrence, H or $-C_{1-4}$ alkyl;
$R^{18}$ is selected from the group consisting of H, $-C(O)C_{1-6}$ alkyl, benzoyl, and stearoyl; r is 1, 2, 3, 4, 5, 6, 7, 8, or 9; and
y and u are, independently at each occurrence, 2, 3, 4, or 5;
provided that any one of the following conditions is present: 1) $R^1$ is $NR^7R^{12}$; or 2) $R^4$ is $R^{12}$; or 3) $R^3$ is

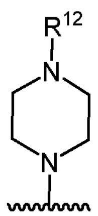.

2. The peptide-oligomer-conjugate of claim 1, or a pharmaceutically acceptable salt thereof, wherein $R^3$ is selected from -OH, $-N(C_{1-6}\text{-alkyl})CH_2C(O)NH_2$,

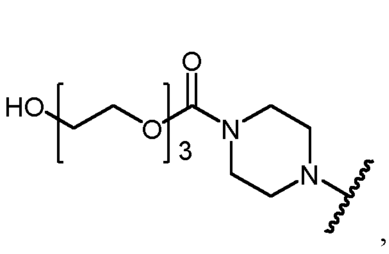, 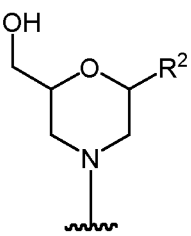, and 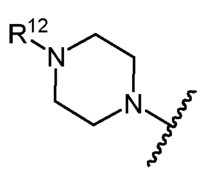.

3. The peptide-oligomer-conjugate of claim 1, or a pharmaceutically acceptable salt thereof, wherein the peptide-oligomer-conjugate of Formula I is a peptide-oligomer-conjugate of Formula Ia:

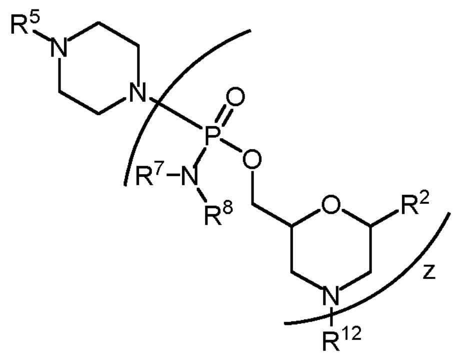

(Ia),

wherein $R^5$ is $-C(O)(O\text{-alkyl})_xOH$, wherein x is 3-10 and each alkyl group is, independently at each occurrence, $C_{2-6}$-alkyl, or $R^5$ is selected from the group consisting of $-C(O)C_{1-6}$ alkyl, trityl, and monomethoxytrityl.

**4.** The peptide-oligomer-conjugate of claim 1 or 3, or a pharmaceutically acceptable salt thereof, wherein $R^5$ is $-C(O)(O\text{-alkyl})_xOH$, wherein each alkyl group is, independently at each occurrence, $C_{2-6}$-alkyl.

**5.** The peptide-oligomer-conjugate of claim 1, or a pharmaceutically acceptable salt thereof, wherein the peptide-oligomer-conjugate of Formula I is a peptide-oligomer-conjugate of Formula Ib:

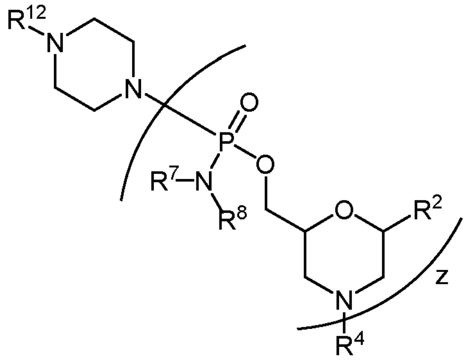

(Ib),

wherein $R^4$ is selected from H, $-C_{1-6}$ alkyl, $-C(O)C_{1-6}$ alkyl, benzoyl, stearoyl, trityl, monomethoxytrityl, dimethoxytrityl, and trimethoxytrityl.

**6.** The peptide-oligomer-conjugate of claim 1 or 5, or a pharmaceutically acceptable salt thereof, wherein $R^4$ is selected from H and $-C(O)CH_3$.

**7.** The peptide-oligomer-conjugate of any one of claims 1-6, or a pharmaceutically acceptable salt thereof, wherein r is 3, 4, 5, 6, 7, or 8, preferably wherein r is 5, 6, or 7, more preferably wherein r is 6.

**8.** The peptide-oligomer-conjugate of any one of claims 1-7, or a pharmaceutically acceptable salt thereof, wherein each $R^1$ is independently $NR^7R^8$, wherein each $R^7$ and $R^8$ are, independently at each occurrence, $C_{1-3}$-alkyl.

**9.** The peptide-oligomer-conjugate of claim 1, wherein the peptide-oligomer-conjugate of Formula I is a peptide-oligomer-conjugate of Formula Ic:

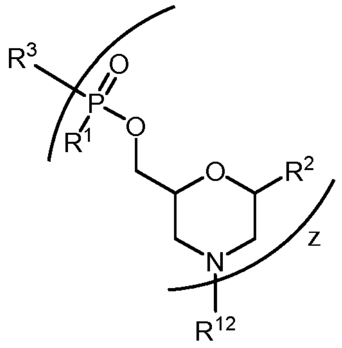

(Ic)

or a pharmaceutically acceptable salt thereof,
wherein:

$R^3$ is OH,

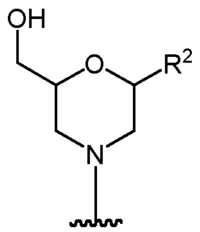, or 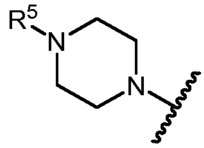;

$R^5$ is -C(O)(O-alkyl)$_x$OH, wherein x is 3-10 and each alkyl group is, independently at each occurrence, $C_{2-6}$-alkyl, or $R^5$ is -C(O)$C_{1-6}$ alkyl;
$R^1$ is, independently at each occurrence, OH or -$NR^7R^8$;
each $R^7$ and $R^8$ are independently at each occurrence -$C_{1-6}$ alkyl;
$R^2$ is, independently at each occurrence, selected from the group consisting of H, adenine, 2,6-diaminopurine, 7-deaza-adenine, guanine, 7-deaza-guanine, hypoxanthine, cytosine, 5-methyl-cytosine, thymine, and uracil;
z is 8-40;
$R^{12}$ is selected from the group consisting of:

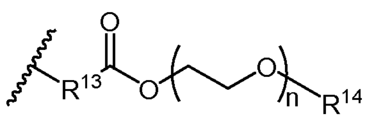, 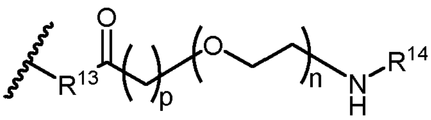,

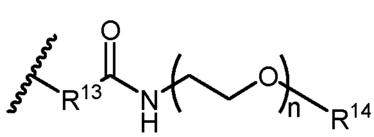, and 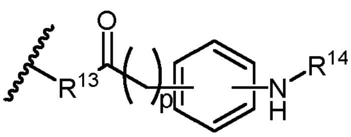;

n is 1, 2, 3, 4, 5, 6, 7, 8, 9, or 10;
p is 2, 3, 4, or 5;
$R^{13}$ is a bond;
$R^{14}$ is selected from the group consisting of:

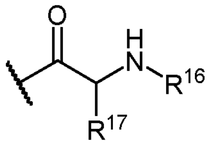 , 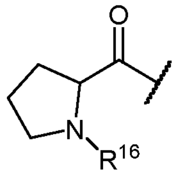 , and 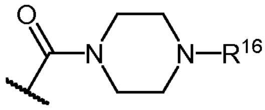 ;

$R^{17}$ is H or $-C_{1-4}$ alkyl;
$R^{16}$ is selected from the group consisting of:

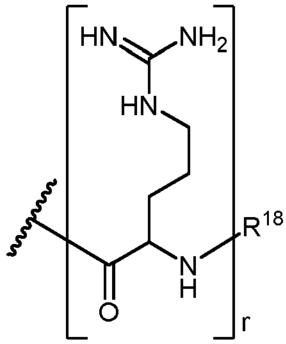 , 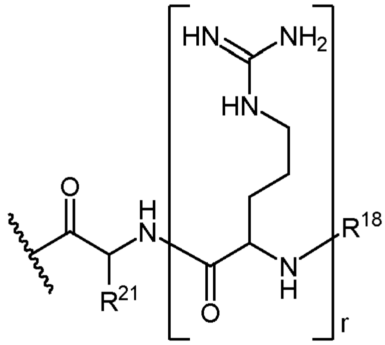 , and 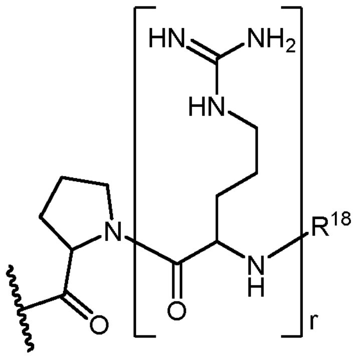 ;

$R^{21}$ is H or $-C_{1-4}$ alkyl;
$R^{18}$ is of H or $-C(O)C_{1-6}$ alkyl; and
r is 1, 2, 3, 4, 5, 6, 7, 8, or 9.

**10.** The peptide-oligomer-conjugate of any one of claims 1-9, or a pharmaceutically acceptable salt thereof, wherein $R^2$, independently at each occurrence, is selected from the group consisting of adenine, guanine, cytosine, 5-methyl-cytosine, thymine, uracil, and hypoxanthine.

**11.** The peptide-oligomer-conjugate of any one of claims 9 and 10, or a pharmaceutically acceptable salt thereof, wherein the peptide-oligomer-conjugate is selected from the group consisting of:

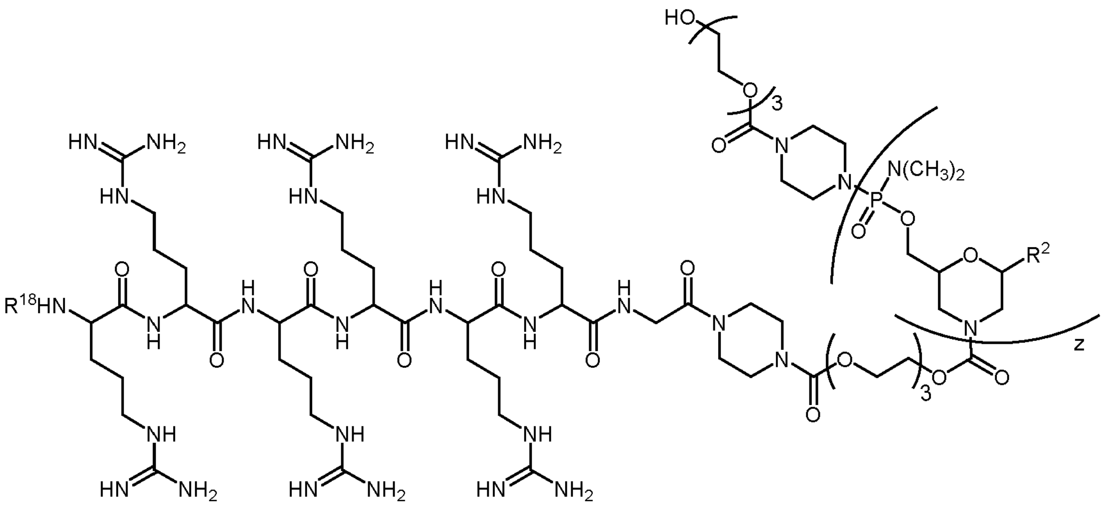 ,

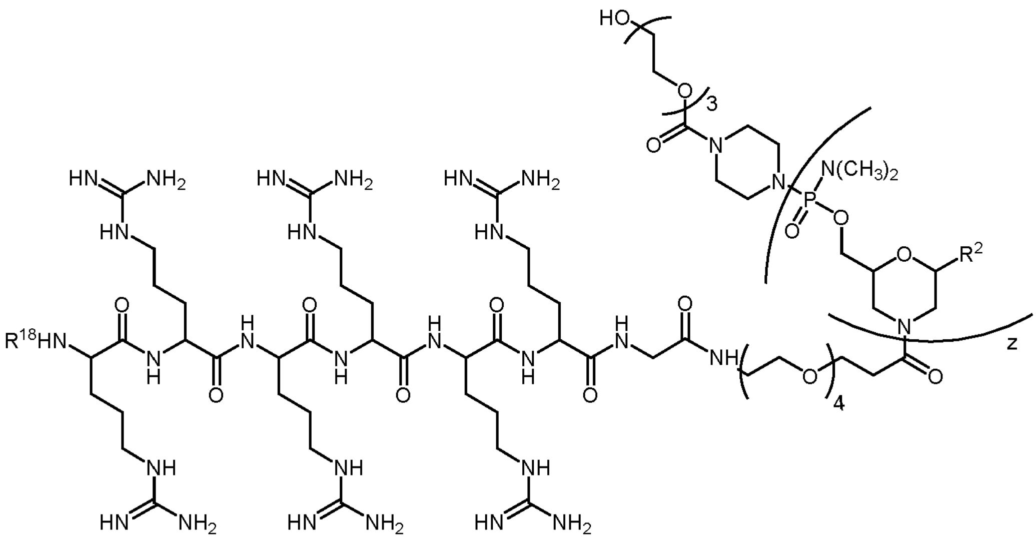

,

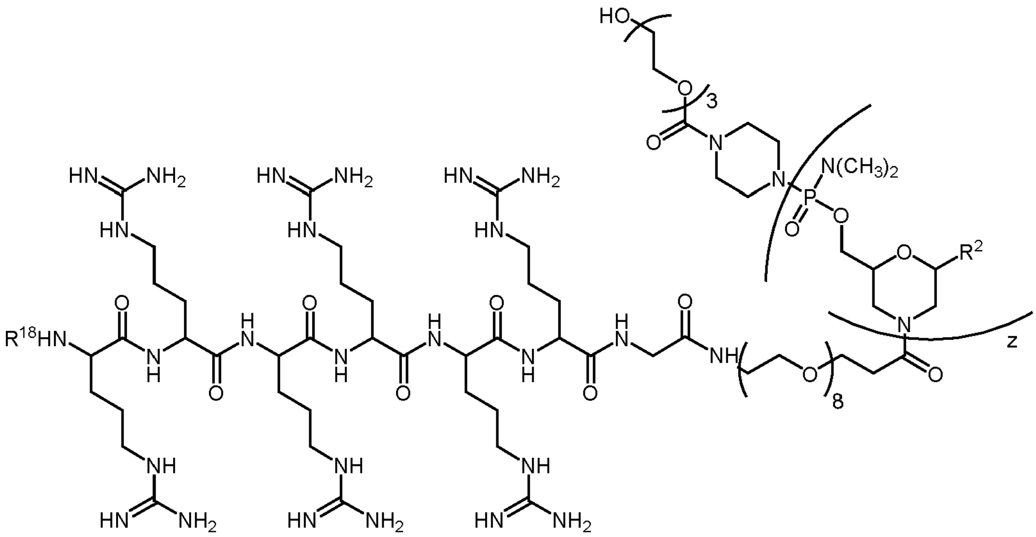

,

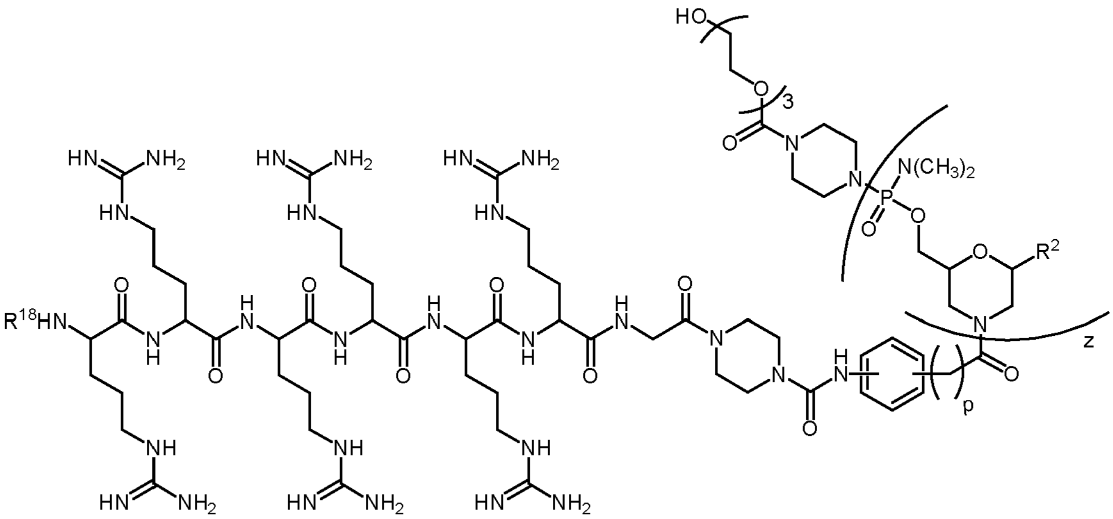

,

and

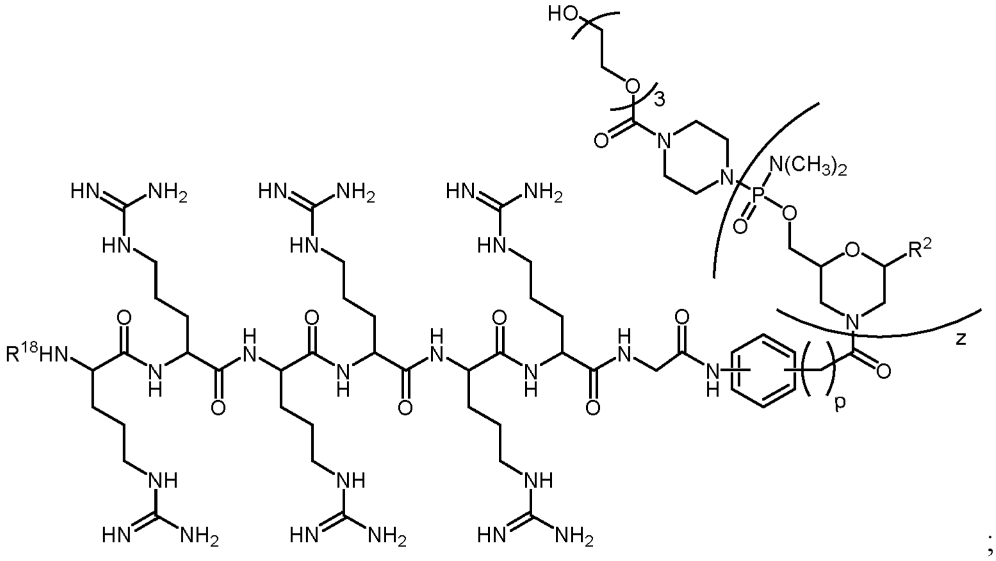

;

wherein
$R^{18}$ is selected from H and $-C(O)CH_3$.

**12.** The peptide-oligomer-conjugate of any one of claims 1-11, or a pharmaceutically acceptable salt thereof, wherein the peptide-oligomer-conjugate comprises a targeting sequence having 50%, 55%, 60%, 65%, 70%, 75%, 80%, 85%, 90%, 95%, 99% or 100% sequence complementarity to an RNA target.

**13.** The peptide-oligomer-conjugate of claim 12, or a pharmaceutically acceptable salt thereof, wherein the targeting sequence has 100% sequence complementarity to the RNA target.

**14.** A peptide-oligomer-conjugate of any one of claims 1-13 for use in treating a central nervous system disorder, a muscle disease, a viral infection, or a bacterial infection in a subject in need thereof.

**15.** The peptide-oligomer-conjugate for use of claim 14, wherein the muscle disease is Duchenne Muscular Dystrophy, or wherein the viral infection is caused by a virus selected from marburg virus, ebola virus, influenza virus, and dengue virus, or wherein the bacterial infection is caused by *Mycobacterium tuberculosis,* or wherein the central nervous system disorder is spinal muscular atrophy.

**Patentansprüche**

**1.** Peptid-Oligomer-Konjugat der Formel I:

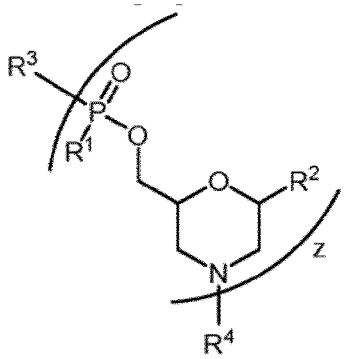

(I)

oder ein pharmazeutisch akzeptables Salz davon,

wobei:
$R^3$ ausgewählt ist aus OH, $-N(H)CH_2C(O)NH_2$, $-N(C_{1-6}\text{-Alkyl})\ CH_2C(O)NH_2$,

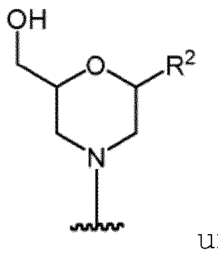
und
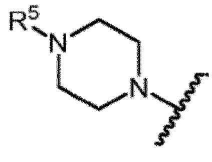
;

$R^5$ ausgewählt ist aus $-C(O)(O\text{-Alkyl})_xOH$, wobei x 3-10 ist und jede Alkylgruppe, unabhängig bei jedem Auftreten, $C_{2-6}$-Alkyl ist, oder $R^5$ ausgewählt ist aus der Gruppe bestehend aus $-C(O)C_{1-6}$-Alkyl, Trityl, Monomethoxytrityl, $-(C_{1-6}\text{-Alkyl})\text{-}R^6$, $-(C_{1-6}\text{-Heteroalkyl})\text{-}R^6$, Aryl-$R^6$, Heteroaryl-$R^6$, $-C(O)O\text{-}(C_{1-6}\text{-Alkyl})\text{-}R^6$, -C(O)O-Aryl-$R^6$, -C(O)O-Heteroaryl-$R^6$, und $R^{12}$;
$R^6$ ausgewählt ist aus OH, SH und $NH_2$ oder $R^6$ O, S oder NH ist, das kovalent an einen festen Träger gebunden ist;
$R^1$ unabhängig bei jedem Auftreten OH, $-NR^7R^{12}$ oder $-NR^7R^8$ ist;
jedes $R^7$ und $R^8$, unabhängig bei jedem Auftreten, H oder $-C_{1-6}$-Alkyl sind;
$R^2$, unabhängig bei jedem Auftreten, ausgewählt ist aus der Gruppe bestehend aus H, einer Nukleobase und einer mit einer chemischen Schutzgruppe funktionalisierten Nukleobase, wobei die Nukleobase, unabhängig bei jedem Auftreten, einen $C_{3-6}$-heterocyclischen Ring umfasst, ausgewählt aus Pyridin, Pyrimidin, Triazinan, Purin und Deaza-Purin;
wobei die chemische Schutzgruppe für Hydroxygruppen ausgewählt ist aus der Gruppe bestehend aus Trityl, Monomethoxytrityl, Dimethoxytrityl, Acetal, Acetyl, tert-Butyldimethylsilyl und Benzyl,
wobei die chemische Schutzgruppe für Carboxylgruppen ausgewählt ist aus der Gruppe bestehend aus Trityl, Monomethoxytrityl, Dimethoxytrityl, Acetal, tert-Butyldimethylsilyl, Methyl, Ethyl, 2,4-Dimethoxybenzyl und Benzyl, und
wobei die chemische Schutzgruppe für Amingruppen ausgewählt ist aus der Gruppe bestehend aus Trifluoracetyl, Carboxybenzyl, tert-Butyloxycarbonyl, Silyloxycarbonyl und Fluorenylmethyloxycarbonyl;
z 8-40 ist;
$R^4$ ausgewählt ist aus H, $-C_{1-6}$-Alkyl, $-C(O)C_{1-6}$-Alkyl, Benzoyl, Stearoyl, Trityl, Monomethoxytrityl, Dimethoxytrityl, Trimethoxytrityl,

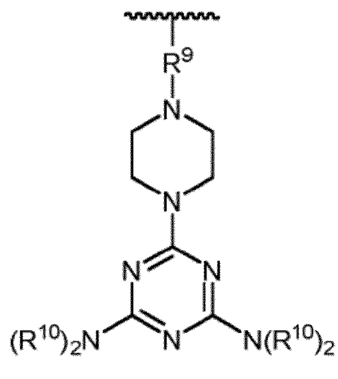
und $R^{12}$,

$R^9$ für $-C(O)(CH_2)_6C(O)$- oder $-C(O)(CH_2)_2S_2(CH_2)_2C(O)$-steht;
$R^{10}$ für $-(CH_2)_2OC(O)N((CH_2)_6N(H)C(=NH)NH_2)_2$ steht;
$R^{11}$ ausgewählt ist aus OH und $-NR^7R^8$;
$R^{12}$ ausgewählt ist aus der Gruppe bestehend aus:

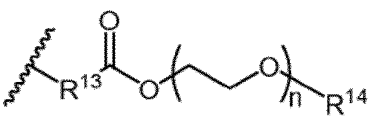,
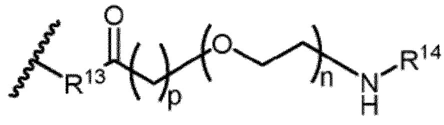,

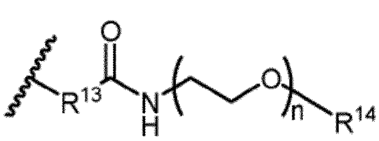,
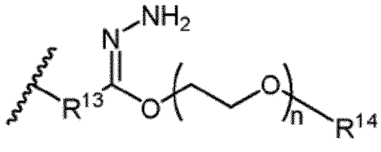,

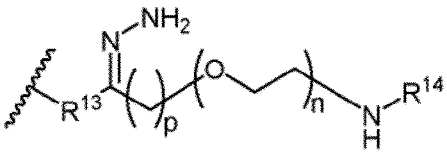,
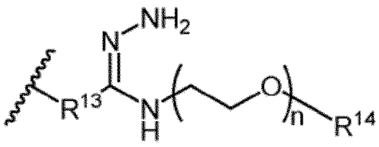

und

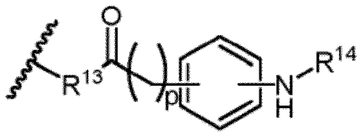;

n für 1, 2, 3, 4, 5, 6, 7, 8, 9 oder 10 steht;
p für 2, 3, 4 oder 5 steht;
$R^{13}$ eine Bindung ist oder $R^{13}$ ausgewählt ist aus der Gruppe bestehend aus:

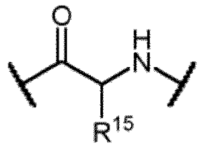,
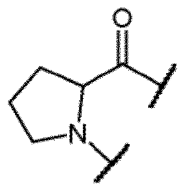,
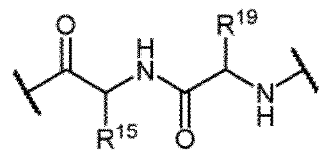,

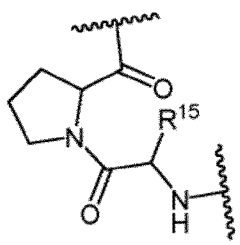,
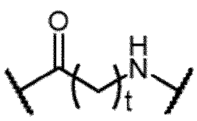
und
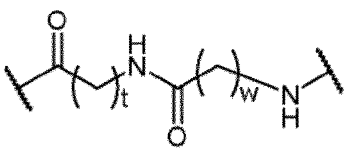;

$R^{15}$ und $R^{19}$ unabhängig bei jedem Auftreten ausgewählt sind aus der Gruppe bestehend aus H, $-C_{1-4}$-Alkyl, $-CH(-C_{1-4}\text{-Alkyl})_2$ und $-(CH_2)_3NH-C(=NH)-NH_2$;
t und w unabhängig bei jedem Auftreten 2, 3, 4 oder 5 sind;
$R^{14}$ ausgewählt ist aus der Gruppe bestehend aus:

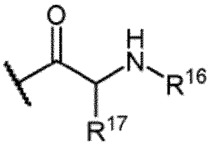, 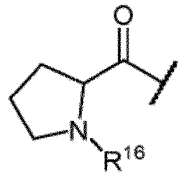, 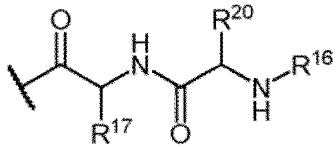,

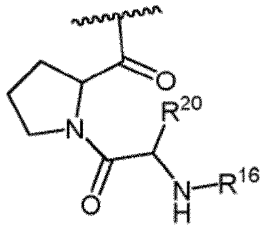, 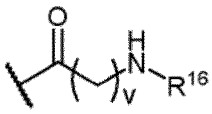, 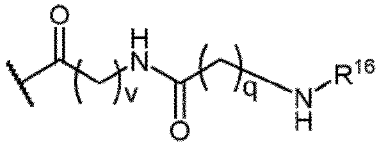,

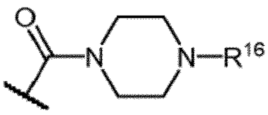, 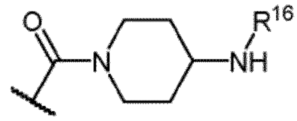 und 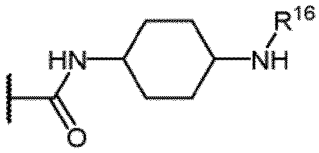;

$R^{17}$ H oder $-C_{1-4}$-Alkyl ist;
$R^{20}$ ausgewählt ist aus der Gruppe bestehend aus H, $-C_{1-4}$-Alkyl, $-CH(-C_{1-4}\text{-Alkyl})_2$ und $-(CH_2)_3NH-C(=NH)-NH_2$;
v und q unabhängig bei jedem Auftreten 2, 3, 4 oder 5 sind;
$R^{16}$ ausgewählt ist aus der Gruppe bestehend aus:

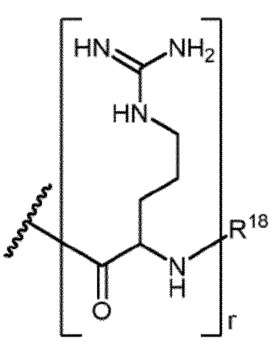, 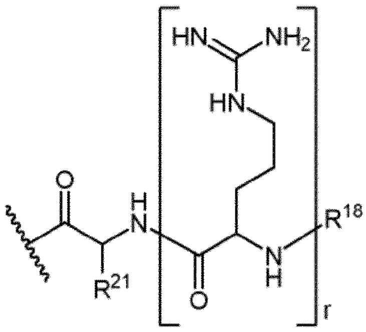, 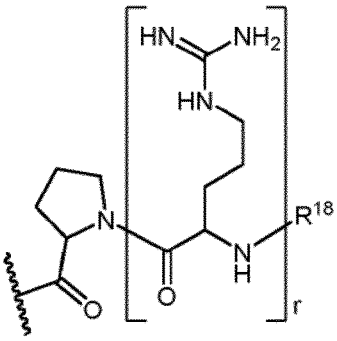,

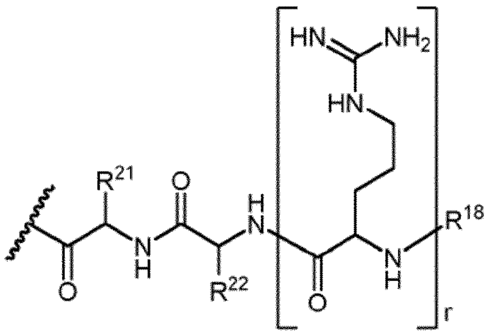, 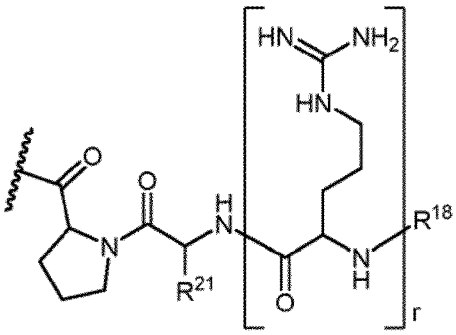,

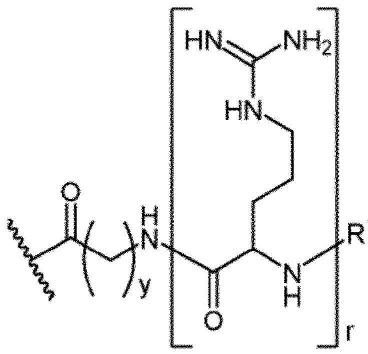 und 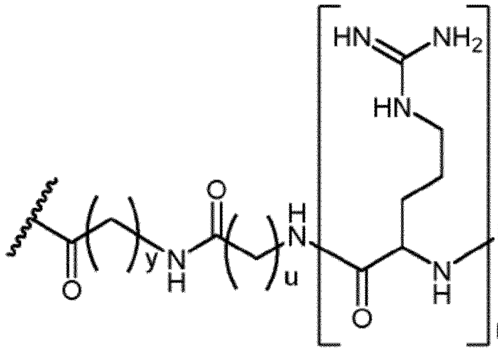 ;

$R^{21}$ und $R^{22}$ unabhängig bei jedem Auftreten H oder $-C_{1-4}$-Alkyl sind;
$R^{18}$ ausgewählt ist aus der Gruppe bestehend aus H, $-C(O)C_{1-6}$-Alkyl, Benzoyl und Stearoyl;
r für 1, 2, 3, 4, 5, 6, 7, 8 oder 9 steht; und
y und u unabhängig bei jedem Auftreten 2, 3, 4 oder 5 sind;
mit der Maßgabe, dass eine der folgenden Bedingungen vorliegt: 1) $R^1$ ist $NR^7R^{12}$; oder 2) $R^4$ ist $R^{12}$; oder 3) $R^3$ ist

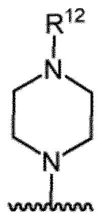.

2. Peptid-Oligomer-Konjugat nach Anspruch 1 oder ein pharmazeutisch akzeptables Salz davon, wobei $R^3$ ausgewählt ist aus -OH, $-N(C_{1-6}\text{-Alkyl})CH_2C(O)NH_2$,

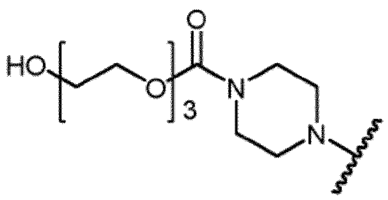, 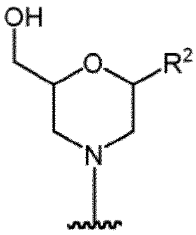 und 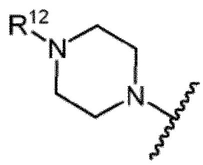.

3. Peptid-Oligomer-Konjugat nach Anspruch 1 oder ein pharmazeutisch akzeptables Salz davon, wobei das Peptid-Oligomer-Konjugat der Formel I ein Peptid-Oligomer-Konjugat der Formel Ia ist:

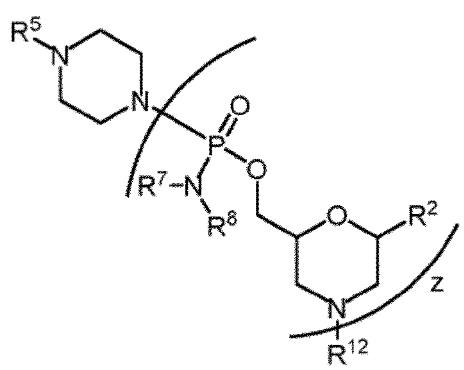

(Ia),

wobei $R^5$ $-C(O)(O\text{-Alkyl})_xOH$ ist, wobei x 3-10 ist und jede Alkylgruppe unabhängig bei jedem Auftreten $C_{2-6}$-Alkyl ist, oder $R^5$ ausgewählt ist aus der Gruppe bestehend aus $-C(O)C_{1-6}$-Alkyl, Trityl und Monomethoxytrityl.

**4.** Peptid-Oligomer-Konjugat nach Anspruch 1 oder 3, oder ein pharmazeutisch akzeptables Salz davon, wobei $R^5$ $-C(O)(O\text{-Alkyl})_xOH$ ist, wobei jede Alkylgruppe unabhängig bei jedem Auftreten $C_{2-6}$-Alkyl ist.

**5.** Peptid-Oligomer-Konjugat nach Anspruch 1 oder ein pharmazeutisch akzeptables Salz davon, wobei das Peptid-Oligomer-Konjugat der Formel I ein Peptid-Oligomer-Konjugat der Formel Ib ist:

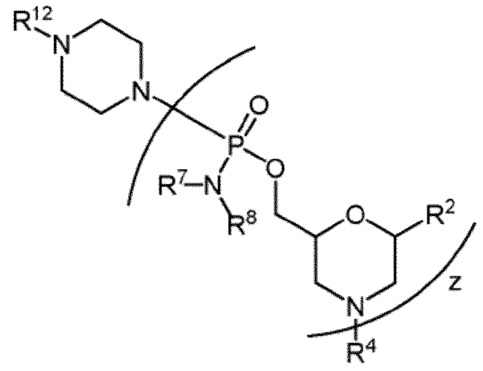

(Ib),

wobei $R^4$ ausgewählt ist aus H, $-C_{1-6}$-Alkyl, $-C(O)C_{1-6}$-Alkyl, Benzoyl, Stearoyl, Trityl, Monomethoxytrityl, Dimethoxytrityl und Trimethoxytrityl.

**6.** Peptid-Oligomer-Konjugat nach Anspruch 1 oder 5 oder ein pharmazeutisch akzeptables Salz davon, wobei $R^4$ ausgewählt ist aus H und $-C(O)CH_3$.

**7.** Peptid-Oligomer-Konjugat nach einem der Ansprüche 1-6 oder ein pharmazeutisch akzeptables Salz davon, wobei r 3, 4, 5, 6, 7 oder 8 ist, vorzugsweise wobei r 5, 6 oder 7 ist, stärker bevorzugt wobei r 6 ist.

**8.** Peptid-Oligomer-Konjugat nach einem der Ansprüche 1-7 oder ein pharmazeutisch akzeptables Salz davon, wobei jedes $R^1$ unabhängig $NR^7R^8$ ist, wobei jedes $R^7$ und $R^8$ unabhängig bei jedem Auftreten $C_{1-3}$-Alkyl sind.

**9.** Peptid-Oligomer-Konjugat nach Anspruch 1, wobei das Peptid-Oligomer-Konjugat der Formel I ein Peptid-Oligomer-Konjugat der Formel Ic:

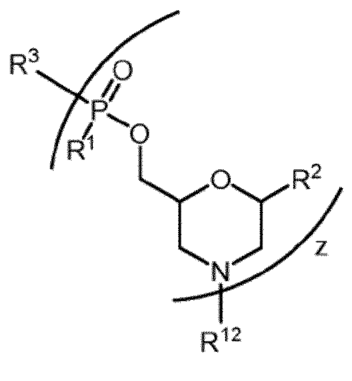

(Ic)

oder ein pharmazeutisch akzeptables Salz davon ist,
wobei:

$R^3$ OH,

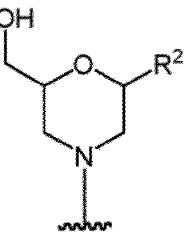

oder

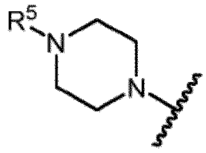

ist;

$R^5$ -C(O)(O-Alkyl)$_x$OH ist, wobei x 3-10 ist und jede Alkylgruppe unabhängig bei jedem Auftreten $C_{2-6}$-Alkyl ist, oder $R^5$ -C(O)$C_{1-6}$-Alkyl ist;

$R^1$ unabhängig bei jedem Auftreten OH oder -$NR^7R^8$ ist; jedes $R^7$ und $R^8$ unabhängig bei jedem Auftreten -$C_{1-6}$-Alkyl sind;

$R^2$ unabhängig bei jedem Auftreten ausgewählt ist aus der Gruppe bestehend aus H, Adenin, 2,6-Diaminopurin, 7-Deaza-Adenin, Guanin, 7-Deaza-Guanin, Hypoxanthin, Cytosin, 5-Methyl-Cytosin, Thymin, und Uracil;

z 8-40 ist;

$R^{12}$ ausgewählt ist aus der Gruppe bestehend aus:

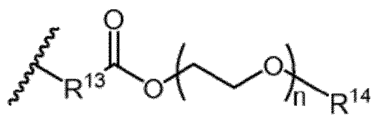

,

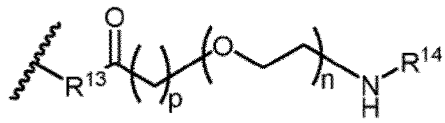

,

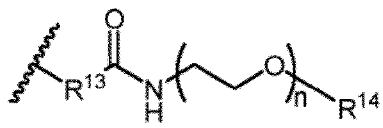

und

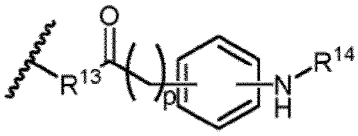

;

n für 1, 2, 3, 4, 5, 6, 7, 8, 9 oder 10 steht;

p für 2, 3, 4 oder 5 steht;

$R^{13}$ eine Bindung ist;

$R^{14}$ ausgewählt ist aus der Gruppe bestehend aus:

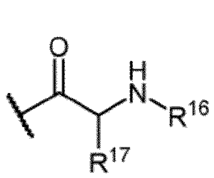

,

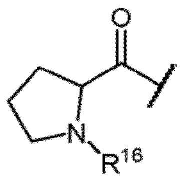

und

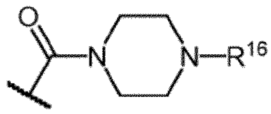

;

$R^{17}$ H oder -$C_{1-4}$-Alkyl ist;

$R^{16}$ ausgewählt ist aus der Gruppe bestehend aus:

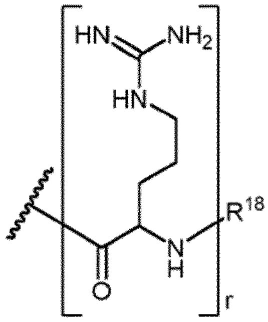

,

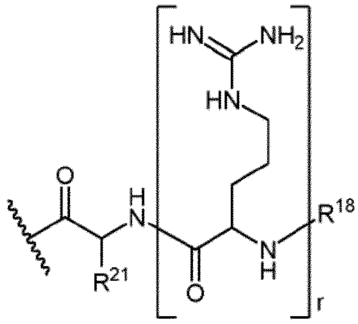

und

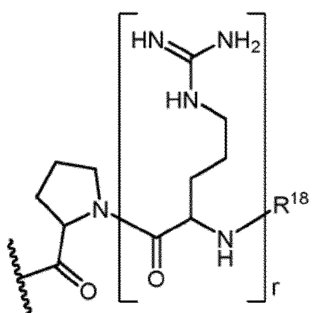

;

$R^{21}$ H oder -$C_{1-4}$-Alkyl ist;
$R^{18}$ H oder -C(O)$C_{1-6}$-Alkyl ist; und
r 1, 2, 3, 4, 5, 6, 7, 8 oder 9 ist.

**10.** Peptid-Oligomer-Konjugat nach einem der Ansprüche 1-9 oder ein pharmazeutisch akzeptables Salz davon, wobei $R^2$ unabhängig bei jedem Auftreten ausgewählt ist aus der Gruppe bestehend aus Adenin, Guanin, Cytosin, 5-Methyl-Cytosin, Thymin, Uracil und Hypoxanthin.

**11.** Peptid-Oligomer-Konjugat nach einem der Ansprüche 9 und 10 oder ein pharmazeutisch akzeptables Salz davon, wobei das Peptid-Oligomer-Konjugat ausgewählt ist aus der Gruppe bestehend aus:

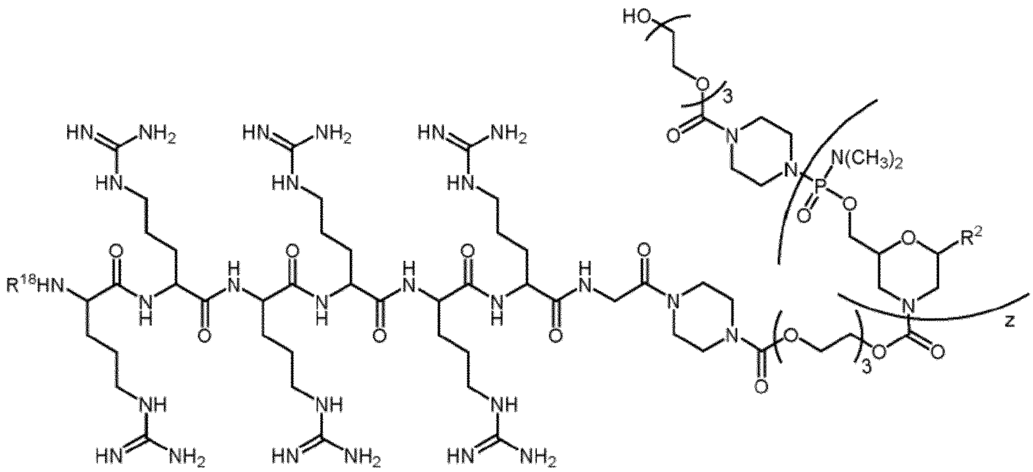

,

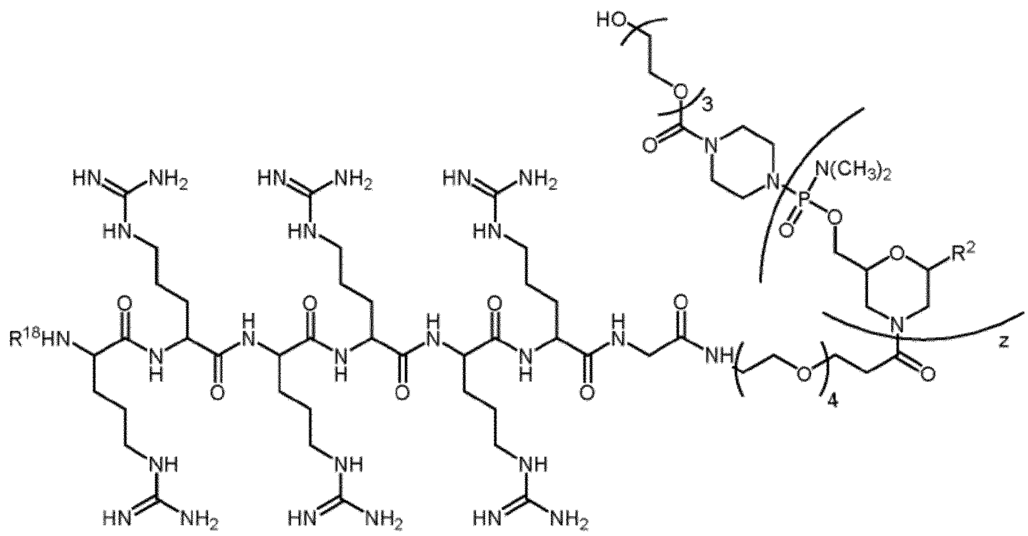

,

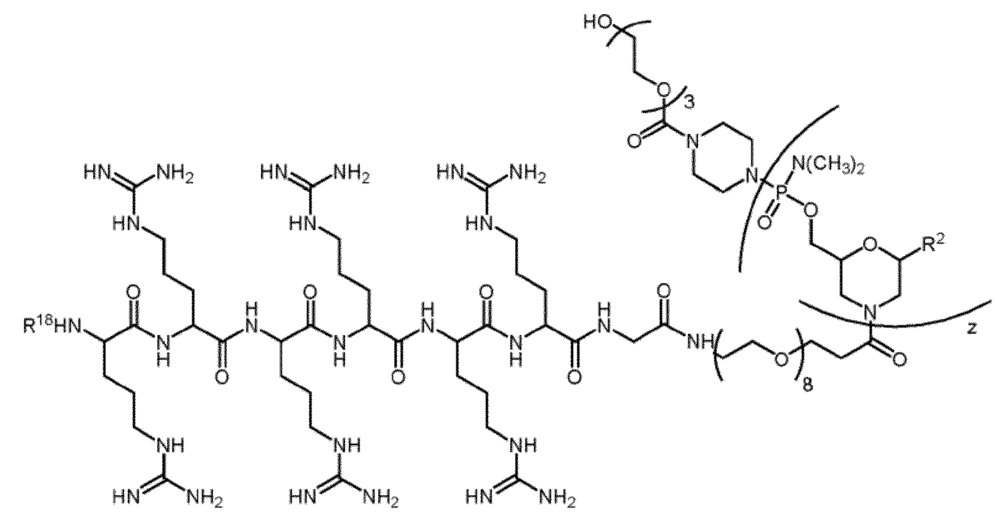

,

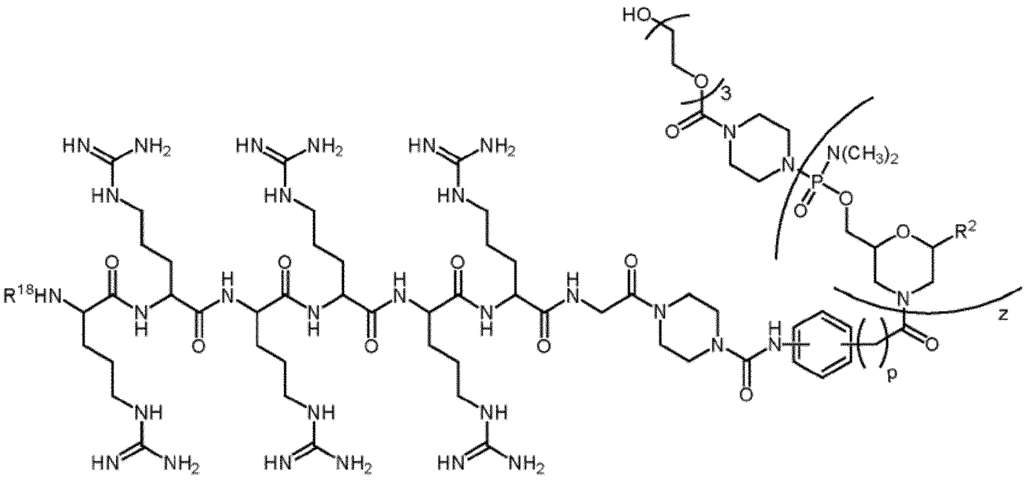

und

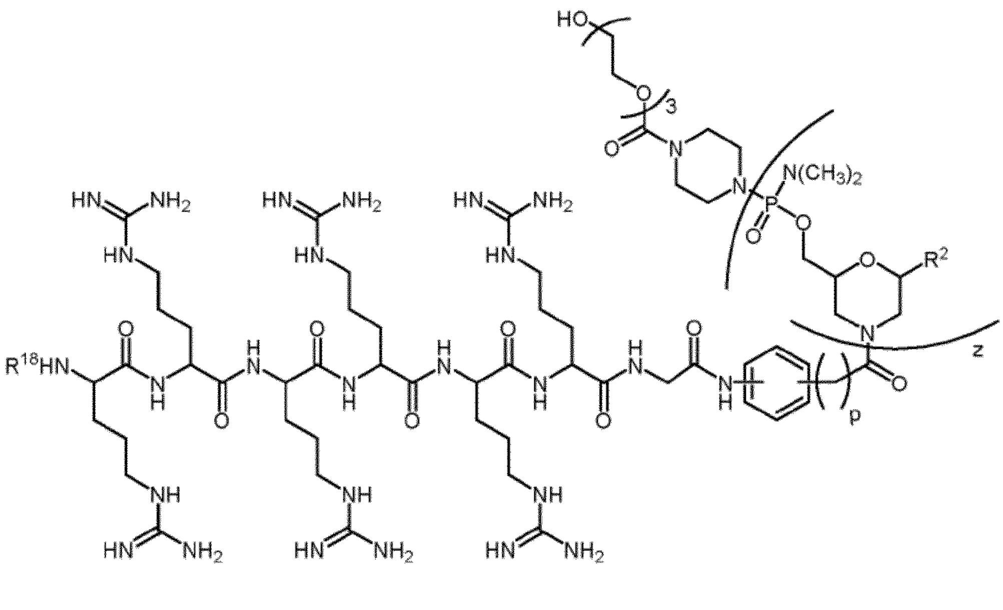

;

wobei

$R^{18}$ aus H und $-C(O)CH_3$ ausgewählt ist.

12. Peptid-Oligomer-Konjugat nach einem der Ansprüche 1-11, oder ein pharmazeutisch akzeptables Salz davon, wobei das Peptid-Oligomer-Konjugat eine Targeting-Sequenz umfasst, welche 50%, 55%, 60%, 65%, 70%, 75%, 80%, 85%, 90%, 95%, 99% oder 100% Sequenzkomplementarität zu einem RNA-Ziel aufweist.

13. Peptid-Oligomer-Konjugat nach Anspruch 12 oder ein pharmazeutisch akzeptables Salz davon, wobei die Targeting-Sequenz 100% Sequenzkomplementarität zum RNA-Ziel aufweist.

14. Peptid-Oligomer-Konjugat nach einem der Ansprüche 1-13 zur Verwendung bei der Behandlung einer Störung des zentralen Nervensystems, einer Muskelerkrankung, einer Virusinfektion oder einer bakteriellen Infektion in einem Subjekt, das diese benötigt.

15. Peptid-Oligomer-Konjugat zur Verwendung nach Anspruch 14, wobei die Muskelerkrankung Duchenne-Muskeldystrophie ist oder wobei die Virusinfektion durch ein Virus, ausgewählt aus Marburg-Virus, Ebola-Virus, Influenza-Virus und Dengue-Virus, verursacht wird, oder wobei die bakterielle Infektion durch *Mycobacterium tuberculosis* verursacht wird oder wobei die Störung des zentralen Nervensystems spinale Muskelatrophie ist.

## Revendications

1. Conjugué peptide-oligomère de formule I :

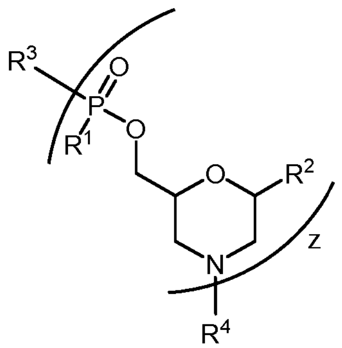

(I)

ou sel pharmaceutiquement acceptable de celui-ci,
dans lequel :
$R^3$ est choisi parmi OH, $-N(H)CH_2C(O)NH_2$, -N(alkyle en $C_{1-6}$) $CH_2C(O)NH_2$,

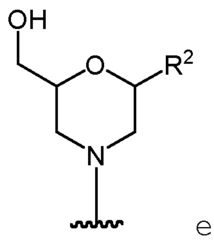

et

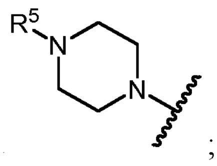

;

$R^5$ est -C(O) $(O\text{-alkyl})_xOH$, où x est 3 à 10 et chaque groupe alkyle est, indépendamment à chaque occurrence, alkyle en $C_{2-6}$, ou $R^5$ est choisi dans le groupe constitué de -C(O) (alkyle en $C_{1-6}$), trityle, monométhoxytrityle, -(alkyle en $C_{1-6}$)$R^6$, -(hétéroalkyle en $C_{1-6}$)-$R^6$, aryl-$R^6$, hétéroaryl-$R^6$, -C(O)O-(alkyle en $C_{1-6}$) -$R^6$, -C(O)O-aryl-$R^6$, -C(O)O-hétéroaryl-$R^6$ et $R^{12}$ ;
$R^6$ est choisi parmi OH, SH et $NH_2$, ou $R^6$ est O, S ou NH, lié de façon covalente à un support solide ;
$R^1$ est, indépendamment à chaque occurrence, OH, $-NR^7R^{12}$ ou $-NR^7R^8$ ;
chaque $R^7$ et $R^8$ sont, indépendamment à chaque occurrence, H ou - (alkyle en $C_{1-6}$) ;
$R^2$ est, indépendamment à chaque occurrence, choisi dans le groupe constitué de H, une base azotée et une

base azotée fonctionnalisée avec un groupe protecteur chimique, où la base azotée, indépendamment à chaque occurrence, comprend un cycle hétérocyclique en $C_{3-6}$ choisi parmi pyridine, pyrimidine, triazinane, purine et déaza-purine ;

dans lequel le groupe protecteur chimique est choisi dans le groupe constitué de trityle, monométhoxytrityle, diméthoxytrityle, acétal, acétyle, tert-butyldiméthylsilyle et benzyle pour des groupes hydroxy,

dans lequel le groupe protecteur chimique est choisi dans le groupe constitué de trityle, monométhoxytrityle, diméthoxytrityle, acétal, tert-butyldiméthylsilyle, méthyle, éthyle, 2,4-diméthoxybenzyle et benzyle pour des groupes carboxyle, et

dans lequel le groupe protecteur chimique est choisi dans le groupe constitué de trifluoroacétyle, carboxybenzyle, tert-butyloxycarbonyle, silyloxycarbonyle et fluorénylméthyloxycarbonyle pour des groupes aminé ;

z est 8 à 40 ;

$R^4$ est choisi parmi H, -(alkyle en $C_{1-6}$), -C(O) (alkyle en $C_{1-6}$), benzoyle, stéaroyle, trityle, monométhoxytrityle, diméthoxytrityle, triméthoxytrityle,

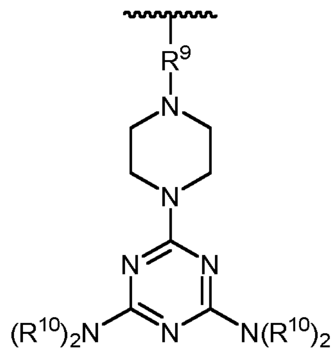 et $R^{12}$ ;

$R^9$ est $-C(O)(CH_2)_6C(O)-$ ou $-C(O)(CH_2)_2S_2(CH_2)_2C(O)-$ ;

$R^{10}$ est $-(CH_2)_2OC(O)N((CH_2)_6N(H)C(=NH)NH_2)_2$ ;

$R^{11}$ est choisi parmi OH et $-NR^7R^8$ ;

$R^{12}$ est choisi dans le groupe constitué de :

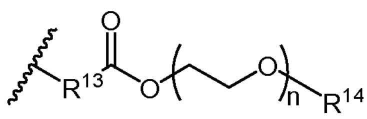, 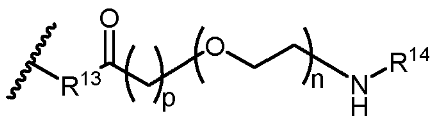,

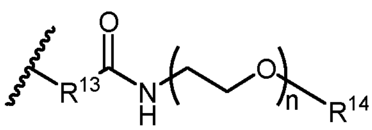, 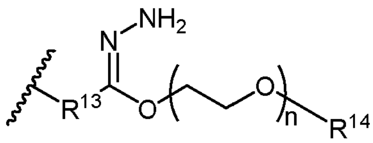,

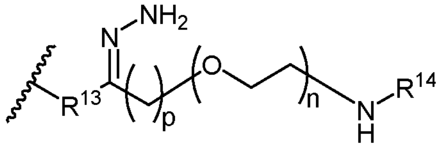, 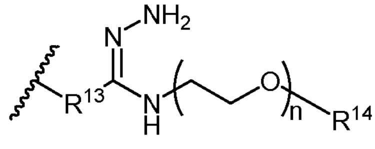 et

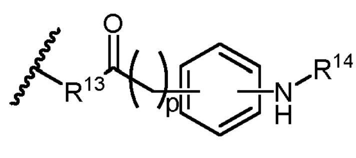 ;

n est 1, 2, 3, 4, 5, 6, 7, 8, 9 ou 10 ;

p est 2, 3, 4 ou 5 ;

$R^{13}$ est une liaison, ou $R^{13}$ est choisi dans le groupe constitué de :

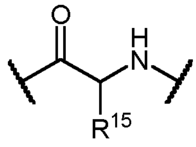 , 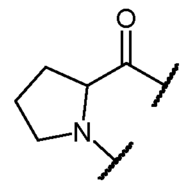 , 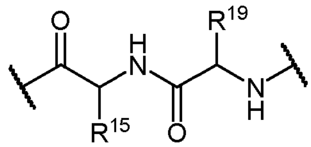 ,

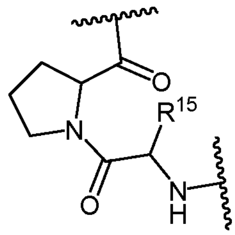 , 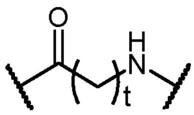 et 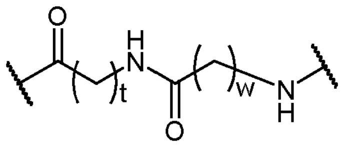 ;

$R^{15}$ et $R^{19}$ sont, indépendamment à chaque occurrence, choisis dans le groupe constitué de H, -(alkyle en $C_{1-4}$), -CH (-alkyle en $C_{1-4})_2$, et $-(CH_2)_3NH-C(=NH)-NH_2$ ;

t et w sont, indépendamment à chaque occurrence, 2, 3, 4 ou 5 ;

$R^{14}$ est choisi dans le groupe constitué de :

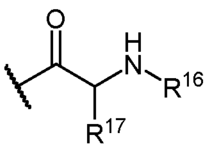 , 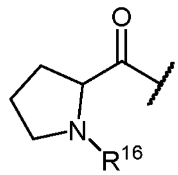 , 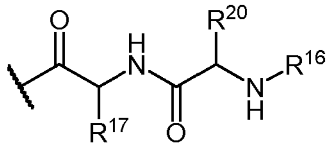 ,

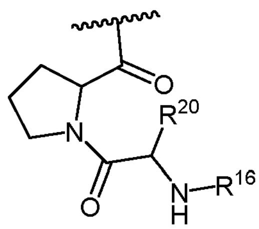 , 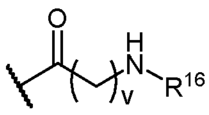 , 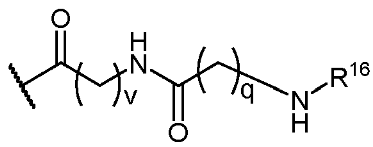 ,

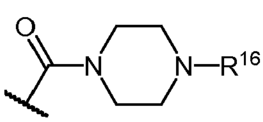 , 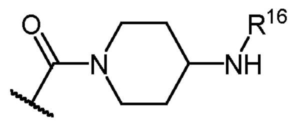 et 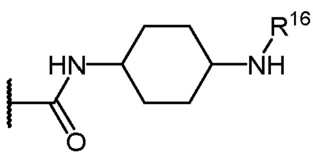 ;

$R^{17}$ est H ou -(alkyle en $C_{1-4}$) ;

$R^{20}$ est choisi dans le groupe constitué de H, -(alkyle en $C_{1-4}$), -CH (-alkyle en $C_{1-4})_2$, et $-(CH_2)_3NH-C(=NH)-NH_2$ ;

v et q sont, indépendamment à chaque occurrence, 2, 3, 4 ou 5 ;

$R^{16}$ est choisi dans le groupe constitué de :

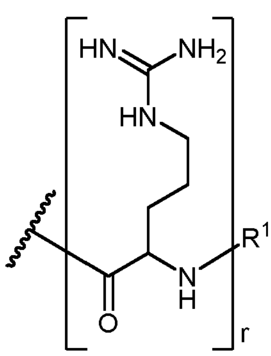,
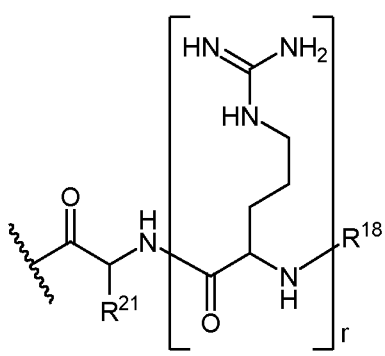,
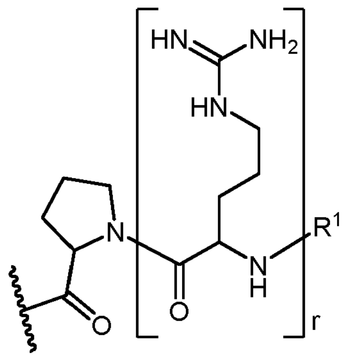,

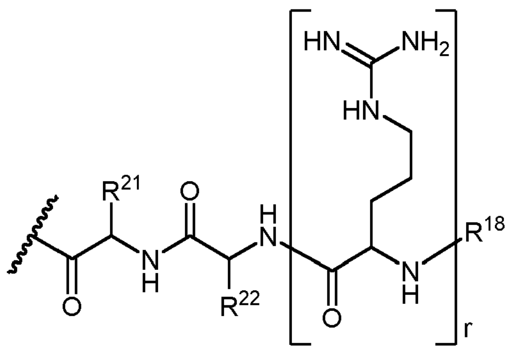,
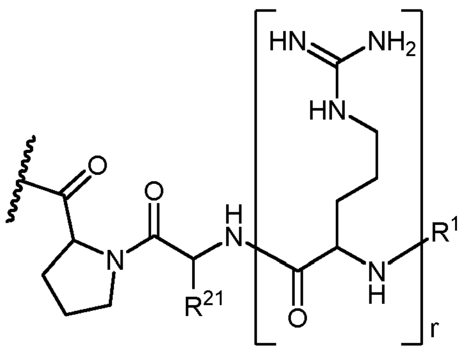,

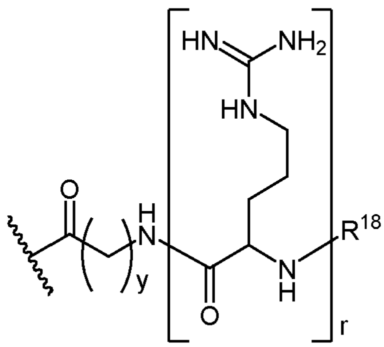 et
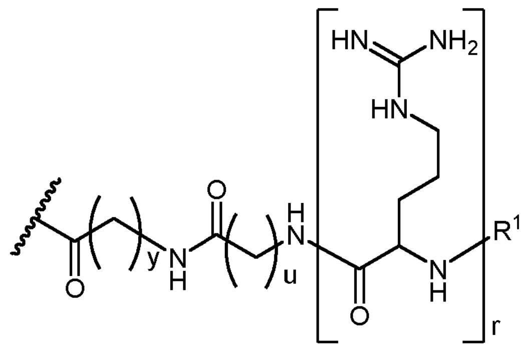;

$R^{21}$ et $R^{22}$ sont, indépendamment à chaque occurrence, H ou -(alkyle en $C_{1-4}$) ;
$R^{18}$ est choisi dans le groupe constitué de H, -C(O)(alkyle en $C_{1-6}$), benzoyle et stéaroyle ;
r est 1, 2, 3, 4, 5, 6, 7, 8, ou 9 ; et
y et u sont, indépendamment à chaque occurrence, 2, 3, 4 ou 5 ;
à condition que l'une quelconque des conditions suivantes soit présente : 1) $R^1$ est $NR^7R^{12}$ ; ou 2) $R^4$ est $R^{12}$ ; ou 3) $R^3$ est

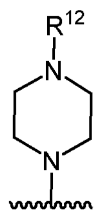.

2. Conjugué peptide-oligomère selon la revendication 1, ou sel pharmaceutiquement acceptable de celui-ci, dans lequel $R^3$ est choisi parmi -OH, -N(alkyle en $C_{1-6}$)$CH_2C(O)NH_2$,

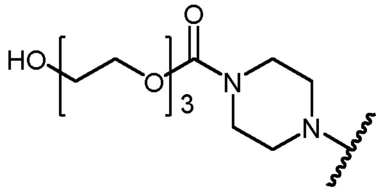 , 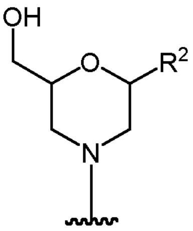 et 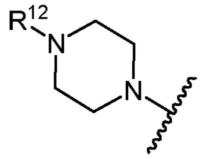 .

3. Conjugué peptide-oligomère selon la revendication 1, ou sel pharmaceutiquement acceptable de celui-ci, où le conjugué peptide-oligomère de formule I est un conjugué peptide-oligomère de formule Ia :

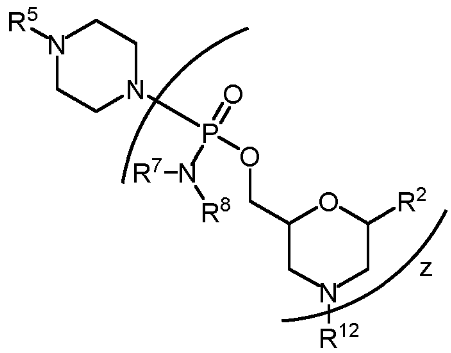

(Ia),

dans lequel $R^5$ est -C(O)(O-alkyl)$_x$OH, où x est 3 à 10 et chaque groupe alkyle est, indépendamment à chaque occurrence, alkyle en $C_{2-6}$, ou $R^5$ est choisi dans le groupe constitué de -C(O) (alkyle en $C_{1-6}$), trityle et monométhoxytrityle.

4. Conjugué peptide-oligomère selon la revendication 1 ou 3, ou sel pharmaceutiquement acceptable de celui-ci, dans lequel $R^5$ est -C(O) (O-alkyl)$_x$OH, où chaque groupe alkyle est, indépendamment à chaque occurrence, alkyle en $C_{2-6}$.

5. Conjugué peptide-oligomère selon la revendication 1, ou sel pharmaceutiquement acceptable de celui-ci, où le conjugué peptide-oligomère de formule I est un conjugué peptide-oligomère de formule Ib :

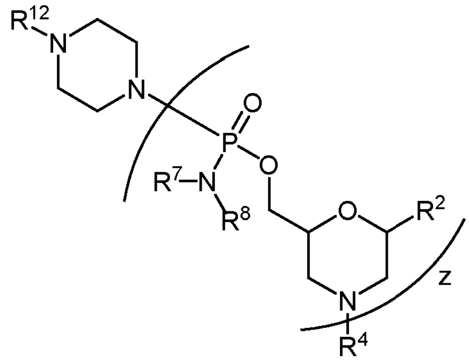

(Ib),

dans lequel $R^4$ est choisi parmi H, -(alkyle en $C_{1-6}$), -C(O)(alkyle en $C_{1-6}$), benzoyle, stéaroyle, trityle, monométhoxytrityle, diméthoxytrityle et triméthoxytrityle.

6. Conjugué peptide-oligomère selon la revendication 1 ou 5, ou sel pharmaceutiquement acceptable de celui-ci, dans lequel $R^4$ est choisi parmi H et -C(O)$CH_3$.

7. Conjugué peptide-oligomère selon l'une quelconque des revendications 1 à 6, ou sel pharmaceutiquement acceptable de celui-ci, dans lequel r est 3, 4, 5, 6, 7 ou 8, de préférence dans lequel r est 5, 6 ou 7, plus préférablement dans lequel r est 6.

**8.** Conjugué peptide-oligomère selon l'une quelconque des revendications 1 à 7, ou sel pharmaceutiquement acceptable de celui-ci, dans lequel
chaque $R^1$ est indépendamment $NR^7R^8$, où chaque $R^7$ et $R^8$ sont, indépendamment à chaque occurrence, alkyle en $C_{1-3}$.

**9.** Conjugué peptide-oligomère selon la revendication 1, où le conjugué peptide-oligomère de formule I est un conjugué peptide-oligomère de formule Ic :

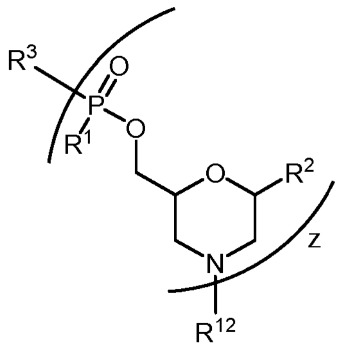

(Ic)

ou un sel pharmaceutiquement acceptable de celui-ci, dans lequel :

$R^3$ est OH,

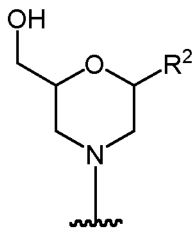
ou
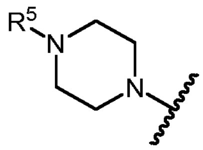
;

$R^5$ est -C(O)(O-alkyl)$_x$OH, où x est 3 à 10 et chaque groupe alkyle est, indépendamment à chaque occurrence, alkyle en $C_{2-6}$, ou $R^5$ est -C(O)(alkyle en $C_{1-6}$) ;
$R^1$ est, indépendamment à chaque occurrence, OH ou $-NR^7R^8$ ; chaque $R^7$ et $R^8$ sont, indépendamment à chaque occurrence, - (alkyle en $C_{1-6}$) ;
$R^2$ est, indépendamment à chaque occurrence, choisi dans le groupe constitué de H, adénine, 2,6-diaminopurine, 7-déaza-adénine, guanine, 7-déaza-guanine, hypoxanthine, cytosine, 5-méthyl-cytosine, thymine, et uracile ;
z est 8 à 40 ;
$R^{12}$ est choisi dans le groupe constitué de :

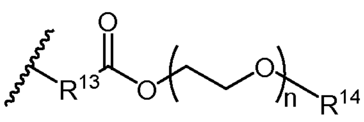
,
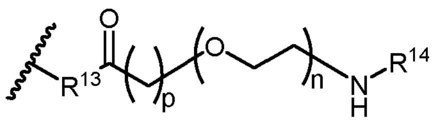
,

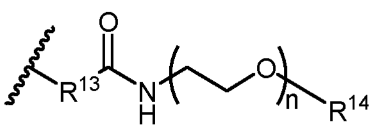 et 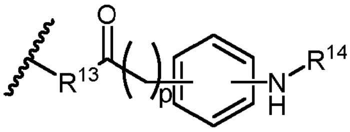 ;

n est 1, 2, 3, 4, 5, 6, 7, 8, 9 ou 10 ;
p est 2, 3, 4 ou 5 ;
$R^{13}$ est une liaison ;
$R^{14}$ est choisi dans le groupe constitué de :

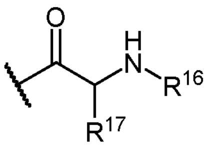 , 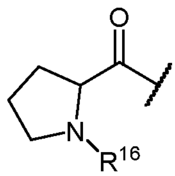 et 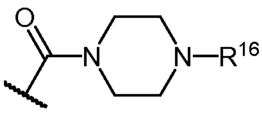 ;

$R^{17}$ est H ou - (alkyle en $C_{1-4}$) ;
$R^{16}$ est choisi dans le groupe constitué de :

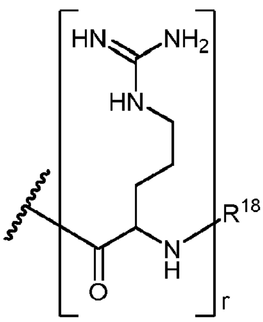 , 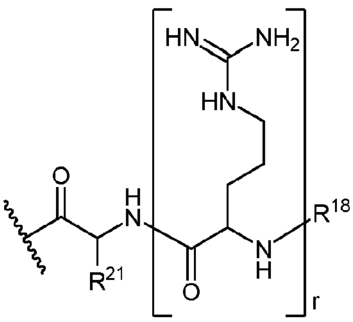 et 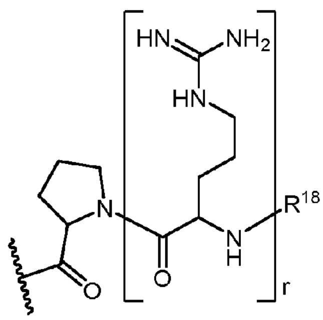 ;

$R^{21}$ est H ou -(alkyle en $C_{1-4}$) ;
$R^{18}$ est H ou -C(O)(alkyle en $C_{1-6}$) ; et
r est 1, 2, 3, 4, 5, 6, 7, 8 ou 9.

**10.** Conjugué peptide-oligomère selon l'une quelconque des revendications 1 à 9, ou sel pharmaceutiquement acceptable de celui-ci, dans lequel $R^2$, indépendamment à chaque occurrence, est choisi dans le groupe constitué d'adénine, guanine, cytosine, 5-méthyl-cytosine, thymine, uracile et hypoxanthine.

**11.** Conjugué peptide-oligomère selon l'une quelconque des revendications 9 et 10, ou sel pharmaceutiquement acceptable de celui-ci, où le conjugué peptide-oligomère est choisi dans le groupe constitué de :

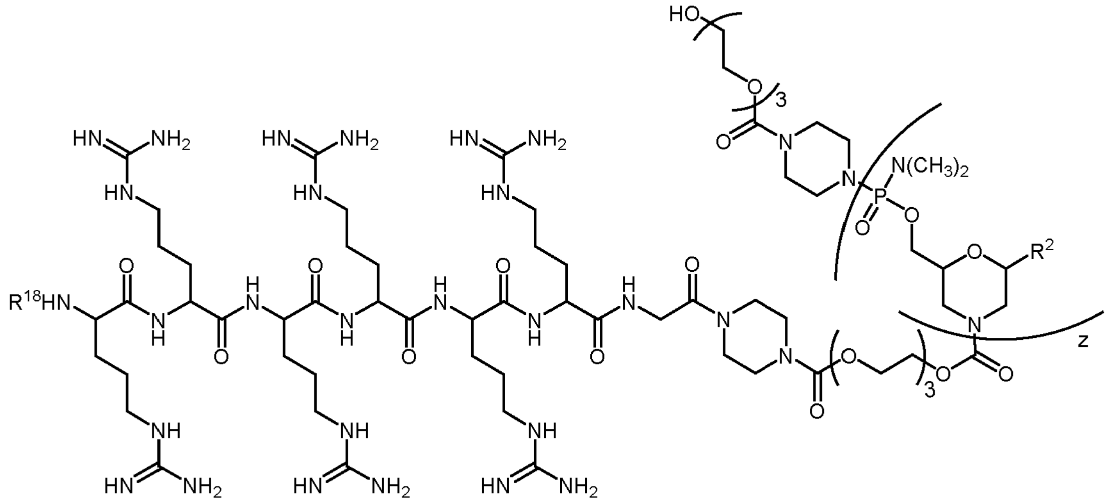

,

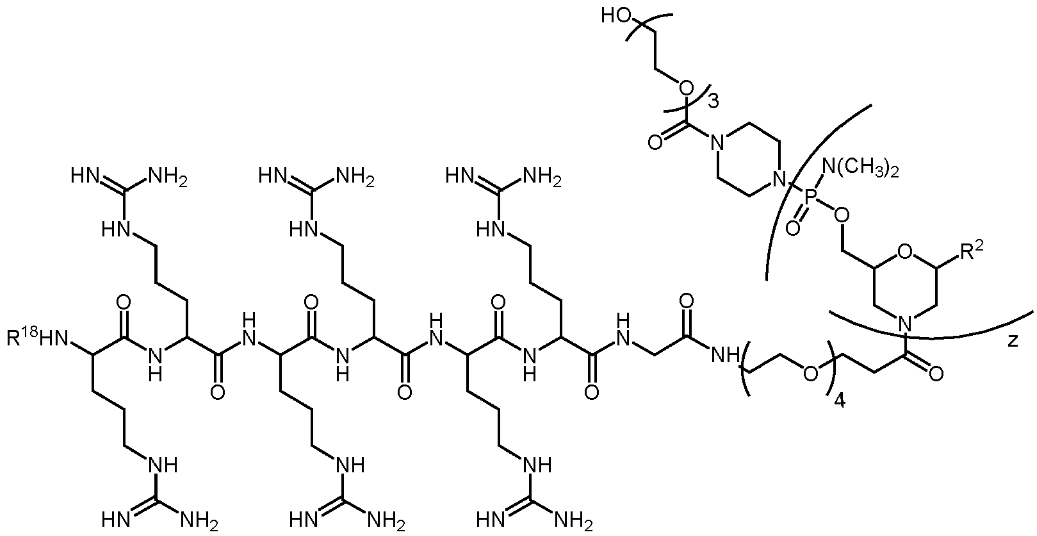

,

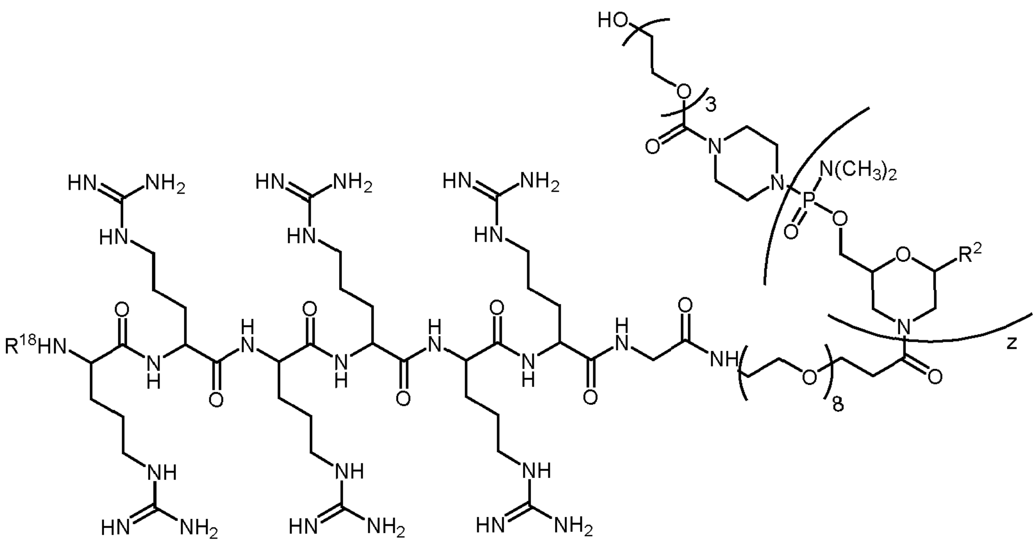

,

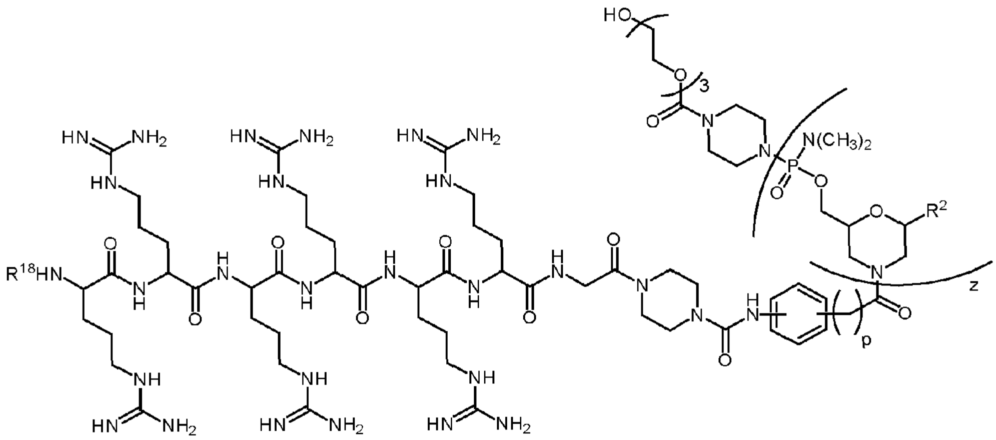

et

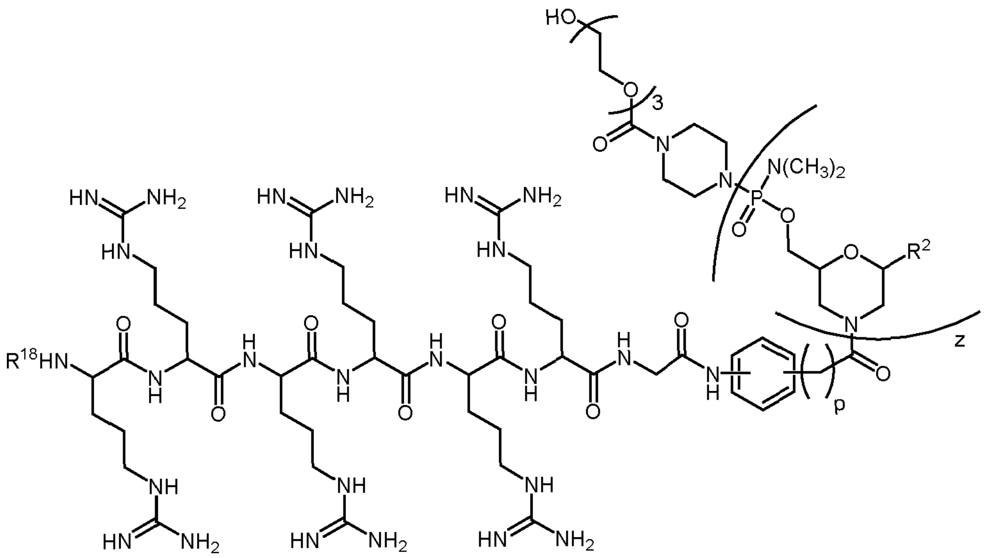

;

dans lequel
$R^{18}$ est choisi parmi H et $-C(O)CH_3$.

**12.** Conjugué peptide-oligomère selon l'une quelconque des revendications 1 à 11, ou sel pharmaceutiquement acceptable de celui-ci, où le conjugué peptide-oligomère comprend une séquence de ciblage présentant 50 %, 55 %, 60 %, 65 %, 70 %, 75 %, 80 %, 85 %, 90 %, 95 %, 99 % ou 100 % de complémentarité de séquence avec un ARN cible.

**13.** Conjugué peptide-oligomère selon la revendication 12, ou sel pharmaceutiquement acceptable de celui-ci, dans lequel la séquence de ciblage présente 100 % de complémentarité de séquence avec l'ARN cible.

**14.** Conjugué peptide-oligomère selon l'une quelconque des revendications 1 à 13 pour utilisation dans le traitement d'un trouble du système nerveux central, d'une maladie musculaire, d'une infection virale ou d'une infection bactérienne chez un sujet en ayant besoin.

**15.** Conjugué peptide-oligomère pour utilisation selon la revendication 14, dans laquelle la maladie musculaire est la dystrophie musculaire de Duchenne, ou dans laquelle l'infection virale est causée par un virus choisi parmi le virus Marburg, le virus Ebola, le virus de la grippe et le virus de la dengue, ou l'infection bactérienne est causée par *Mycobacterium tuberculosis,* ou dans laquelle le trouble du système nerveux central est l'amyotrophie spinale

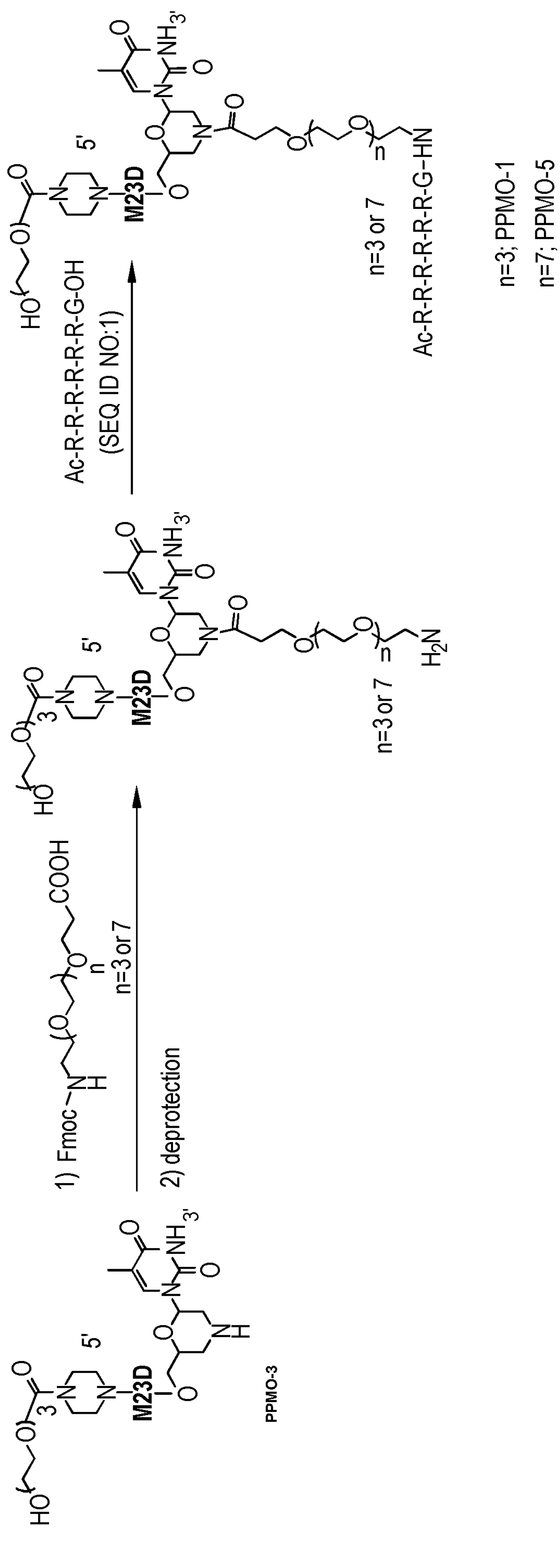
PPMO-3
1) Fmoc-
n=3 or 7
2) deprotection
n=3 or 7
Ac-R-R-R-R-R-R-G-OH
(SEQ ID NO:1)
n=3 or 7
n=3; PPMO-1
n=7; PPMO-5

*Fig. 1*

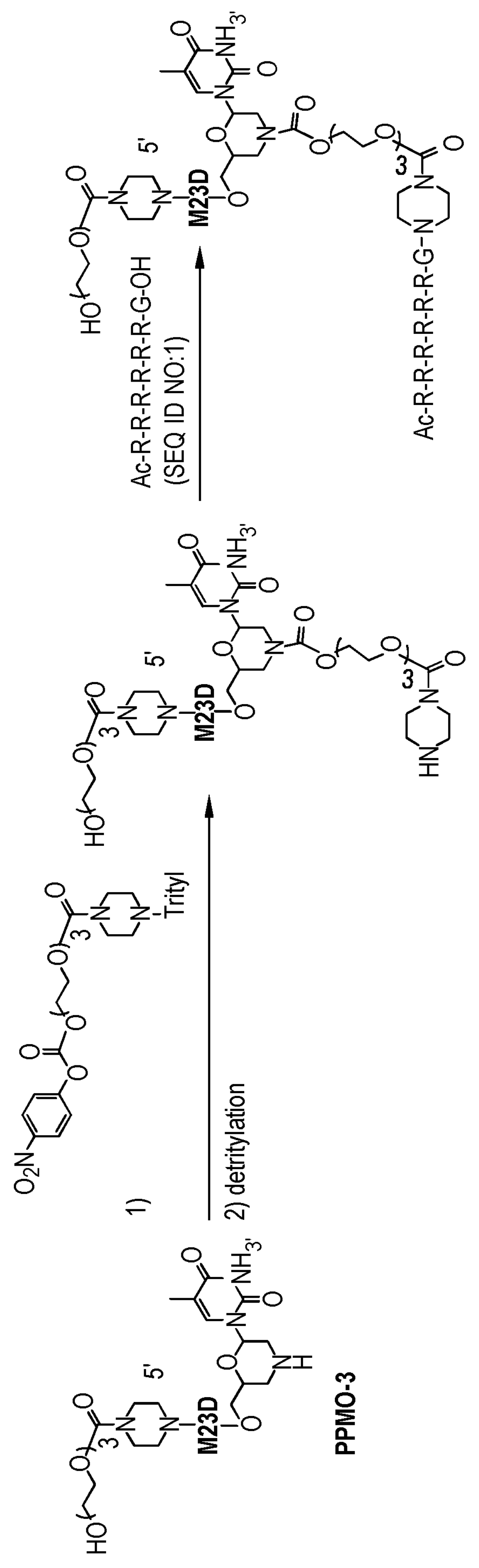


PPMO-3

*Fig. 2*

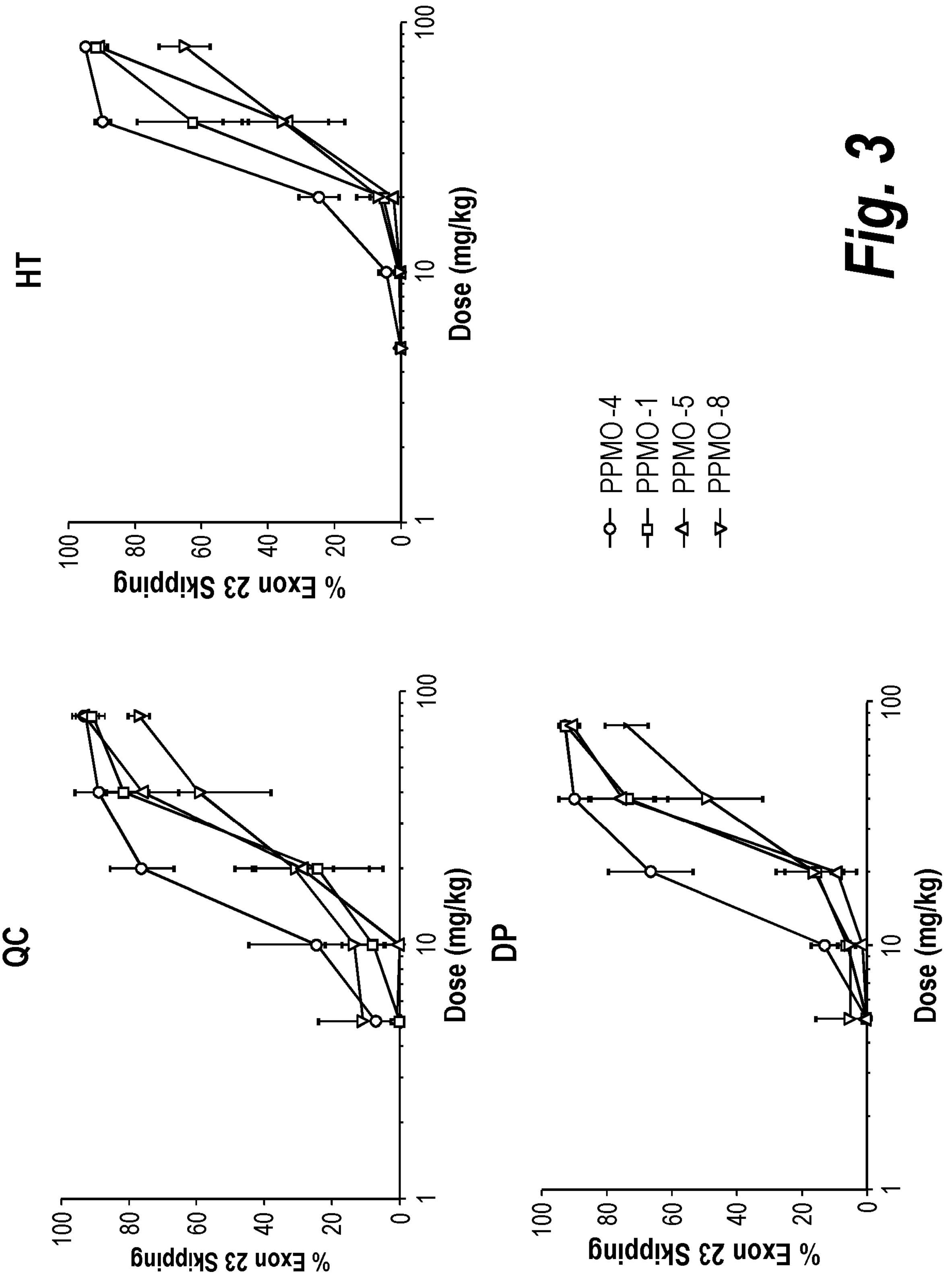
QC
HT
DP
% Exon 23 Skipping
Dose (mg/kg)
0
20
40
60
80
100
1
10
100
PPMO-4
PPMO-1
PPMO-5
PPMO-8

*Fig. 3*

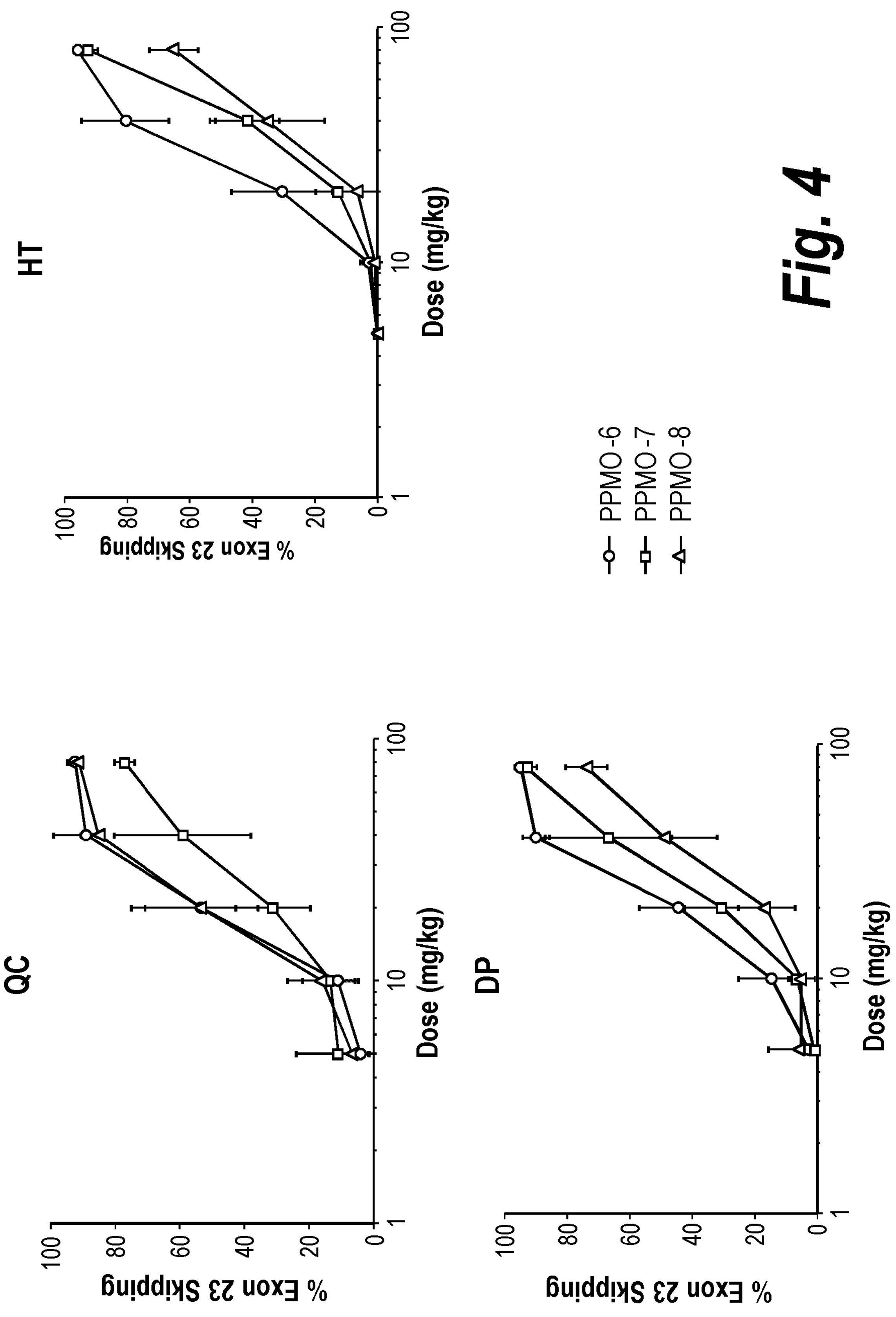
QC
DP
HT
% Exon 23 Skipping
Dose (mg/kg)
0
20
40
60
80
100
1
10
100
PPMO-6
PPMO-7
PPMO-8

Fig. 4

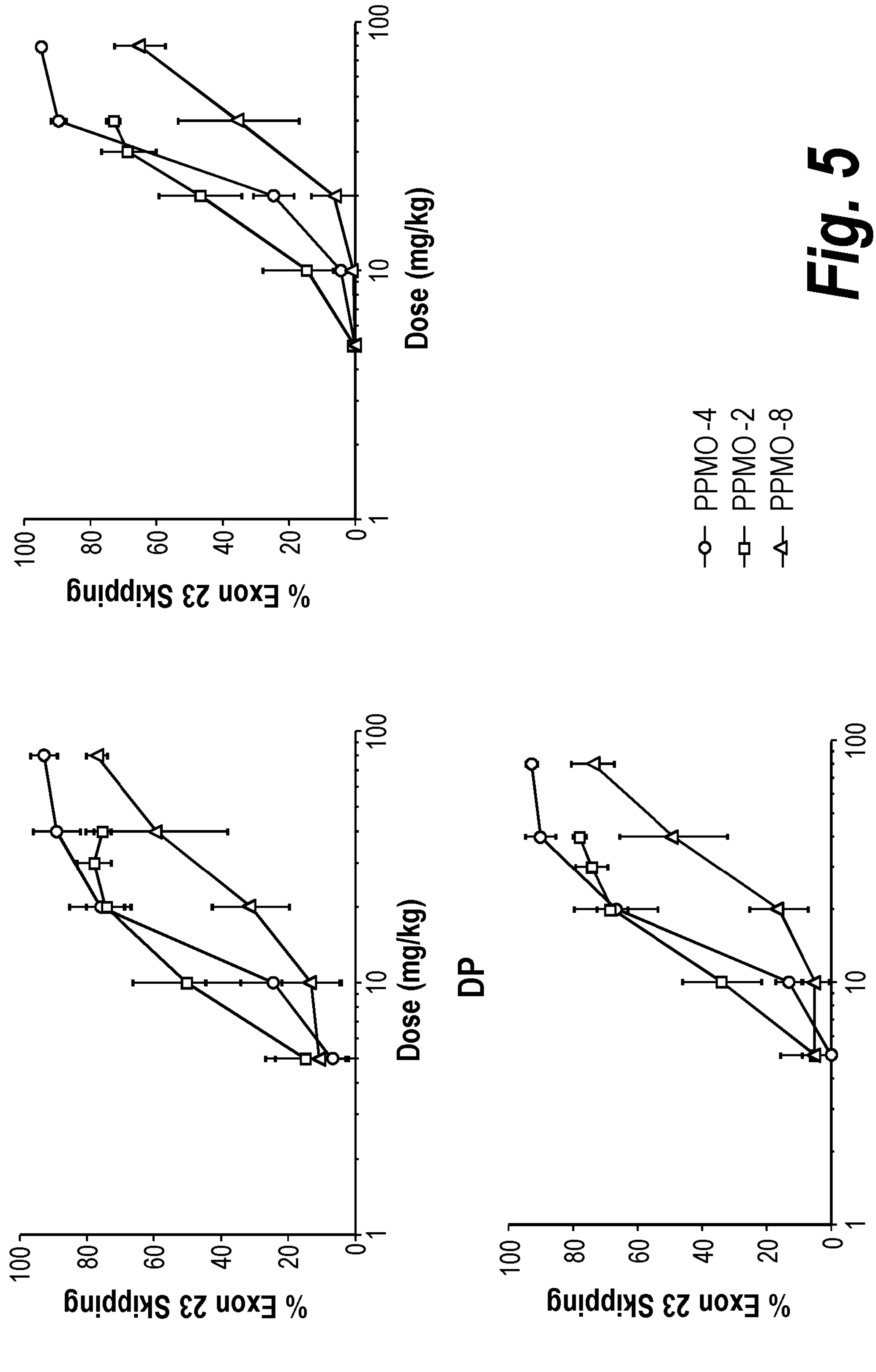
QC
DP
HT
% Exon 23 Skipping
Dose (mg/kg)
0
20
40
60
80
100
1
10
100
PPMO-4
PPMO-2
PPMO-8

*Fig. 5*

6/10

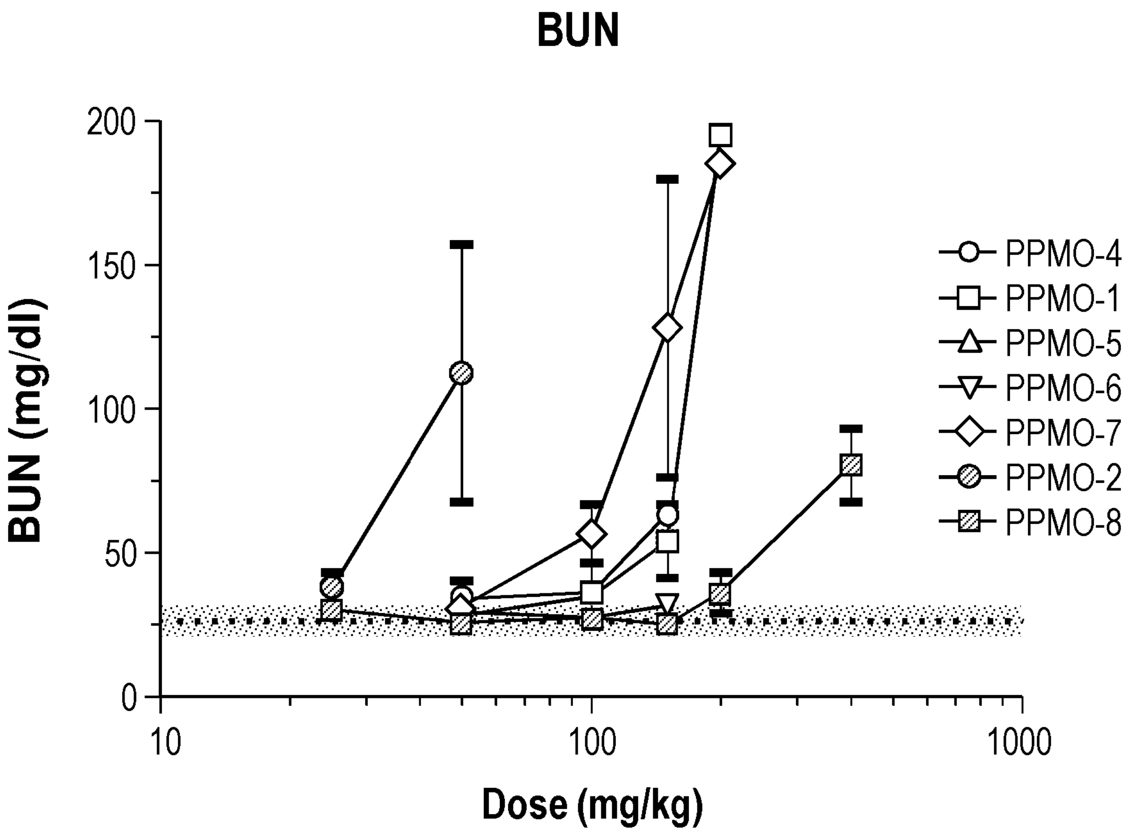
BUN
200
150
100
50
0
BUN (mg/dl)
10
100
1000
Dose (mg/kg)
PPMO-4
PPMO-1
PPMO-5
PPMO-6
PPMO-7
PPMO-2
PPMO-8

*Fig. 6*

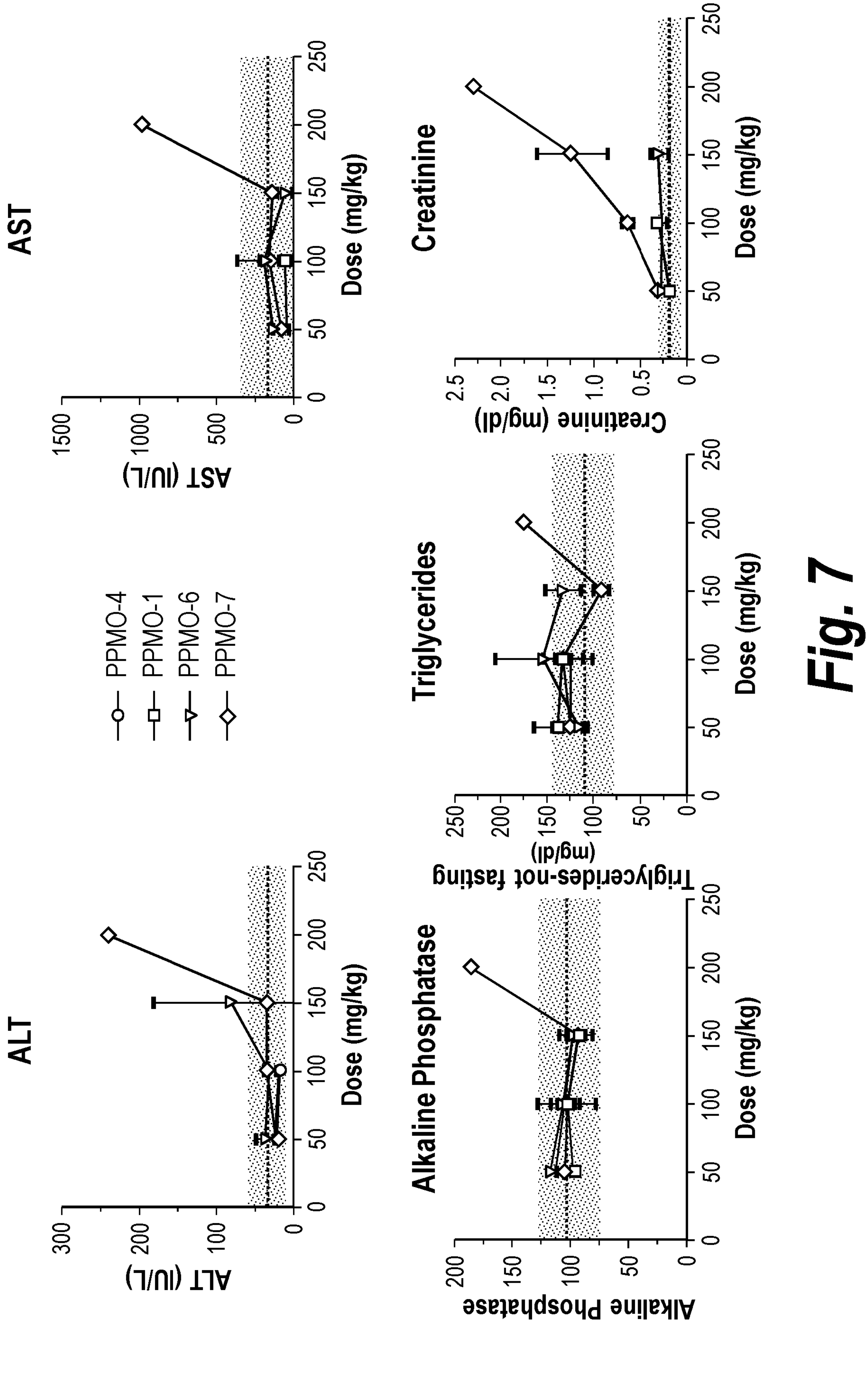
ALT
ALT (IU/L)
Dose (mg/kg)
AST
AST (IU/L)
Dose (mg/kg)
PPMO-4
PPMO-1
PPMO-6
PPMO-7
Alkaline Phosphatase
Alkaline Phosphatase
Dose (mg/kg)
Triglycerides
Triglycerides-not fasting (mg/dl)
Dose (mg/kg)
Creatinine
Creatinine (mg/dl)
Dose (mg/kg)

*Fig. 7*

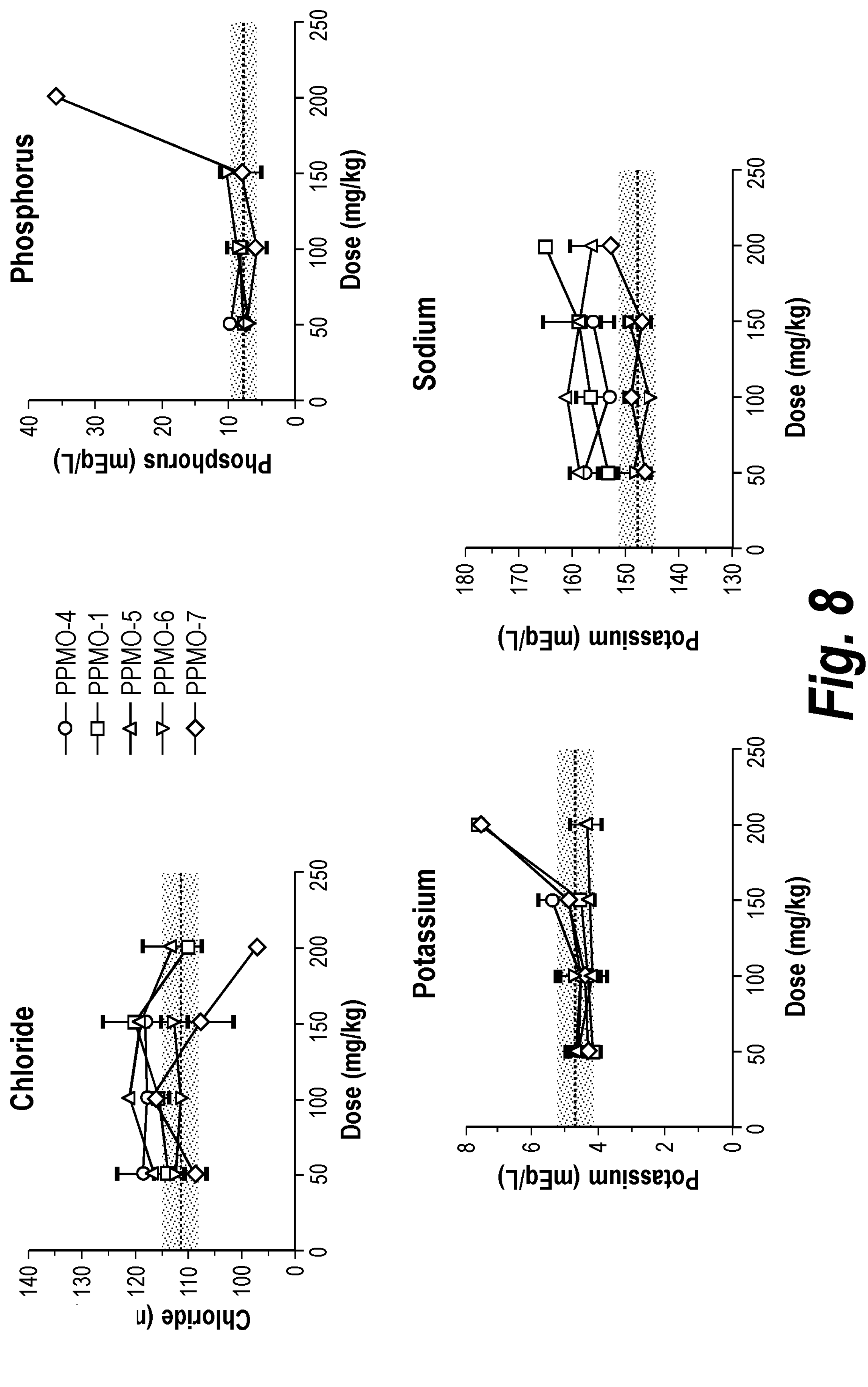
Phosphorus
Phosphorus (mEq/L)
Dose (mg/kg)
PPMO-4
PPMO-1
PPMO-5
PPMO-6
PPMO-7
Chloride
Chloride (n
Dose (mg/kg)
Sodium
Potassium (mEq/L)
Dose (mg/kg)
Potassium
Potassium (mEq/L)
Dose (mg/kg)

*Fig. 8*

9/10

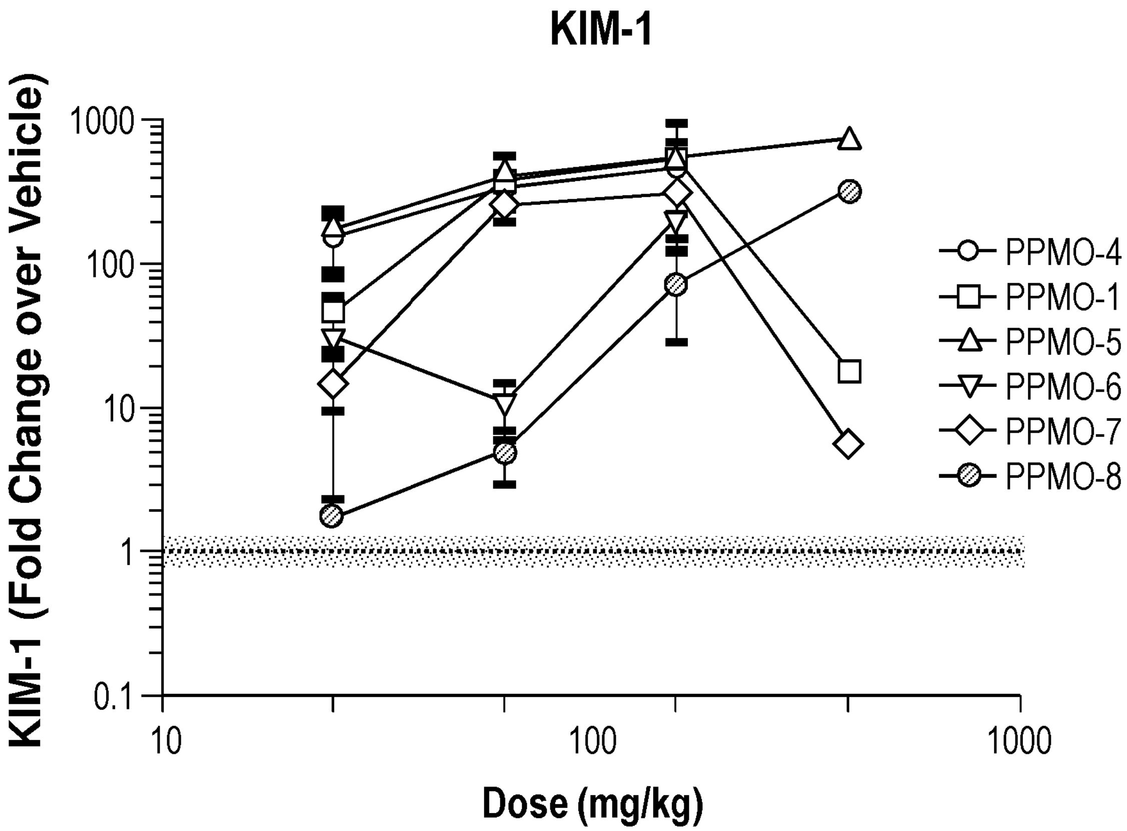
KIM-1
KIM-1 (Fold Change over Vehicle)
1000
100
10
1
0.1
10
100
1000
Dose (mg/kg)
PPMO-4
PPMO-1
PPMO-5
PPMO-6
PPMO-7
PPMO-8

*Fig. 9*

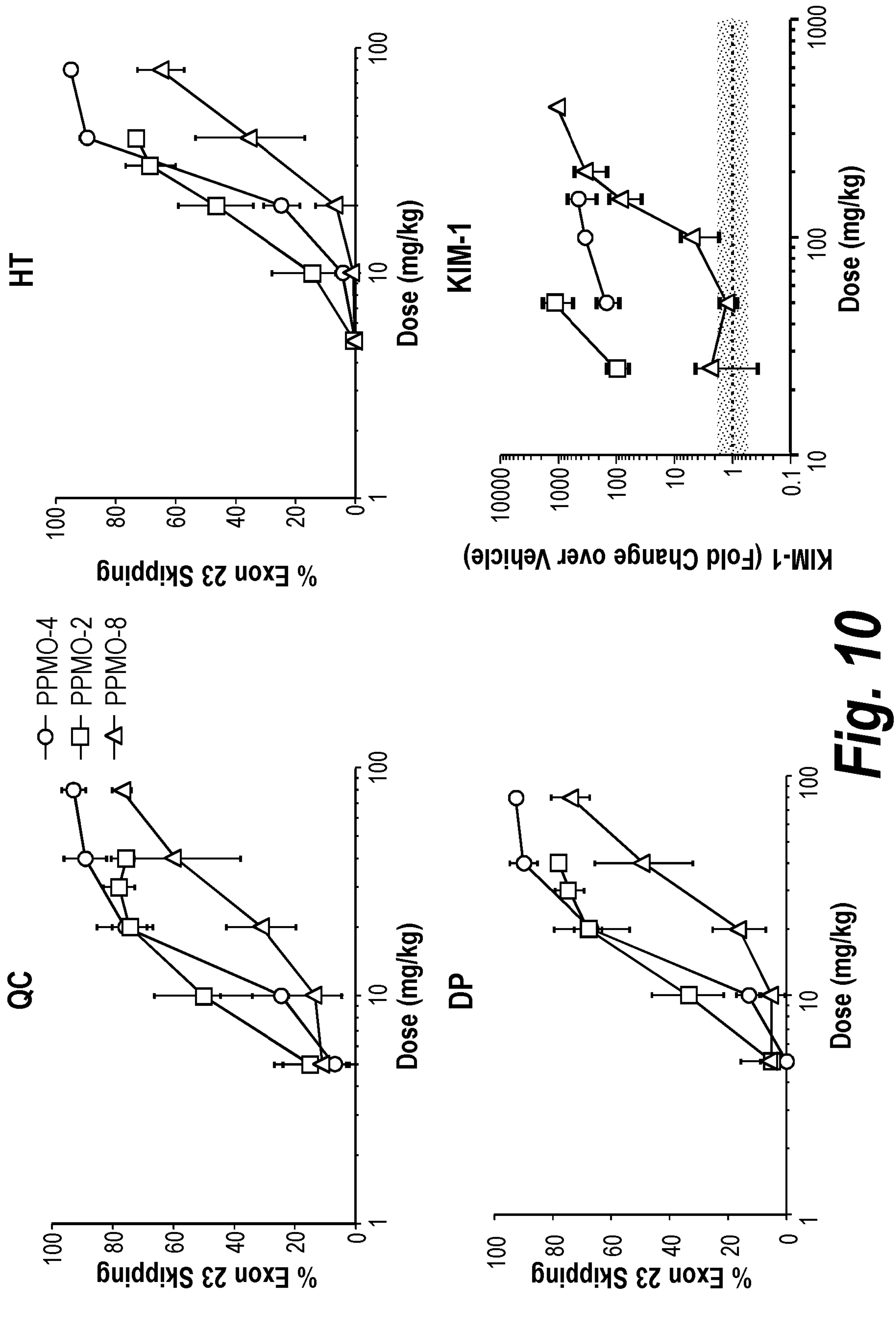
QC
HT
DP
KIM-1
PPMO-4
PPMO-2
PPMO-8
% Exon 23 Skipping
KIM-1 (Fold Change over Vehicle)
Dose (mg/kg)
0
20
40
60
80
100
1
10
100
1000
0.1
10000

*Fig. 10*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62267723
- WO 2014144978 A
- EP 2623507 A
- WO 2016138534 A
- US 5698685 A
- US 5217866 A
- US 5142047 A
- US 5034506 A
- US 5166315 A
- US 5185444 A
- US 5521063 A
- US 5506337 A
- US 8299206 B
- US 8076476 B
- WO 2009064471 A
- WO 2012043730 PCT
- WO 62267723 A


### Non-patent literature cited in the description


- **SAZANI et al.** *Int. J. Toxicol.,* 2011, vol. 30 (2), 313-321
- **T.W. GREENE ; P.G.M. WUTS.** Protective Groups in Organic Synthesis. John Wiley & Sons, 1999
- **CHIU ; RANA.** *RNA,* 2003, vol. 9, 1034-1048
- **LIMBACH et al.** *Nucleic Acids Research,* 1994, vol. 22, 2183-2196
- **REVANKAR ; RAO.** *Comprehensive Natural Products Chemistry,* vol. 7, 313
- **KRUEGER AT et al.** *Acc. Chem. Res.,* 2007, vol. 40, 141-150
- **KOOL, ET.** *Acc. Chem. Res.,* 2002, vol. 35, 936-943
- **BENNER S.A. et al.** *Nat. Rev. Genet.,* 2005, vol. 6, 553-543
- **ROMESBERG, F.E. et al.** *Curr. Opin. Chem. Biol.,* 2003, vol. 7, 723-733
- **HIRAO, I.** *Curr. Opin. Chem. Biol.,* 2006, vol. 10, 622-627
- Remington's Pharmaceutical Sciences. Mack Publishing Company, 1985, 1418
- *Journal of Pharmaceutical Science,* 1977, vol. 66, 2
- **HAMES et al.** Nucleic Acid Hybridization. IRL Press, 1985, 107-108
- **MIYADA C. G. ; WALLACE R. B.** Oligomer Hybridization Techniques. *Methods Enzymol.,* 1987, vol. 154, 94-107
- **SUMMERTON et al.** *Antisense and Nucleic Acid Drug Development,* 1997, vol. 7, 187-195
- Remington's Pharmaceutical Sciences. Mack Publishing Company, 1990
- USDA Animal Welfare Act 9 CFR Parts 1—3. *Federal Register 39129,* 22 July 1993
- Guide for the Care and Use of Laboratory Animals. National Academy Press, 1996

SEQUENCE LISTING

<110> SAREPTA THERAPEUTICS, INC.

<120> PEPTIDE OLIGONUCLEOTIDE CONJUGATES

<130> 586558: SPT-002PC

<140>
<141>

<150> 62/267,723
<151> 2015-12-15

<160> 12

<170> PatentIn version 3.5

<210> 1
<211> 7
<212> PRT
<213> Artificial Sequence

<220>
<223> Description of Artificial Sequence: Synthetic peptide

<220>
<221> MISC_FEATURE
<222> (1)..(1)
<223> Acetylation

<400> 1

```
Arg Arg Arg Arg Arg Arg Gly
1               5
```

<210> 2
<211> 18
<212> DNA
<213> Artificial Sequence

<220>
<223> Description of Artificial Sequence: Synthetic morpholino oligonucleotide

<220>
<221> misc_feature
<222> (1)..(18)
<223> Morpholino-based oligomer; nucleotide moieties according to Table 1

<220>
<221> misc_feature
<222> (1)..(18)
<223> Phosphoramidate or phosphorodiamidate linkage between nucleotides

<400> 2
gctattacct taacccag 18

<210> 3
<211> 19
<212> DNA
<213> Artificial Sequence

<220>
<223> Description of Artificial Sequence: Synthetic morpholino oligonucleotide

<220>
<221> misc_feature
<222> (1)..(19)
<223> Morpholino-based oligomer; nucleotide moieties according to Table 1

<220>
<221> misc_feature
<222> (1)..(19)
<223> Phosphoramidate or phosphorodiamidate linkage between nucleotides

<400> 3
gctattacct taacccagt 19

<210> 4
<211> 6
<212> PRT
<213> Artificial Sequence

<220>
<223> Description of Artificial Sequence: Synthetic peptide

<220>
<221> MISC_FEATURE
<222> (1)..(1)
<223> Acetylation

<220>
<221> MOD_RES
<222> (6) . . (6)
<223> Arg-APA

<400> 4

```
Arg Arg Arg Arg Arg Arg
1               5
```

<210> 5
<211> 7
<212> PRT
<213> Artificial Sequence

<220>
<223> Description of Artificial Sequence: Synthetic peptide

<220>
<221> MISC_FEATURE
<222> (1)..(1)
<223> Acetylation

<220>

<221> MOD_RES
<222> (7)..(7)
<223> Gly-PIP-PEG3

<400> 5

```
Arg Arg Arg Arg Arg Arg Gly
1               5
```

<210> 6
<211> 7
<212> PRT
<213> Artificial Sequence

<220>
<223> Description of Artificial Sequence: Synthetic peptide

<220>
<221> MISC_FEATURE
<222> (1)..(1)
<223> Acetylation

<220>
<221> MOD_RES
<222> (7)..(7)
<223> Gly-PEG4

<400> 6

```
Arg Arg Arg Arg Arg Arg Gly
1               5
```

<210> 7
<211> 7
<212> PRT
<213> Artificial Sequence

<220>
<223> Description of Artificial Sequence: Synthetic peptide

<220>
<221> MISC_FEATURE
<222> (1)..(1)
<223> Acetylation

<220>
<221> MOD_RES
<222> (7)..(7)
<223> Gly-PEG8

<400> 7

```
Arg Arg Arg Arg Arg Arg Gly
1               5
```

<210> 8
<211> 7
<212> PRT

<213> Artificial Sequence

<220>
<223> Description of Artificial Sequence: Synthetic peptide

<220>
<221> MISC_FEATURE
<222> (1)..(1)
<223> Acetylation

<220>
<221> MOD_RES
<222> (1)..(6)
<223> D-amino acid

<400> 8

```
Arg Arg Arg Arg Arg Arg Gly
1               5
```

<210> 9
<211> 27
<212> DNA
<213> Artificial Sequence

<220>
<223> Description of Artificial Sequence: Synthetic primer

<400> 9
caatgtttct ggatgcagac tttgtgg 27

<210> 10
<211> 27
<212> DNA
<213> Artificial Sequence

<220>
<223> Description of Artificial Sequence: Synthetic primer

<400> 10
gttcagcttc actctttatc ttctgcc 27

<210> 11
<211> 21
<212> DNA
<213> Artificial Sequence

<220>
<223> Description of Artificial Sequence: Synthetic primer

<400> 11
cacatctttg atggtgtgag g 21

<210> 12
<211> 22
<212> DNA
<213> Artificial Sequence

<220>
<223> Description of Artificial Sequence: Synthetic primer

<400> 12
caacttcagc catccatttc tg 22